# EUROPEAN PATENT SPECIFICATION

(11) **EP 1 261 932 B1**
(45) Date of publication and mention of the grant of the patent: **30.09.2009**
(21) Application number: 00972148.1
(22) Date of filing: 13.10.2000
(51) Int. Cl.: G06F 19/00

(54) **METHODS FOR IDENTIFYING POLYMORPHIC GENETIC MARKERS**
VERFAHREN ZUR IDENTIFIZIERUNG VON POLYMORPHEN GENETISCHEN MARKERN
METHODES D'IDENTIFICATION DE MARQUEURS GENETIQUES POLYMORPHES

(30) Priority: 13.10.1999 US 159176 P; 10.07.2000 US 217658 P; 10.07.2000 US 217251 P; 19.09.2000 US 663968
(43) Date of publication of application: 04.12.2002
(60) Divisional application: 09157036.6
(73) Proprietor: SEQUENOM, INC., San Diego, California 92121 (US)
(72) Inventor: BRAUN, Andreas, San Diego, CA 92130 (US); KÖSTER, Hubert, 6900 Lugano-Cassarate (CH); VAN DEN BOOM, Dirk, D-63303 Dreieich (DE); PING, Yip, San Diego, CA 92116 (US); RODI, Charlie, Del Mar, CA 92014 (US); HE, Liyan, San Diego, CA 92128 (US); CHIU, Norman, San Diego, CA 92126 (US); JURINKE, Christian, 20255 Hamburg (DE)
(74) Representative: Baldock, Sharon Claire
(86) International application number: PCT/US2000/028413
(87) International publication number: WO 2001/027857

(56) References cited:
- WO-A-00/51053
- WO-A-97/40462
- WO-A-98/24935
- WO-A-98/35609
- WO-A-99/05323
- COLLINS F S ET AL: "A DNA Polymorphism Discovery Resource for Research on Human Genetic Variation" GENOME RESEARCH, vol. 8, 1998, pages 1229-1231, XP002177106
- SARKAR C ET AL: "Human Genetic Bi-allelic Sequences (HGBASE), a Database of Intra-genic Polymorphisms" MEM INST OSWALDO CRUZ, [Online] vol. 93, no. 5, September 1998 (1998-09) - October 1998 (1998-10), pages 693-694, XP002177107 Rio de Janeiro Retrieved from the Internet: <URL:http://www.cgb.ki.se/cgb/groups/brook es/publications.htm> [retrieved on 2001-09-05]
- FOSTER M W AND FREEMAN W L: "Naming Names in Human Genetic Variation Research" GENOME RESEARCH, vol. 8, 1998, pages 755-757, XP002177108
- LAKEN S.J. ET AL: 'Genotyping by mass spectrometric analysis of short DNA fragments' NATURE BIOTECHNOLOGY vol. 16, no. 13, December 1998, NEW YORK, pages 1352 - 1356, XP002138136
- GRIFFIN TIMOTHY J. ET AL: 'Direct genetic analysis by matrix-assisted laser desorption/ionization mass spectrometry' PROCEEDINGS OF THE NATIONAL ACADEMY OF SCIENCES OF USA vol. 96, no. 11, 25 May 1999, WASHINGTON, D.C., pages 6301 - 6306, XP002154776
- BUTLER J.M. ET AL: 'Reliable genotyping of short tandem repeat loci without an allelic ladder using time-of-flight mass spectrometry' INTERNATIONAL JOURNAL OF LEGAL MEDICINE vol. 112, no. 1, 01 January 1998, pages 45 - 49, XP002094023
- FEI Z.; ONO T. AND SMITH L.M.: 'MALDI-TOF mass spectrometric typing of single nucleotide polymorphisms with mass-tagged ddNTPs' NUCLEIC ACIDS RESEARCH vol. 26, no. 11, 01 June 1998, MADISON, WI, USA, pages 2827 - 2828
- HAFF L.A.; SMIRNOV I.P.: 'Multiplex genotyping of PCR products with Mass Tag-labeled primers' NUCLEIC ACIDS RESEARCH vol. 25, no. 18, 1997, SURREY, GB, pages 3749 - 3750, XP002975048

## Description

### FIELD OF THE INVENTION

Methods that use a database to identify and correlate witch polymorphic genetic markers and other markers with diseases and conditions are provided.

### BACKGROUND

Diseases in all organisms have a genetic component, whether inherited or resulting from the body's response to environmental stresses, such as viruses and toxins. The ultimate goal of ongoing genomic research is to use this information to develop new ways to identify, treat and potentially cure these diseases. The first step has been to screen disease tissue and identify genomic changes at the level of individual samples. The identification of these "disease" markers has then fueled the development and commercialization of diagnostic tests that detect these errant genes or polymorphisms. With the increasing numbers of genetic markers, including single nucleotide polymorphisms (SNPs), microsatellites, tandem repeats, newly mapped introns and exons, the challenge to the medical and pharmaceutical communities is to identify genotypes which not only identify the disease but also follow the progression of the disease and are predictive of an organism's response to treatment.

Currently the pharmaceutical and biotechnology industries find a disease and then attempt to determine the genomic basis for the disease. This approach is time consuming and expensive and in many cases involves the investigator guessing as to what pathways might be involved in the disease.

### Genomics

Presently the two main strategies employed in analyzing the available genomic information are the technology driven reverse genetics brute force strategy and the knowledge-based pathway oriented forward genetics strategy. The brute force approach yields large databases of sequence information but little information about the medical or other uses of the sequence information. Hence this strategy yields intangible products of questionable value. The knowledge-based strategy yields small databases that contain a lot of information about medical uses of particular DNA sequences and other products in the pathway and yield tangible products with a high value.

### Polymorphisms

Polymorphisms have been known since 1901 with the identification of blood types. In the 1950's they were identified on the level of proteins using large population genetic studies. In the 1980's and 1990's many of the known protein.polymorphisms were correlated with genetic loci on genomic DNA. For example, the gene dose of the apolipoprotein E type 4 allele was correlated with the risk of Alzheimer's disease in late onset families (see, *e.g.,* Corder et al. (1993) Science 261: 921-923; mutation in blood coagulation factor V was associated with resistance to activated protein C (see, *e.g.,* Bertina et al. (1994) Nature 369:64-67); resistance to HIV-1 infection has been shown in caucasian individuals bearing mutant alleles of the CCR-5 chemokine receptor gene (see, *e.g.,* Samson et al. (1996) Nature 382:722-725); and a hypermutable tract in antigen presenting cells (APC, such as macrophages), has been identified in familial colorectal cancer in individuals of Ashkenzi jewish background (see, *e.g.,* Laken et al. (1997) Nature Genet. 17:79-83). There may be more than three million polymorphic sites in the human genome. Many have been identified, but not yet characterized or mapped or associated with a marker.

### Single nucleotide polymorphisms (SNPs)

Much of the focus of genomics has been in the identification of SNPs, which are important for a variety of reasons. They allow indirect testing (association of haplotypes) and direct testing (functional variants). They are the most abundant and stable genetic markers. Common diseases are best explained by common genetic alterations, and the natural variation in the human population aids in understanding disease, therapy and environmental interactions.

Currently, the only available method to identify SNPs in DNA is by sequencing, which is expensive, difficult and laborious. Furthermore, once a SNP is discovered it must be validated to determine if it is a real polymorphism and not a sequencing error. Also, discovered SNPs must then be evaluated to determine if they are associated with a particular phenotype. Thus, there is a need to develop new paradigms for identifying the genomic basis for disease and markers thereof. Therefore, it is an object herein to provide methods for identifying the genomic basis of disease and markers thereof.

WO9905323 discloses a system for a gene expression database. This allows easy identification of genes or expressed sequence tags whose expression correlates to a particular tissue type.

WO97/40462 discloses a database containing value sets indicating polymorphic forms and phenotypes for each member of a subject population, and methods of analysing the database to determine the correlation inbetween the polymorphic form at at least one genetic locus and at least two phenotypes.

Laken et al., Nature biotechnology Vol 16 No. 13 1998 pages 1352 - 1356. Discloses methods of phenotyping by mass spectrometric analysis of short DNA fragments.

Griffin et al., PNAS, Vol 96 No. 11, 1999, pages 6301 - 6306. Discloses direct genetic analysis by MALDI-TOF mass spectrometry.

Butler et al., Journal of legal medicine, Vol 112 No. 1, 1998, page 45 - 49. Discloses methods of genotyping short tandem repeat loci using TOF mass spectrometry.

Fei et al., Nucleic acid research, Vol 26 No. 11, 1998, Page 2827 - 2828. Discloses MALDI-TOF mass spectrometric typing of single nucleotide polymorphisms with mass tagged ddNTP's.

Hoff et al., Nucleic acid research, Vol 25 No. 18, 1997, pages 3749 - 3750. Discloses multiplex genotyping of PCR products with mass tagged labelled primers.

### SUMMARY

Methods of using databases as described in the claims are provided herein. The databases for use in the methods comprise sets of parameters associated with subjects in populations selected only on the basis of being healthy (i.e., where the subjects are mammals, such as humans, they are selected based upon apparent health and no detectable infections). The databases can be sorted based upon one or more of the selected parameters.

The databases are preferably relational databases, in which an index that represents each subject serves to relate parameters, which are the data, such as age, ethnicity, sex, medical history, etc and ultimately genotypic information, that was inputted into and stored in the database. The database can then be sorted according to these parameters. Initially, the parameter information is obtained from a questionnaire answered by each subject from whom a body tissue or body fluid sample is obtained. As additional information about each sample is obtained, this information can be entered into the database and can serve as a sorting parameter.

The databases obtained from healthy individuals have numerous uses, such as correlating known polymorphisms with a phenotype or disease. The databases can be used to identify alleles that are deleterious, that are beneficial, and that are correlated with diseases.

For purposes herein, genotypic information can be obtained by any method known to those of skill in the art, but is preferably obtained using mass spectrometry.

Existing databases of subjects and genotypic and other parameters, such as age, ethnicity, race, and gender can be used in the current methods. The database can be sorted according to the methods herein and alleles that exhibit statistically significant correlations with any of the sorting parameters can be identified. It is noted, that the databases described herein and randomly selected databases will perform better in these methods. The healthy database described herein, provides advantages not heretofore recognized or exploited. In addition, the databases described herein represent a greater genetic diversity than the unselected databases typically utilized for the discovery of polymorphisms and thus allow for the enhanced discovery and correlation of polymorphisms.

The databases described herein can be used for taking an identified polymorphism, and ascertaining whether it changes in frequency when the data is sorted according to a selected parameter.

One use of these methods is correlating a selected marker with a particular parameter by following the occurrence of known genetic markers and then, having made this correlation, determining or identifying correlations with diseases. Examples of this use are p53 and Lipoprotein Lipase polymorphism. As exemplified herein, known markers are shown to have particular correlation with certain groups, such as a particular ethnicity or race or one sex. Such correlations will then permit development of better diagnostic tests and treatment regimens.

These methods are valuable for identifying one or more genetic markers whose frequency changes within the population as a function of age, ethnic group, sex or some other criteria. This can allow the identification of previously unknown polymorphisms and ultimately a gene or pathway involved in the onset and progression of disease.

The databases and methods provided herein permit, among other things, identification of components, particularly key components, of a disease process by understanding its genetic underpinnings and also permit an understanding of processes, such as individual drug responses. The databases and methods provided herein also can be used in methods involving elucidation of pathological pathways, in developing new diagnostic assays, identifying new potential drug targets, and in identifying new drug candidates.

The methods and databases can be used with experimental procedures, including, but are not limited to, *in silico* SNP identification, *in vitro* SNP identification/verification, genetic profiling of large populations, and in biostatistical analyses and interpretations.

Also, provided herein, are methods for determining a genetic marker that correlate with age, comprising identifying a polymorphism and determining the frequency of the polymorphism with increasing age in a healthy population.

Further provided herein are methods for determining whether a genetic marker correlates with susceptibility to morbidity, early mortality, or morbidity and early mortality, comprising identifying a polymorphism and determining the frequency of the polymorphism with increasing age in a healthy population.

Any of the methods herein described can be used out in a multiplex format.

The data set is DNA spectrometry data received from a mass spectrometer. The data set is denoised, and a baseline is deleted. Since possible compositions of the biological sample may be known, expected peak areas may be determined. Using the expected peak areas, a residual baseline is generated to further correct the data set. Probable peaks are then identifiable in the corrected data set, which are used to identify the composition of the biological sample. In a disclosed example, statistical methods are employer to determine the probability that a probable peak is an actual peak, not an actual peak, or that the data too inconclusive to call.

In such a manner, complete SNP profile information, for example, may be collected efficiently. More importantly, the collected data is analyzed with highly accurate results. For example, when a particular composition is called, the result may be relied upon with great confidence. Such confidence is provided by the robust computational process employed .

### DESCRIPTION OF THE DRAWINGS

Figure 1 depicts an exemplary sample bank which may be used in the current methods Panel 1 shows the samples as a function of sex and ethnicity. Panel 2 shows the caucasians as a function of age. Panel 3 shows the Hispanics as a function of age.
Figures 2A and 2C show an age- and sex-distribution of the 291S allele of the lipoprotein lipase gene in which a total of 436 males and 589 females were investigated. Figure 2B shows an age distribution for the 436 males.
Figure 3 is an exemplary questionnaire for population-based sample banking.
Figure 4 depicts processing and tracking of blood sample components.

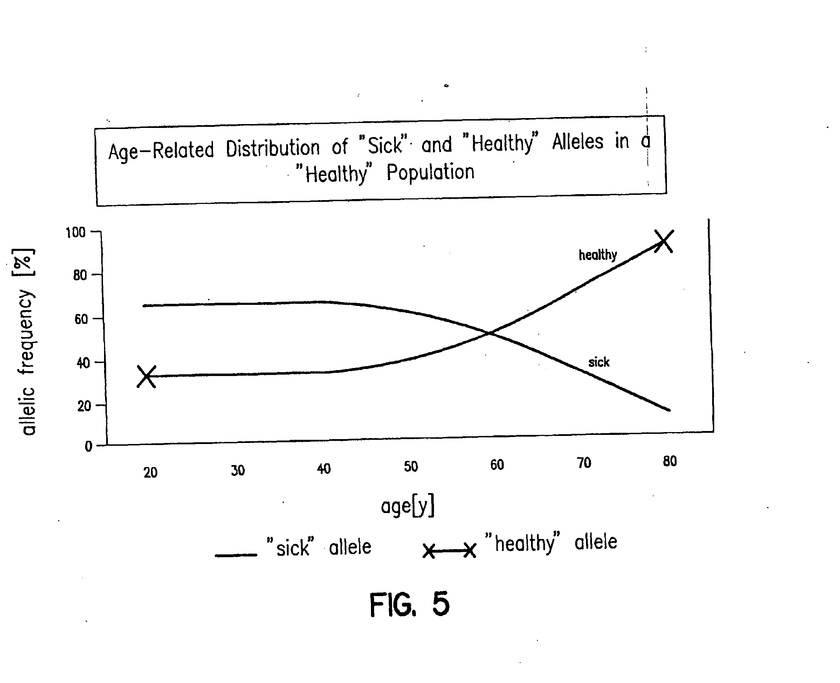

Figure 5 depicts the allelic frequency of "sick" alleles and "healthy" alleles as a function of age. It is noted that the relative frequency of healthy alleles increases in a population with increasing age.

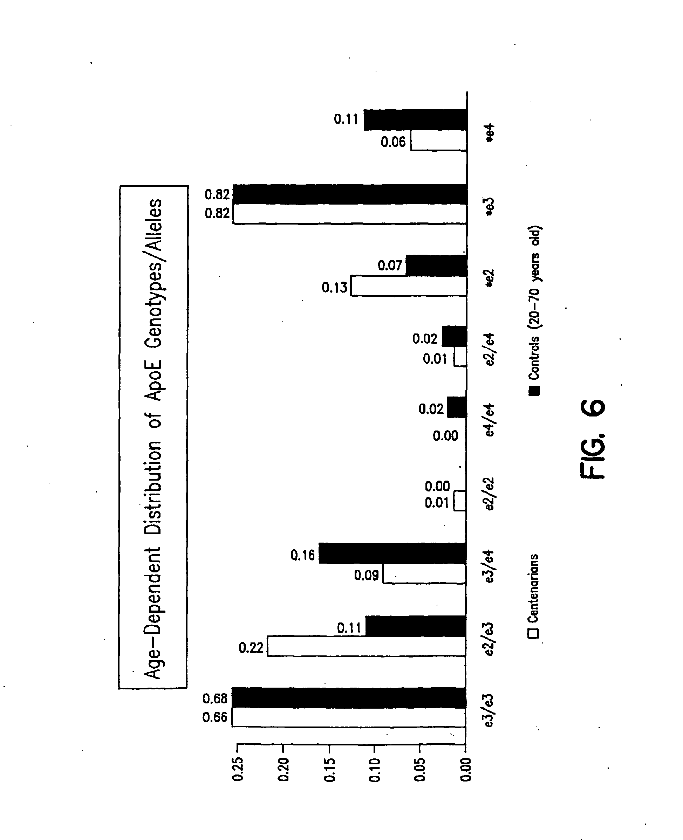

Figure 6 depicts the age-dependent distribution of ApoE genotypes (see, Schachter et al. (1994) Nature Genetics 6:29-32)*.*
Figure 7A-D depicts age-related and genotype frequency of the p53 (tumor suppressor) codon 72 among the caucasian population in the database. *R72 and *P72 represent the frequency of the allele in the database population. R72, R72P, and P72 represent the genotypes of the individuals in the population. The frequency of the homozygous P72 allele drops from 6.7% to 3.7% with age.
Figure 8 depicts the allele and genotype frequencies of the p21 S31 R allele as a function of age.
Figure 9 depicts the frequency of the FVII Allele 353Q in pooled versus individual samples.
Figure 10 depicts the frequency of the CETP (cholesterol ester transfer protein) allele in pooled versus individual samples

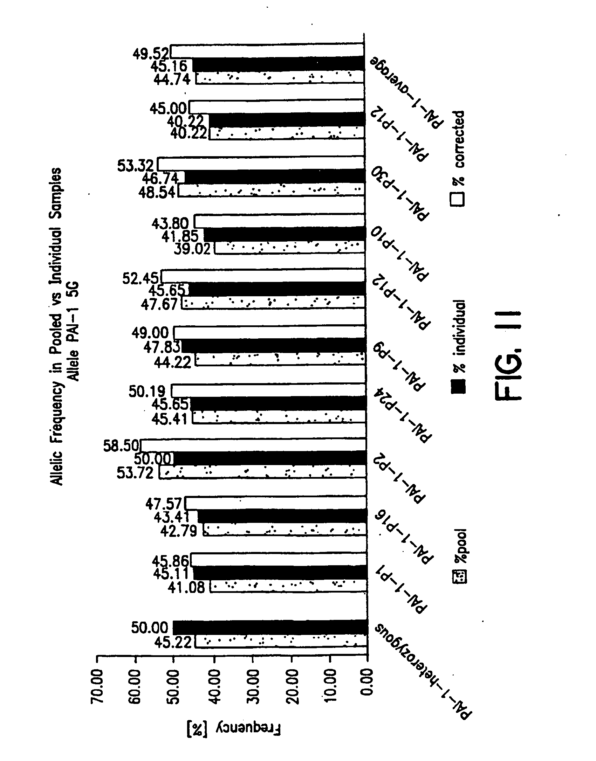

Figure 11 depicts the frequency of the plasminogen activator inhibitor-1 (PAI-1) 5G in pooled versus individual samples
Figure 12 shows mass spectra of the samples and the ethnic diversity of the PAI-1 alleles.
Figure 13 shows mass spectra of the samples and the ethnic diversity of the CETP 405 alleles.
Figure 14 shows mass spectra of the samples and the ethnic diversity of the Factor VII 353 alleles.
Figure 15 shows ethnic diversity of PAI-1, CETP and Factor VII using the pooled DNA samples.

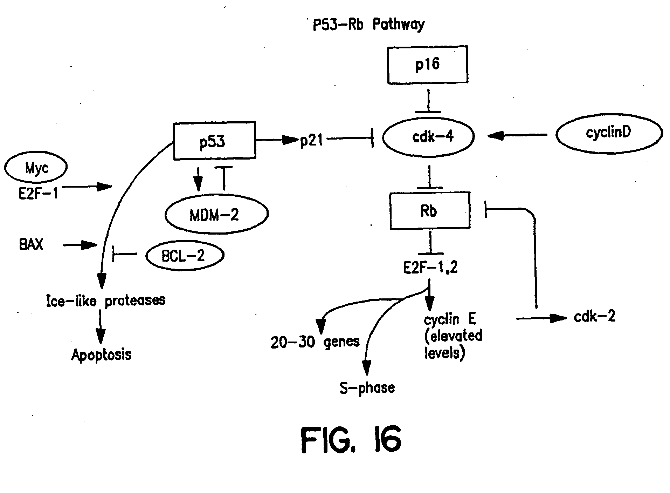

Figure 16 shows the p53-Rb pathway and the relationships among the various factors in the pathway.
Figure 17, which is a block diagram of a computer constructed to provide and process the databases, depicts a typical computer system for storing and sorting the databases and practicing the methods provided herein.
Figure 18 is a flow diagram that illustrates the processing steps performed using the computer illustrated in Figure 17, to maintain and, provide access to the databases for identifying polymorphic genetic markers.
Figure 19 is a histogram showing the allele and genotype distribution in the age and sex stratified Caucasian population for the AKAP10-1 locus.
Figure 20 is a histogram showing the allele and genotype distribution in the age and sex stratified Caucasian population for the AKAP10-5 locus. Bright green bars show frequencies in individuals younger than 40 years; dark green bars show frequencies in individuals older than 60 years.
Figure 21 is a histogram showing the allele and genotype distribution in the age and sex stratified Caucasian population for the h-msrA locus. Genotype difference between male age groups is significant.
Figure 22A-D is a sample data collection questionnaire used for the healthy database.
Figure 23 is a flowchart showing processing performed by the computing device of Figure 24 when performing genotyping of sense strands and antisense strands from assay fragments.
Figure 24 is a block diagram showing a system which is not within the scope of the present invention;
Figure 25 is a flowchart of a method of identifying a biological sample;
Figure 26 is a graphical representation of data from a mass spectrometer;
Figure 27 is a diagram of wavelet transformation of mass spectrometry data;
Figure 28 is a graphical representation of wavelet stage O hi data;
Figure 29 is a graphical representation of stage O noise profile;
Figure 30 is a graphical representation of generating stage noise standard deviations;
Figure 31 is a graphical representation of applying a threshold to data stages;
Figure 32 is a graphical representation of a sparse data set;
Figure 33 is a formula for signal shifting;
Figure 34 is a graphical representation of a wavelet transformation of a denoised and shifted signal;
Figure 35 is a graphical representation of a denoised and shifted signal;
Figure 36 is a graphical representation of removing peak sections;
Figure 37 is a graphical representation of generating a peak free signal ;
Figure 38 is a block diagram of a method of generating a baseline correction;
Figure 39 is a graphical representation of a baseline and signal;
Figure 40 is a graphical representation of a signal with baseline removed;
Figure 41 is a table showing compressed data;
Figure 42 is a flowchart of method for compressing data;
Figure 43 is a graphical representation of mass shifting;
Figure 44 is a graphical representation of determining peak width;
Figure 45 is a graphical representation of removing peaks;
Figure 46 is a graphical representation of a signal with peaks removed;
Figure 47 is a graphical representation of a residual baseline;
Figure 48 is a graphical representation of a signal with residual baseline removed;
Figure 49 is a graphical representation of determining peak height;
Figure 50 is a graphical representation of determining signal-to-noise for each peak;
Figure 51 is a graphical representation of determining a residual error for each peak;
Figure 52 is a graphical representation of peak probabilities;
Figure 53 is a graphical representation of applying an allelic ratio to peak probability;
Figure 54 is a graphical representation of determining peak probability
Figure 55 is a graphical representation of calling a genotype;
Figure 56 is a flowchart showing a statistical procedure for calling genotype;
Figure 57 is a flowchart showing processing performed by the computing device of Figure 1 when performing standardless genotyping; and
Figure 58 is graphical representation of applying an allelic ratio to peak probability for standardless genotype processing.

### DETAILED DESCRIPTION

### Definitions

Unless defined otherwise, all technical and scientific terms used herein have the same meaning as is commonly understood by one of ordinary skill in the art to which this invention belongs. All patents, applications, published applications and other publications and sequences from GenBank and other databases referred to herein throughout the disclosure are incorporated by reference in their entirety.

As used herein, a biopolymer includes, but is not limited to, nucleic acid, proteins, polysaccharides, lipids and other macromolecules. Nucleic acids include DNA, RNA, and fragments thereof. Nucleic acids may be derived from genomic DNA, RNA, mitochondrial nucleic acid, chloroplast nucleic acid and other organelles with separate genetic material.

As used herein, morbidity refers to conditions, such as diseases or disorders, that compromise the health and well-being of an organism, such as an animal. Morbidity susceptibility or morbidity-associated genes are genes that, when altered, for example, by a variation in nucleotide sequence, facilitate the expression of a specific disease clinical phenotype. Thus, morbidity susceptibility genes have the potential, upon alteration, of increasing the likelihood or general risk that an organism will develop a specific disease.

As used herein, mortality refers to the statistical likelihood that an organism, particularly an animal, will not survive a full predicted lifespan. Hence, a trait or a marker, such as a polymorphism, associated with increased mortality is observed at a lower frequency in older than younger segments of a population.

As used herein, a polymorphism, e.g. genetic variation, refers to a variation in the sequence of a gene in the genome amongst a population, such as allelic variations and other variations that arise or are observed. Thus, a polymorphism refers to the occurrence of two or more genetically determined alternative sequences or alleles in a population. These differences can occur in coding and non-coding portions of the genome, and can be manifested or detected as differences in nucleic acid sequences, gene expression, including, for example transcription, processing, translation, transport, protein processing, trafficking, DNA synthesis, expressed proteins, other gene products or products of biochemical pathways or in post-translational modifications and any other differences manifested amongst members of a population. A single nucleotide polymorphism (SNP) refers to a polymorphism that arises as the result of a single base change, such as an insertion, deletion or change in a base.

A polymorphic marker or site is the locus at which divergence occurs. Such site may be as small as one base pair (an SNP). Polymorphic markers include, but are not limited to, restriction fragment length polymorphisms, variable number of tandem repeats (VNTR's), hypervariable regions, minisatellites, dinucleotide repeats, trinucleotide repeats, tetranucleotide repeats and other repeating patterns, simple sequence repeats and insertional elements, such as Alu. Polymorphic forms also are manifested as different mendelian alleles for a gene. Polymorphisms may be observed by differences in proteins, protein modifications, RNA expression modification, DNA and RNA methylation, regulatory factors that alter gene expression and DNA replication, and any other manifestation of alterations in genomic nucleic acid or organelle nucleic acids.

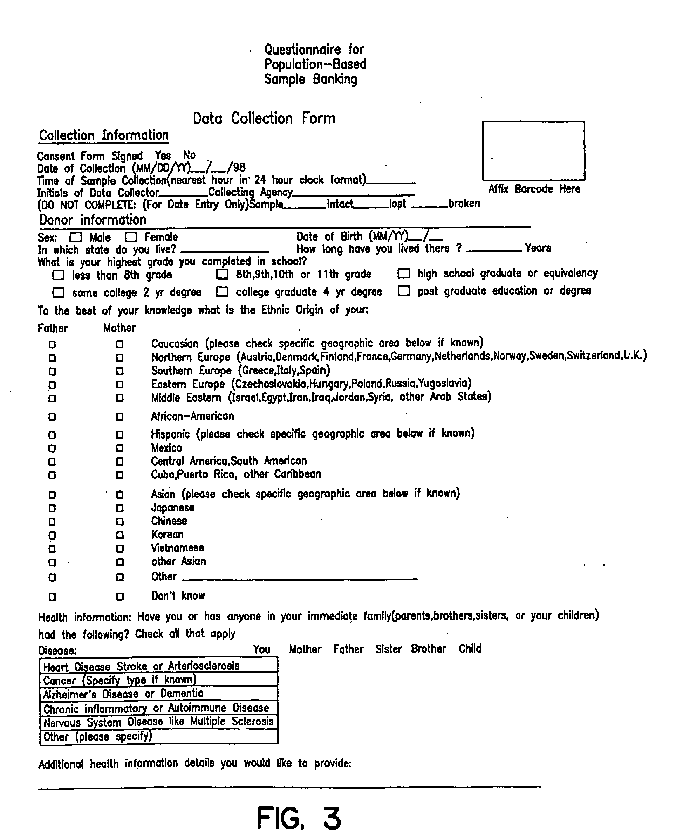

As used herein, a healthy population, refers to a population of organisms, including but are not limited to, animals, bacteria, viruses, parasites, plants, eubacteria, and others, that are disease free. The concept of disease-free is a function of the selected organism. For example, for mammals it refers to a subject not manifesting any disease state. Practically a healthy subject, when human, is defined as human donor who passes blood bank criteria to donate blood for eventual use in the general population. These criteria are as follows: free of detectable viral, bacterial, mycoplasma, and parasitic infections; not anemic; and then further selected based upon a questionnaire regarding history (see Figure 3). Thus, a healthy population represents an unbiased population of sufficient health to donate blood according to blood bank criteria, and not further selected for any disease state. Typically such individuals are not taking any medications. For plants, for example, it is a plant population that does not manifest diseases pathology associated with plants. For bacteria it is a bacterial population replicating without environmental stress, such as selective agents, heat and other pathogens.

As used herein, a healthy database (or healthy patient database) refers to a database of profiles of subjects that have not been pre-selected for any particular disease. Hence, the subjects that serve as the source of data for the database are selected, according to predetermined criteria, to be healthy. In contrast to other such databases that have been pre-selected for subjects with a particular disease or other characteristic, the subjects for the database provided herein are not so-selected. Also, if the subjects do manifest a disease or other condition, any polymorphism discovered or characterized should be related to an independent disease or condition. In a preferred embodiment, where the subjects are human, a healthy subject manifests no disease symptoms and meets criteria, such as those set by blood banks for blood donors.

Thus, the subjects for the database are a population of any organism, including, but are not limited to, animals, plants, bacteria, viruses, parasites and any other organism or entity that has nucleic acid. Among preferred subjects are mammals, preferably, although not necessarily, humans. Such a database can capture the diversity of the a population, thus providing for discovery of rare polymorphisms.

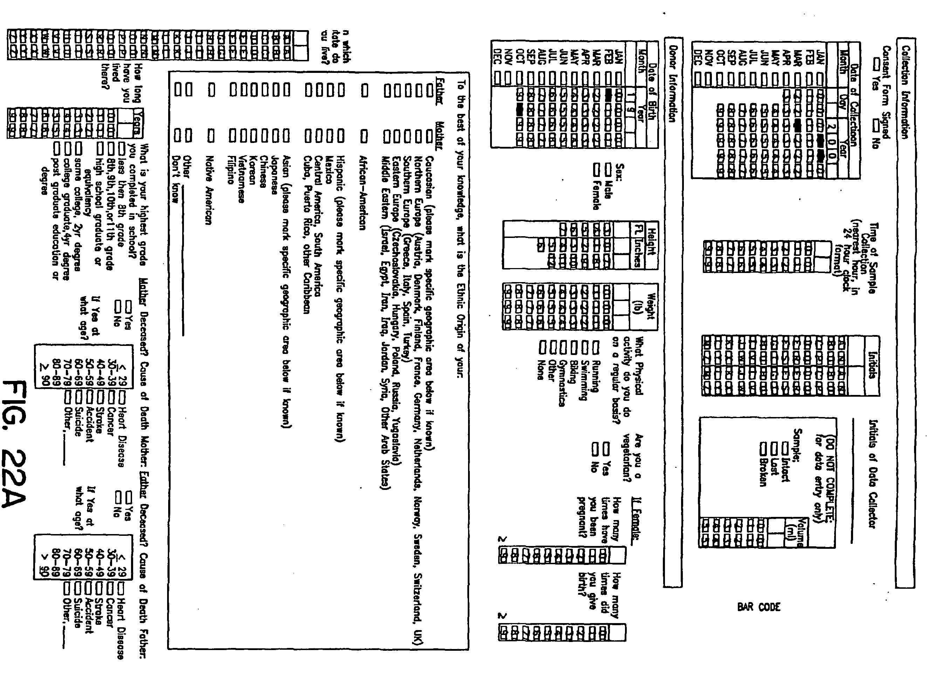

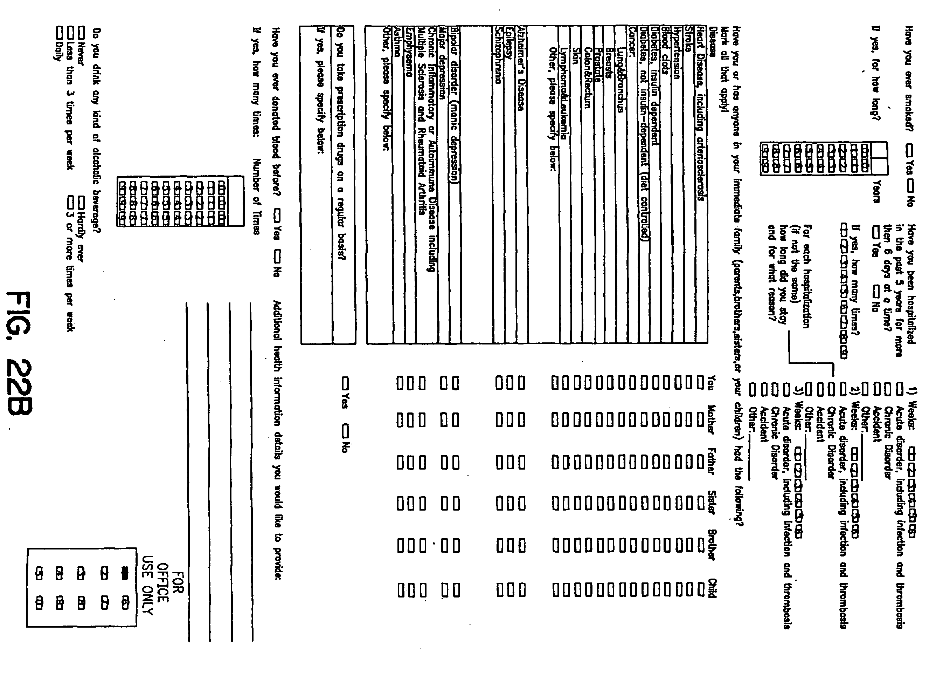

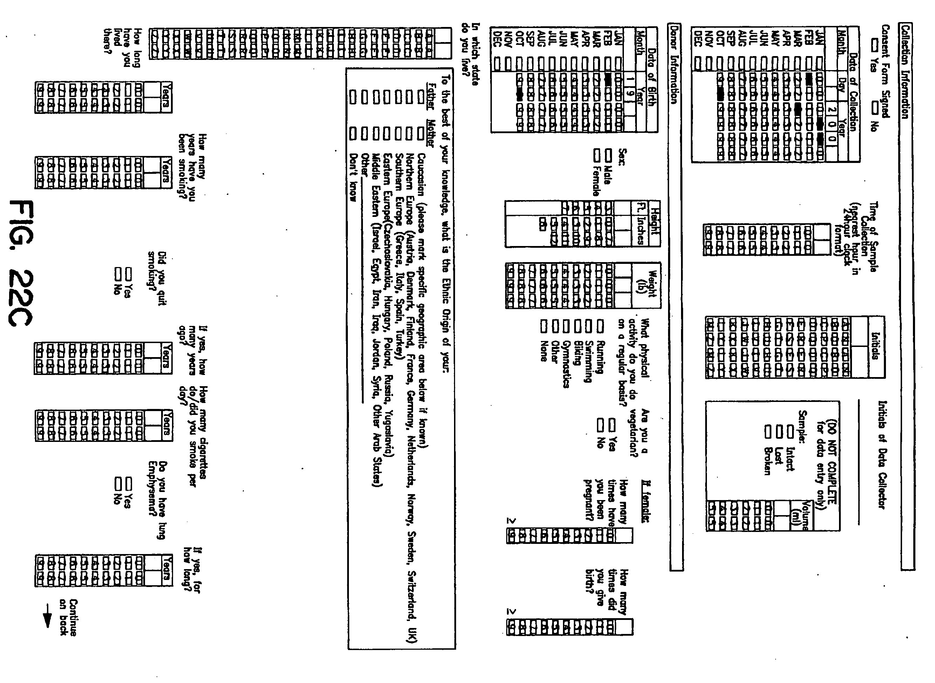

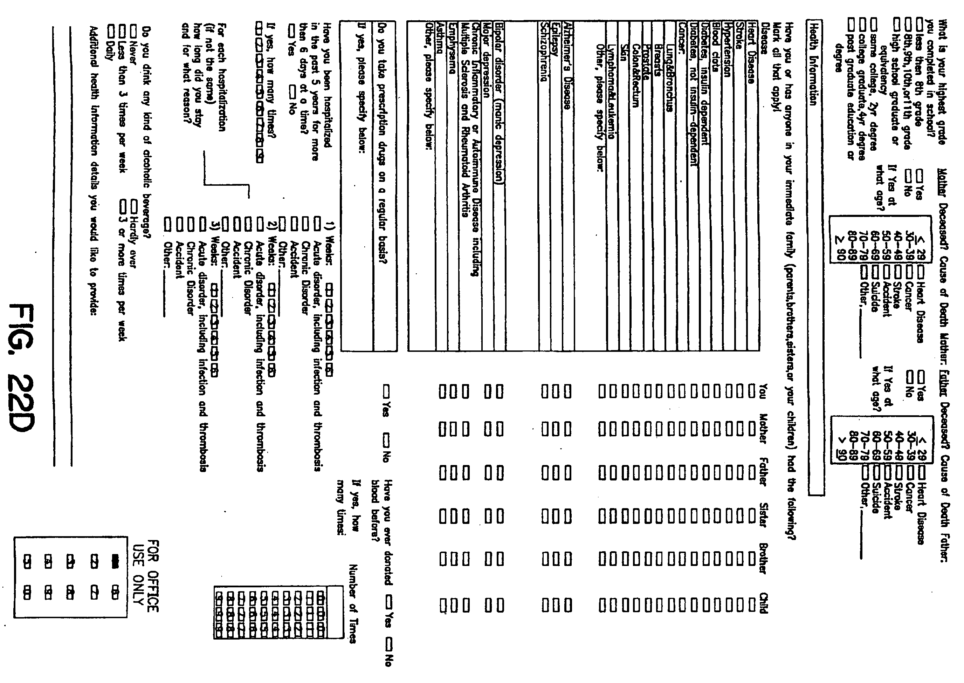

As used herein, a profile refers to information relating to, but not limited to and not necessarily including all of, age, sex, ethnicity, disease history, family history, phenotypic characteristics, such as height and weight and other relevant parameters. A sample collect information form is shown in Figure 22, which illustrates profile intent.

As used herein, a disease state is a condition or abnormality or disorder that may be inherited or result from environmental stresses, such as toxins, bacterial, fungal and viral infections.

As used herein, set of non-selected subjects means that the subjects have not been pre-selected to share a common disease or other characteristic. They can be selected to be healthy as defined herein.

As used herein, a phenotype refers to a set of parameters that includes any distinguishable trait of an organism. A phenotype can be physical traits and can be, in instances in which the subject is an animal, a mental trait, such as emotional traits. Some phenotypes can be determined by observation elicited by questionnaires (see, *e.g.*, Figures 3 and 22) or by referring to prior medical and other records. For purposes herein, a phenotype is a parameter around which the database can be sorted.

As used herein, a parameter is any input data that will serve as a basis for sorting the database. These parameters will include phenotypic traits, medical histories, family histories and any other such information elicited from a subject or observed about the subject. A parameter may describe the subject, some historical or current environmental or social influence experienced by the subject, or a condition or environmental influence on someone related to the subject. Paramaters include, but are not limited to, any of those described herein, and known to those of skill in the art. '

As used herein, haplotype refers referes to two or polymorphism located on a single DNA strand. Hence, haplotyping refers to identification of two or more polymorphisms on a single DNA strand. Haplotypes can be indicative of a phenotype. For some disorders a single polymorphism may suffice to indicate a trait; for others a plurality (i.e., a haplotype) may be needed. Haplotyping can be performed by isolating nucleic acid and separating the strands. In addition, when using enzymes such a certain nucleases, that produce, different size fragments from each strand, strand separation is not needed for haplotyping.

As used herein, used herein, pattern with reference to a mass spectrum or mass spectrometric analyses, refers to a characteristic distribution and number of signals (such peaks or digital representations thereof).

As used herein, signal in the context of a mass spectrum and analysis thereof refers to the output data, which the number or relative number of moleucles having a particular mass. Signals include "peaks" and digital representations thereof.

As used herein, adaptor, when used with reference to haplotyping use Fen ligase, refers to a nucleic acid that specifically hybridizes to a polymorphism of insterest. An adaptor can be partially double-stranded. An adaptor complex is formed when an adaptor hybridizes to its target.

As used herein, a target nucleic acid refers to any nucleic acid of interest in a sample. It can contain one or more nucleotides.

As used herein, standardless analysis refers to a determination based upon an internal standard. For example, the frequency of a polymorphism can be determined herein by comparing signals within a single mass spectrum.

As used herein, amplifying refers to means for increasing the amount of a bipolymer, especially nucleic acids. Based on the 5' and 3' primers that are chosen, amplication also serves to restrict and define the region of the genome which is subject to analysis. Amplification can be by any means known to those skilled in the art, including use of the polymerase chain reaction (PCR) etc. Amplification, e.g., PCR must be done quantitatively when the frequency of polymorphism is required to be determined.

As used herein, cleaving refers to non-specific and specific fragmentation of a biopolymer.

As used herein, multiplexing refers to the simultaneous detection of more than one polymorphism. Methods for performing multiplexed reactions, particularly in conjunction with mass spectrometry are known (see, *e.g.*, U.S. Patent Nos. 6,043,031, 5,547,835 and International PCT application No. WO 97/37041).

As used herein, reference to mass spectrometry encompasss any suitable mass spectrometric format known to those of skill in the art. Such formats cinlude, but are not limited to, Matrix-Assisted Laser Desorption/Ionization, Time-of-Flight (MALDI-TOF), Electrospray (ES), IR-MALDI (see, *e.g.*, published International PCT application No.99/57318 and U.S. Patent No. 5,118,937), Ion Cyclotron Resonance (ICR), Fourier Transform and combinations thereof. MALDI, particular UV and IR, are among the preferred formats.

As used herein, mass spectrum refers to the presentation of data obtained from analyzing a biopolymer or fragment thereof by mass spectrometry either graphically or encoded numerically.

As used herein, a blood component is a component that is separated from blood and includes, but is not limited to red blood cells and platelets, blood clotting factors, plasma, enzymes, plasminogen, immunoglobulins. A cellular blood component is a component of blood, such as a red blood cell, that is a cell. A blood protein is a protein that is normally found in blood. Examples of such proteins are blood factors VII and VIII. Such proteins and components are well-known to those of skill in the art.

As used herein, plasma can be prepared by any method known to those of skill in the art. For example, it can be prepared by centrifuging blood at a force that pellets the red cells and forms an interface between the red cells and the buffy coat, which contains leukocytes, above which is the plasma. For example, typical platelet concentrates contain at least about 10% plasma.

Blood may be separated into its components, including, but not limited to, plasma, platelets and red blood cells by any method known to those of skill in the art. For example, blood can be centrifuged for a sufficient time and at a sufficient acceleration to form a pellet containing the red blood cells. Leukocytes collect primarily at the interface of the pellet and supernatant in the buffy coat region. The supernatant, which contains plasma, platelets, and other blood components, may then be removed and centrifuged at a higher acceleration, whereby the platelets pellet.

As used herein, p53 is a cell cycle control protein that assesses DNA damage and acts as a transcription factor regulation gene which control cell growth, DNA repair and apoptosis. The p53 mutations have been found in a wide variety of different cancers, including all of the different types of leukemia, with varying frequency. The loss of normal p53 functions results in genomic instability and uncontrolled growth of the host cell.

As used herein, p21 is a cyclin-dependent kinase inhibitor, associated with G1 phase arrest of normal cells. Expression triggers apoptosis or programmed cell death and has been associated with Wilms' tumor, a pediatric kidney cancer.

As used herein, Factor VII is a serine protease involved the extrinsic blood coagulation cascade. This factor is activated by thrombin and works with tissue factor (Factor III) in the processing of Factor X to Factor Xa. Evidence has supported an association between polymorphisms in the gene and increase Factor VII activity which can result in an elevated risk of ischemic cardiovascular disease including myocardial infarction.

As used herein, a relational database stores information in a form representative of matrices, such as two-dimensional tables, including rows and columns of data, or higher dimensional matrices. For example, in one embodiment, the relational database has separate tables each with a parameter. The tables are linked with a record number, which also acts as an index. The database can be searched or sorted by using data in the tables and is stored in any suitable storage medium, such as floppy disk, CD rom disk, hard drive or other suitable medium.

As used herein, a bar codes refers any array of optically readable marks of any desired size and shape that are arranged in a reference context or frame of, preferably, although not necessarily, one or more columns and one or more rows. For purposes herein, the bar code refers to any symbology, not necessary "bar" but may include dots, characters or any symbol or symbols.

As used herein, symbology refers to an identifier code or symbol, such as a bar code, that is linked to a sample. The index will reference each such symbology. ' The symbology is any code known or designed by the user. The symbols are associated with information stored in the database. For example, each sample can be uniquely identified with an encoded symbology. The parameters, such as the answers to the questions and subsequent genotypic and other information obtained upon analysis of the samples is included in the database and associated with the symbology. The database is stored on any suitable recording medium, such as a hard drive, a floppy disk, a tape, a CD ROM, a DVD disk and any other suitable medium.

### DATABASES

Human genotyping is currently dependent on collaborations with , hospitals, tissues banks and research institutions that provide samples of disease tissue. This approach is based on the concept that the onset and/or progression of diseases can be correlated with the presence of a polymorphisms or other genetic markers. This approach does not consider that disease correlated with the presence of specific markers and the absence of specific markers. It is shown herein that identification and scoring of the appearance and disappearance of markers is possible only if these markers are measured in the background of healthy subjects where the onset of disease does not mask the change in polymorphism occurrence. Databases of information from disease populations suffer from small sample size, selection bias and heterogeneity. The databases provided herein, but which do not form part of the invention, from healthy populations solve these problems by permitting large sample bands, simple selection methods and diluted heterogeneity.

Discussed herein are first databases of parameters, associated with non-selected, particularly healthy, subjects. Also discussed are combinations of the databases with indexed samples obtained from each of the subjects. Further discussed are databases produced from the first databases. These contain in addition to the original parameters information, such as genotypic information, including, but are not limited to, genomic sequence information, derived from the samples.

The databases, which are herein designated healthy databases, are so-designated because they are not obtained from subjects pre-selected for a particular disease. Hence, although individual members may have a disease, the collection of individuals is not selected to have a particular disease.

The subjects from whom the parameters are obtained comprise either a set of subjects who are randomly selected across, preferably, all populations, or are pre-selected to be disease-free or healthy. As a result, the database is not selected to be representative of any pre-selected phenotype, genotype, disease or other characteristic. Typically the number of subjects from which the database is prepared is selected to produce statistically significant results when used in the methods provided herein. Preferably, the number of subjects will be greater than 100, more preferably greater than 200, yet more preferably greater than 1000. The precise number can be empirically determined based upon the frequency of the parameter(s) that be used to sort the database. Generally the population can have at least 50, at least 100, at least 200, at least 500, at least 1000, at least 5000 or at least 10,000 or more subjects.

Upon identification of a collection of subjects, information about each subject is recorded and associated with each subject as a database. The information associated with each of the subjects, includes, but is not limited to, information related to historical characteristics of the subjects, phenotypic characteristics and also genotypic characteristics, medical characteristics and any other traits and characteristics about the subject that can be determined. This information will serve as the basis for sorting the database.

In an example the subjects are mammals, such as humans, and the information relates to one or more of parameters, such as age, sex, medical history, ethnicity and any other factor. Such information, when the animals are humans, for example, can be obtained by a questionnaire, and by observations about the individual, such as hair color, eye color and other characteristics. Genotypic information will be obtained from tissue or other body and body fluid samples from the subject.

The healthy genomic database can include profiles and polymorphisms from healthy individuals from a library of blood samples where each sample in the library is an individual and separate blood or other tissue sample. Each sample in the database is profiled as to the sex, age, ethnic group, and disease history of the donor.

The databases are generated by first identifying healthy populations of subjects and obtaining information about each subject that will serve as the sorting parameters for the database. This information is preferably entered into a storage medium, such as the memory of a computer.

The information obtained about each subject in a population used for generating the database is stored in a computer memory or other suitable storage medium. The information is linked to an identifier associated with each subject. Hence the database will identify a subject, for example by a datapoint representative of a bar code, and then all information, such as the information from a questionnaire, regarding the individual is associated with the datapoint. As the information is collected the database is generated.

Thus, for example, profile information, such as subject histories obtained from questionnaires, is collected in the database. The resulting database can be sorted as desired, using standard software, such as by age, sex and/or ethnicity. An exemplary questionnaire for subjects from whom samples are to be obtained is shown in Figures 22A-D. Each questionnaire preferably is identified by a bar code, particularly a machine readable bar code for entry into the database. After a subject provides data and is deemed to be healthy (*i.e.*, meets standards for blood donation), the data in the questionnaire is entered into the database and is associated with the bar code. A tissue, cell or blood sample is obtained from the subject.

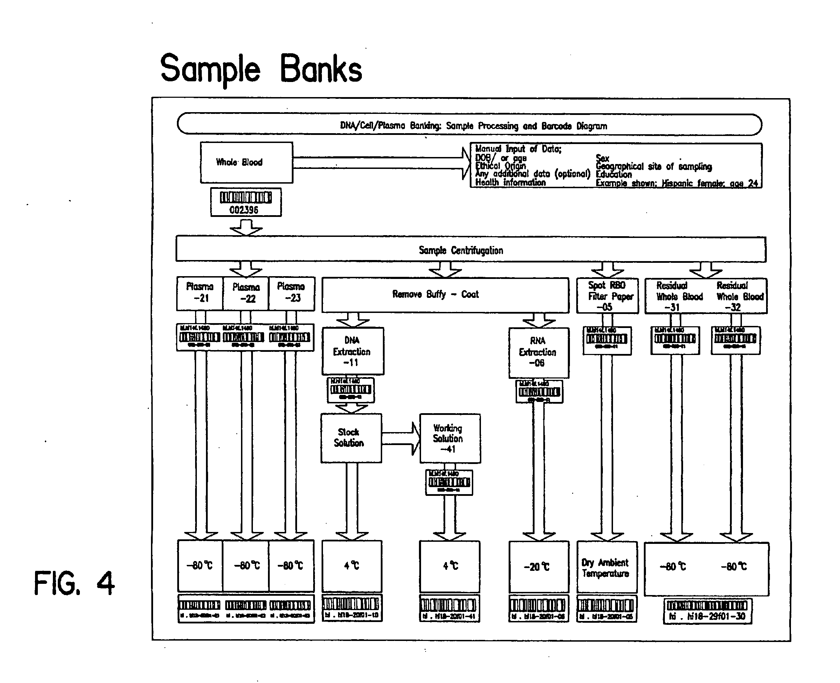

Figure 4 exemplifies processing and tracking of blood sample components. Each component is tracked with a bar code, dated, is entered into the database and associated with the subject and the profile of the subject. Typically, the whole blood is centrifuged to produce plasma, red blood cells (which pellet) and leukocytes found in the buffy coat which layers in between. Various samples are obtained and coded with a bar code and stored for use as needed.

Samples are collected from the subjects. The samples include, but are not limited to, tissues, cells, and fluids, such as nucleic acid, blood, plasma, amniotic fluid, synovial fluid, urine, saliva, aqueous humor, sweat, sperm samples and cerebral spinal fluid. It is understood that the particular set of samples depends upon the organisms in the population.

Once samples are obtained the collection can be stored and, in preferred embodiments, each sample is indexed with an identifier, particularly a machine readable code, such as a bar code. For analyses, the samples or components of the samples, particularly biopolymers and small molecules; such as nucleic acids and/or proteins and metabolites, are isolated.

After samples are analyzed, this information is entered into the database in the memory of the storage medium and associated with each subject. This information includes, but is not limited to, genotypic information. Particularly, nucleic acid sequence information and other information indicative of polymorphisms, such as masses of PCR fragments, peptide fragment sequences or masses, spectra of biopolymers and small molecules and other indicia of the structure or function of a gene, gene product or other marker from which the existence of a polymorphism within the population can be inferred.

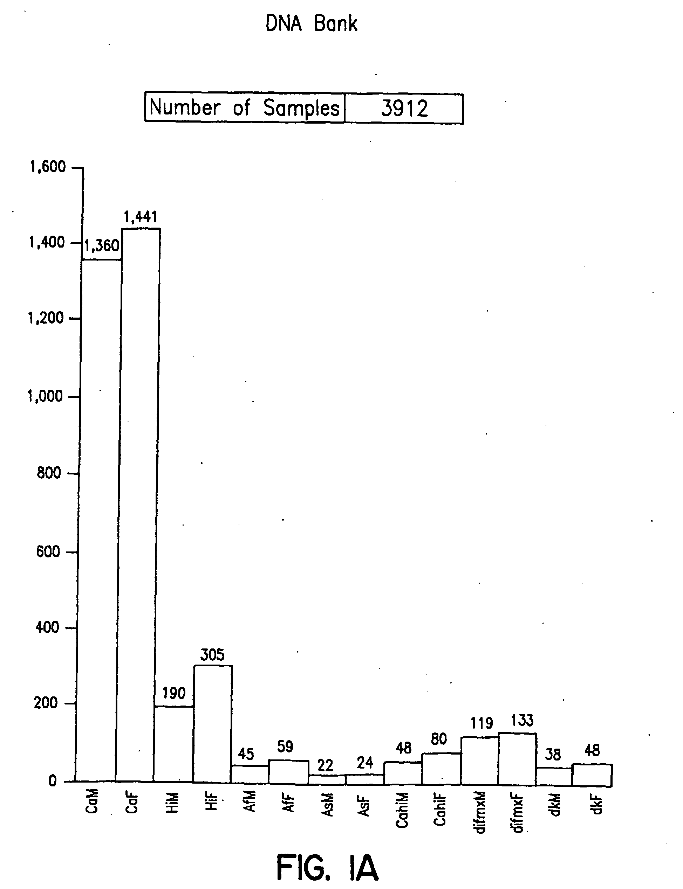

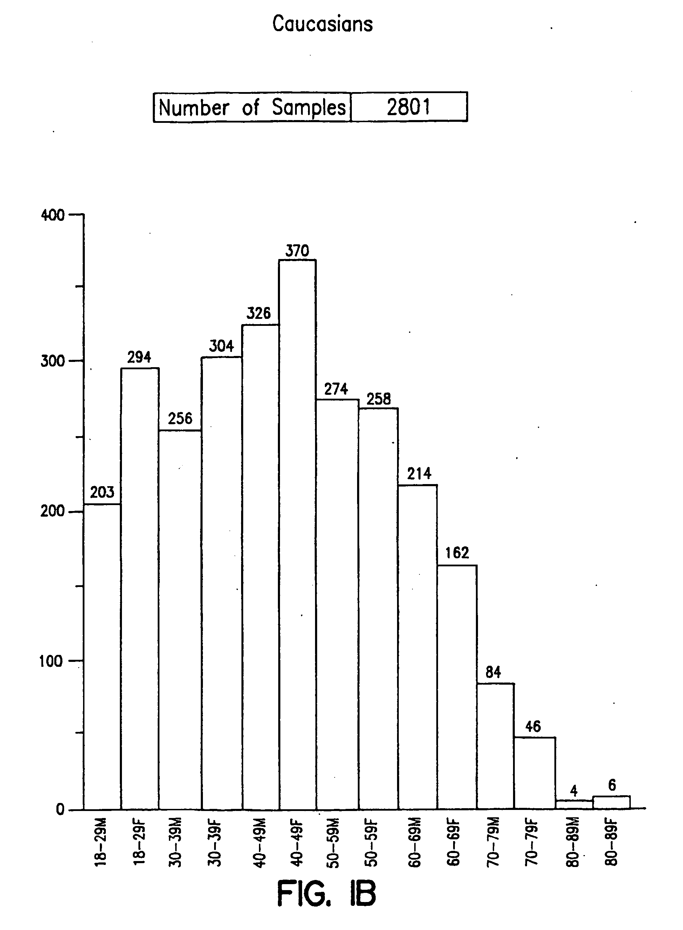

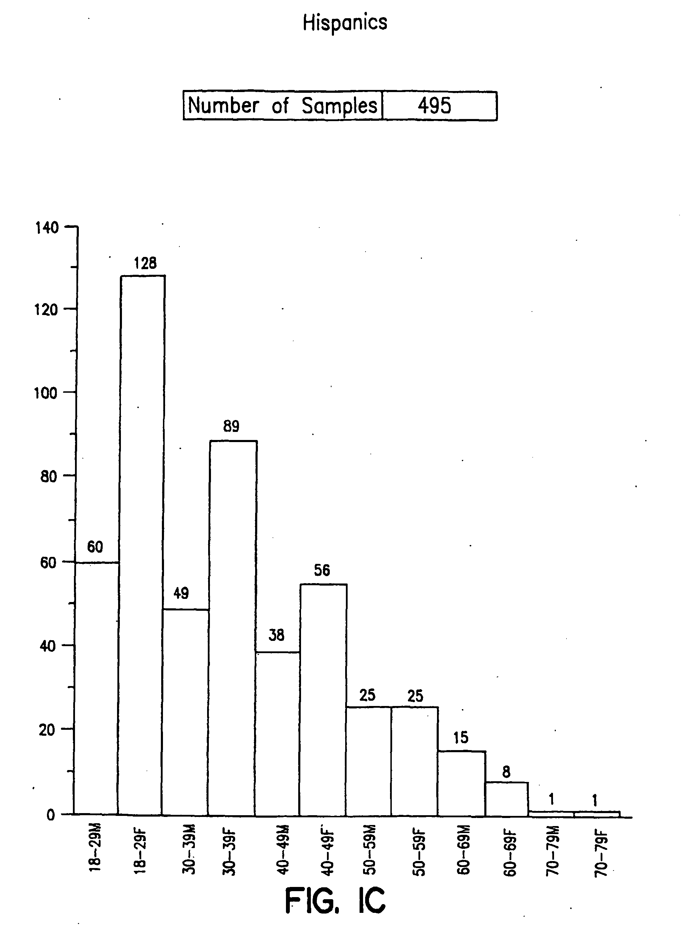

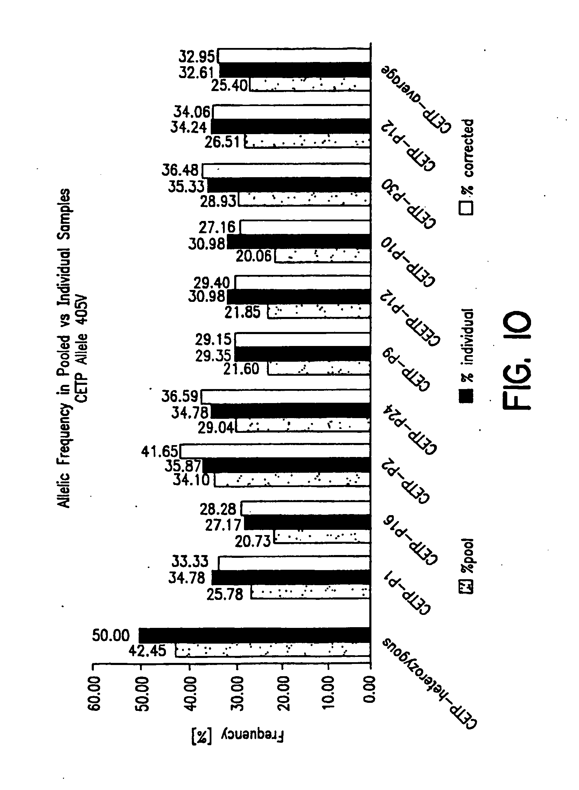

In one example, a database can be derived from a collection of blood samples. For example, Figure 1 (see, also Figure 10) shows the status of a collection of over 5000 individual samples. The samples were processed in the laboratory following SOP (standard operating procedure) guidelines. Any standard blood processing protocol may be used.

For the exemplary database described herein, the following criteria were used to select subjects:
No testing is done for infectious agents.
Age: At least 17 years old
Weight: Minimum of 110 pounds
Permanently Disqualified:
History of hepatitis (after age 11)
Leukemia Lymphoma
Human immunodeficiency virus (HiV),AIDS
Chronic kidney disease
Temporarily Disqualified:
Pregnancy - until six weeks after delivery, miscarriage or abortion
Major surgery or transfusions - for one year
Mononucleosis - until complete recovery
Prior whole blood donation - for eight weeks
Antibiotics by injection for one week; by mouth, for forty-eight hours, except antibiotics for skin complexion;
5 year Deferment:
Internal cancer and skin cancer if it has been removed, is healed and there is no recurrence
These correspond to blood bank criteria for donating blood and represent a healthy population as defined herein for a human healthy database.

### Structure of the database

Any suitable database structure and format known to those of skill in the art may be employed. For example, a relational database is a preferred format in which data is stored as matrices or tables of the parameters linked by an indexer that identifies each subject. Software for preparing and manipulating, including sorting the database, can be readily developed or adapted from commercially available software, such as Microsoft Access.

### Quality control

Quality control procedures can be implemented. For example, after collection of samples, the quality of the collection in the bank can be assessed. For example, mix-up of samples can be checked by testing for known markers, such as sex. After samples are separated by ethnicity, samples are randomly tested for a marker associated with a particular ethnicity, such as HLA DQA1 group specific component, to assess whether the samples have been properly sorted by ethnic group. An exemplary sample bank is depicted in Figure 4.

### Obtaining genotypic data and other parameters for the database

After informational and historical parameters are entered into the database, material from samples obtained from each subject, is analyzed. Analyzed material include proteins, metabolites, nucleic acids, lipids and any other desired constituent of the material. For example, nucleic acids, such as genomic DNA, can be analyzed by sequencing.

If a polymorphism is identified or known, and it is desired to assess its frequency or presence among the subjects in the database, the region of interest from each sample can be isolated, such as by PCR or restriction fragments, hybridization or other suitable method known to those of skill in the art and sequenced. For purposes herein, sequencing analysis is effected using mass spectrometry (see, *e.g.*, U.S. Patent Nos. 5,547,835, 5,622,824, 5,851,765, and 5,928,906).

In other detection methods, it is necessary to first amplify prior to identifying the allelic variant. Amplification can be performed, e.g., by PCR and/or LCR, according to methods known in the art. In one embodiment, genomic DNA of a cell is exposed to two PCR primers and amplification for a number of cycles sufficient to produce the required amount of amplified DNA. In preferred embodiments, the primers are located between 150 and 350 base pairs apart.

Alternative amplification methods include: self sustained sequence replication (Guatelli, J. C. et al., 1990, Proc. Natl. Acad. Sci. U.S.A. 87:1874-18781, transcriptional amplification system (Kwoh, D. Y. et al., 1989, Proc. Natl. Acad. Sci. U.S.A. 88:1173-1177), Q-Beta Replicase (Lizardi, P. M. et al., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

Nucleic acids can also be analyzed by detection methods and protocols, particularly those that rely on mass spectrometry (see, *e.g.*, U.S. Patent No. 5,605,798, 6,043,031, 6,428,955, 6,268,131 and International PCT application No. WO 99/31273, International PCT application No. WO 98/20019). These methods can be automated (see, *e_{.}g.*, US 2002-0009394 and published International PCT application No. PCT/US00/08111, which describes an automated process line). Preferred among the methods of analysis herein are those involving the primer oligo base extension (PROBE) reaction with mass spectrometry for detection (described herein and elsewhere, see *e.g.*, U.S. Patent No. 6,043,031; 6,277,573; 6,235,478 ; 6,197,498 ; 6,258,538 and 6,428,955, , International PCT application No. PCT/US97/20444, published as International PCT application No. WO 98/20019, ; see, also US 6,723,564 , U.S. Patent No. 6,024,925, 7,285,422 and published International PCT application No. WO 98/20020)

A preferred format for performing the analyses is a chip based format in which the biopolymer is linked to a solid support, such as a silicon or silicon-coated substrate, preferably in the form of an array. More preferably, when analyses are performed using mass spectrometry, particularly MALDI, small nanoliter volumes of sample are loaded on, such that the resulting spot is about, or smaller than, the size of the laser spot. It has been found that when this is achieved, the results from the mass spectrometric analysis are quantitative. The area under the signals in the resulting mass spectra are proportional to concentration (when normalized and corrected for background). Methods for preparing and using such chips are described in U.S. Patent No. 6,024,925, 7,285,422; 7,232,688 ; 2001-0008615 ; 6,818,394 ; see, also WO 98/20020. Chips and kits for performing these analyses are commercially available from SEQUENOM under the trademark MassARRAY. MassArray relies on the fidelity of the enzymatic primer extension reactions combined with the miniaturized array and MALDI-TOF (Matrix-Assisted Laser Desorption Ionization-Time of Flight) mass spectrometry to deliver results rapidly. It accurately distinguishes single base changes in the size of DNA fragments associated with genetic variants without tags.

The methods permit quantitative determination of alleles. The areas under the signals in the mass spectra can be used for quantitative determinations. The frequency is determined from the ratio of the signal to the total area of all of the spectrum and corrected for background. This is possible because of the PROBE technology as described in the above applications

Additional methods of analyzing nucleic acids include amplification- based methods including polymerase chain reaction (PCR), ligase chain reaction (LCR), mini-PCR, rolling circle amplification, autocatalytic methods, such as those using Qβ replicase, TAS, 3SR, and any other suitable method known to those of skill in the art.

The methods described below provide ways to fragment given amplified or non-amplified nucleotide sequences thereby producing a set of mass signals when mass spectrometry is used to analyze the fragment mixtures. Amplified fragments are yielded by standard polymerase chain methods (US 4,683,195 and 4,683,202). The fragmentation method involves the use of enzymes that cleave single or double strands of DNA and enzymes that ligate DNA. The cleavage enzymes can be glycosylases, nickases, and site-specific and non site-specific nucleases with the most preferred enzymes being glycosylases, nickases, and site-specific nucleases.

### Glycosylase Fragmentation Method

DNA glycosylases specifically remove a certain type of nucleobase from a given DNA fragment. These enzymes can thereby produce abasic sites, which can be recognized either by another cleavage enzyme, cleaving the exposed phosphate backbone specifically at the abasic site and producing a set of nucleobase specific fragments indicative of the sequence, or by chemical means, such as alkaline solutions and or heat. The use of one combination of a DNA glycosylase and its targeted nucleotide would be sufficient to generate a base specific signature pattern of any given target region.

Numerous DNA glcosylases are known, For example, a DNA glycosylase can be uracil-DNA glycolsylase (UDG) , 3-methyladenine DNA glycosylase, 3-methyladenine DNA glycosylase II, pyrimidine hydrate-DNA glycosylase, FaPy-DNA glycosylase, thymine mismatch-DNA glycosylase, hypoxanthine-DNA glycosylase, 5-Hydroxymethyluracil DNA glycosylase (HmUDG), 5-Hydroxymethylcytosine DNA glycosylase, or 1,N6-ethenoadenine DNA glycosylase (see, *e.g.*, U.S. Patent Nos. 5,536,649, 5,888, 795, 5,952,176 and 6,099,553, International PCT application Nos. WO 97/03210, WO 99/54501; see, also, Eftedal et al. (1993) Nucleic Acids Res 21:2095-2101, Bjelland and Seeberg (1987) Nucleic Acids Res. 15:2787-2801, Saparbaev et al. (1995) Nucleic Acids Res. 23:3750-3755, Bessho (1999) Nucleic Acids Res. 27:979-983) corresponding to the enzyme's modified nucleotide or nucleotide analog target. A preferred glycosylase is uracil-DNA glycolsylase (UDG).

Uracil, for example, can be incorporated into an amplified DNA molecule by amplifying the DNA in the presence of normal DNA precursor nucleotides (e.g. dCTP, dATP, and dGTP) and dUTP. When the amplified product is treated with UDG, uracil residues are cleaved. Subsequent chemical treatment of the products from the UDG reaction results in the cleavage of the phosphate backbone and the generation of nucleobase specific fragments. Moreover, the separation of the complementary strands of the amplified product prior to glycosylase treatment allows complementary patterns of fragmentation to be generated. Thus, the use of dUTP and Uracil DNA glycosylase allows the generation of T specific fragments for the complementary strands, thus providing information on the T as well as the A positions within a given sequence. Similar to this, a C-specific reaction on both (complementary) strands (i.e. with a C-specific glycosylase) yields information on C as well as G positions within a given sequence if the fragmentation patterns of both amplification strands are analyzed separately. Thus, with the glycosylase method and mass spectrometry, a full series of A, C, G and T specific fragmentation patterns can be analyzed.

### Nickase Fragmentation Method

A DNA nickase, or DNase, can be used recognize and cleave one strand of a DNA duplex. Numerous nickases are known. Among these, for example, are nickase NY2A nickase and NYS1 nickase (Megabase) with the following cleavage sites:
NY2A: 5'...R AG...3'
   3'...Y TC...5' where R = A or G and Y = C or T
NYS1: 5'... CCIAIG/T1...3'
   3'... GG[T/C/A]...5'.

### Fen-Ligase Fragmentation Method

The Fen-ligase method involves two enzymes: Fen-1 enzyme and a ligase. The Fen-1 enzyme is a site-specific nuclease known as a "flap" endonuclease (US 5,843,669, 5,874,283, and 6,090,606). This enzymes recognizes and cleaves DNA "flaps" created by the overlap of two oligonucleotides hybridized to a target DNA strand. This cleavage is highly specific and can recognize single base pair mutations, permitting detection of a single homologue from an individual heterozygous at one SNP of interest and then genotyping that homologue at other SNPs occurring within the fragment. Fen-1 enzymes can be Fen-1 like nucleases e.g. human, murine, and Xenopus XPG enzymes and yeast RAD2 nucleases or Fen-1 endonucleases from, for example, *M. jannaschii, P. furiosus,* and *P. woesei.* Among preferred enzymes are the Fen-1 enzymes.

The ligase enzyme forms a phosphodiester bond between two double stranded nucleic acid fragments. The ligase can be DNA Ligase I or DNA Ligase III (see, *e.g.,* U.S. Patent Nos. US 5,506,137, 5,700,672, 5,858,705 and 5,976,806; see, also, Waga, et al. (1994) J. Biol. Chem. 269:10923-10934, Li et al. (1994) Nucleic Acids Res. 22:632-638, Arrand et al. (1986) J. Biol. Chem. 261 :9079-9082, Lehman (1974) Science 186:790-797, Higgins and Cozzarelli (1979) Methods Enzymol. 68:50-71, Lasko et al. (1990) Mutation Res. 236:277-287, and Lindahl and Barnes (1992) Ann. Rev. Biochem. 61:251-2811.

Thermostable ligase (Epicenter Technologies), where "thermostable" denotes that the ligase retains activity even after exposure to temperatures necessary to separate two strands of DNA, are among preferred ligases for use herein.

### Type lls Enzyme Fragmentation Method

Restriction enzymes bind specifically to and cleave double-stranded DNA at specific sites within or adjacent to a particular recognition sequence. These enzymes have been classified into three groups (e.g. Types I, II, and III) as known to those of skill in the art. Because of the properties of type I and type III enzymes, they have not been widely used in molecular biological applications. Thus, for this invention type 11 enzymes are preferred. Of the thousands of restriction enzymes known in the arts, there are 179 different type II specificities. Of the 179 unique type 11 restriction endonucleases, 31 have a 4-base recognition sequence, 1 have a 5-base recognition sequence, 127 have a 6-base recognition sequence, and 10 have recognition sequences of greater than six bases (US 5,604,098). Of category type II enzymes, type IIS is preferred.

Type IIS enzymes can be *Alw* XI, *Bbv* I, *Bce* 83, *Bpm* I, *Bsg* I, *Bsm* Al, *Bsm* Fl, *Bsa* I, *Bcc* I, *Bcg* I, *Ear* I, *Eco* 571, *Esp* 31, *Fau* I, *Fok* I, *Gsu* I, *Hga* I, *Mme* I, *Mbo* II, *Sap* I, and the like. The preferred type IIS enzyme is Fok I.

The Fok I enzyme endonuclease is an exemplary well characterized member of the Type IIS class (see, *e.g.*, U.S. Patent Nos. 5,714,330, 5,604,098, 5,436,150, 6,054,276 and 5,871,91 1; see, also, Szybalski et al. (1991) Gene 100:13-26, Wilson and Murray (1991) Ann. Rev. Genet. 25:585-627, Sugisaki et al. (1981) Gene 16:73-78, Podhajska and Szalski (1985) Gene 40:175-182. Fok I recognizes the sequence 5'GGATG-3' and cleaves DNA accordingly. Type IIS restriction sites can be introduced into DNA targets by incorporating the site into primers used to amplify such targets. Fragments produced by digestion with Fok I are site specific and can be analyzed by mass spectrometry methods such as MALDI-TOF mass spectrometry, ESI-TOF mass spectrometry, and any other type of mass spectrometry well known to those of skill in the art.

Once a polymorphism has been found to correlatate with a parameter such as age. The possibility of false results due to allelic dropout is examined by doing comparative PCR in an adjacent region of the genome.

### Analyses

In using the database, Haplotying is carried out. Genetic mapping can be effected using standard methods and may also require use of databases of others, such as databases previously determined to be associated with a disorder.

Exemplary analyses have been performed and these are shown in the figures, and discussed herein.

### Sample pooling

It has been found that using the databases provided herein, or any other database of such information, substantially the same frequencies that were obtained by examining each sample separately can be obtained by pooling samples, such as in batches of 10, 20, 50, 100, 200, 500, 1000 or any other number. A precise number may be determined empirically if necessary, and can be as low as 3.

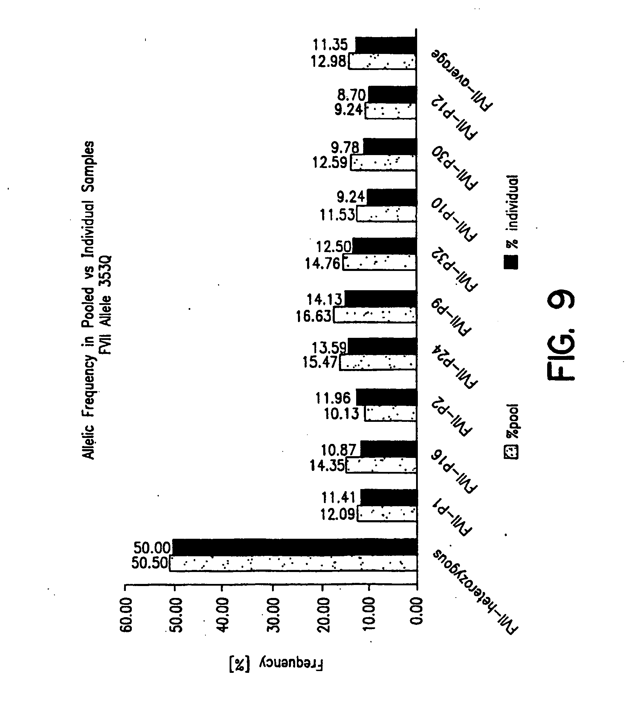

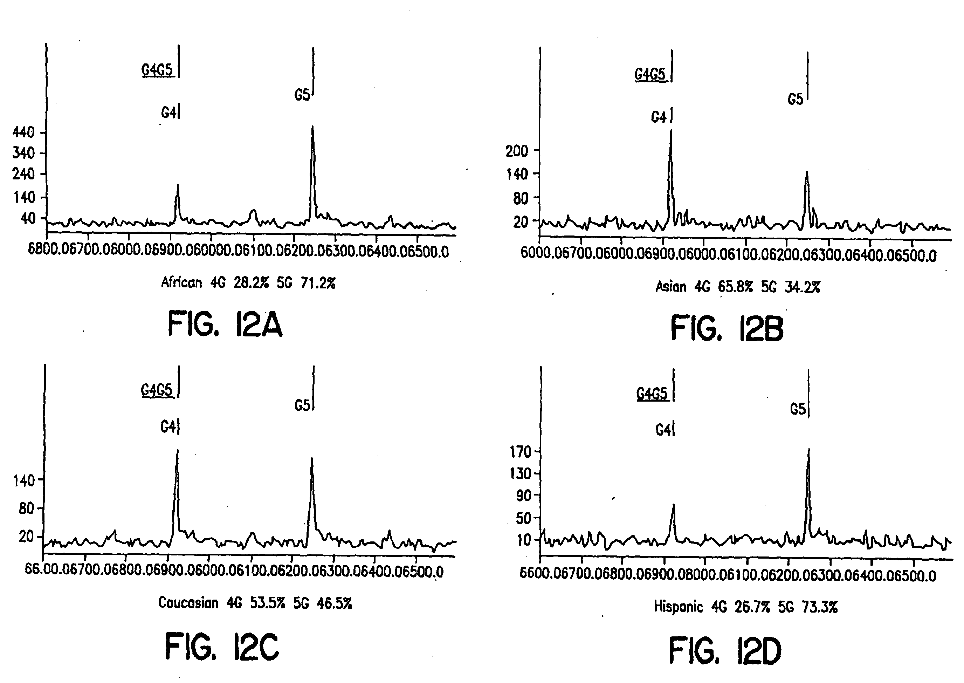

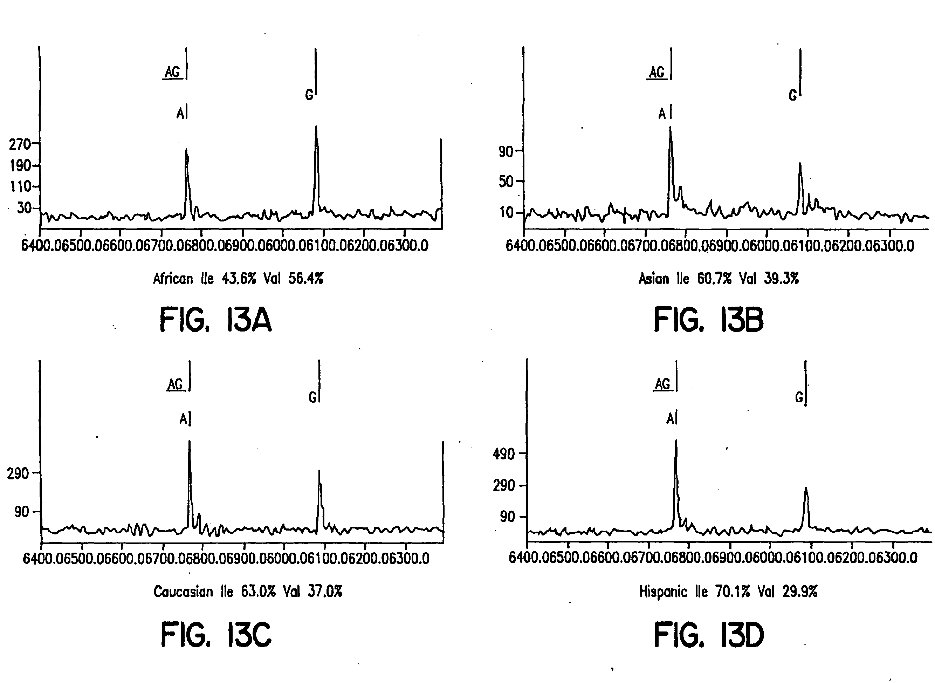

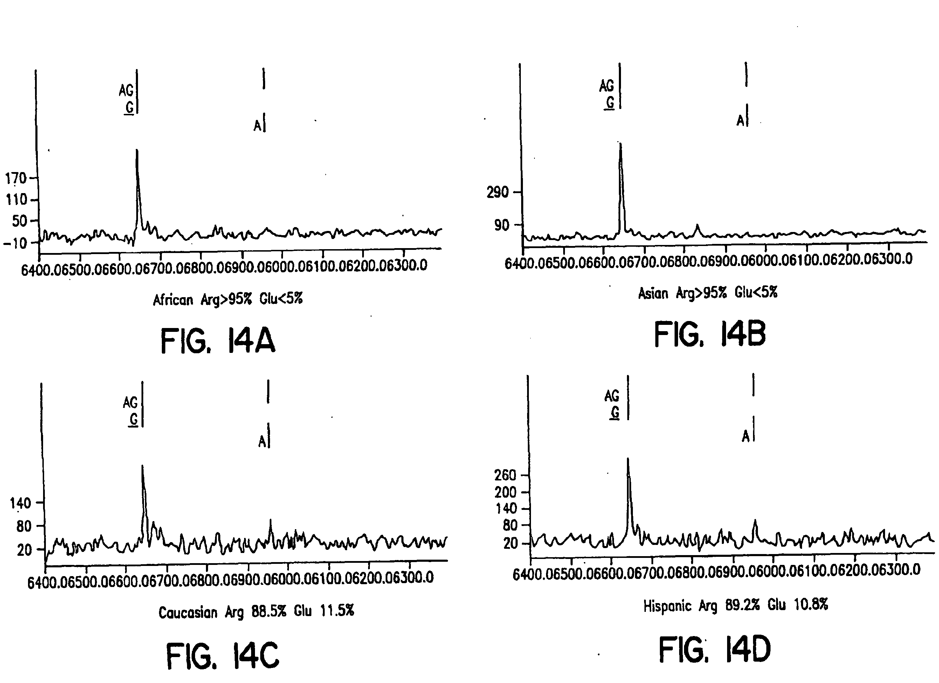

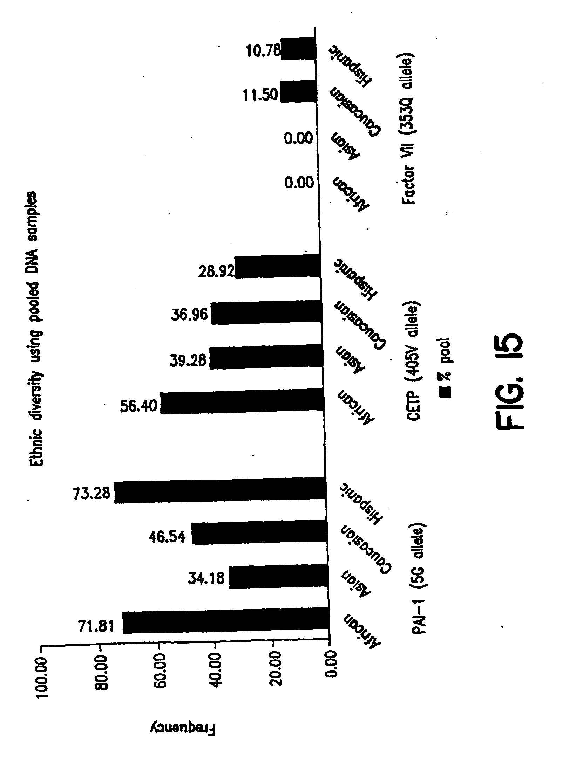

For example, the frequency of genotypic and other markers can be obtained by pooling samples. To do this a target population and a genetic variation to be assessed is selected, a plurality of samples of biopolymers are obtained from members of the population, and the biopolymer from which the marker or genotype can be inferred is determined or detected. A comparison of samples tested in pools and individually and the sorted results therefrom are shown in Figure 9, which shows frequency of the factor VII Allele 353Q. Figure 10 depicts the frequency of the CETP Allele CETP in pooled versus individual samples. Figure 15 shows ethnic diversity among various ethnic groups in the database using pooled DNA samples to obtain the data. Figures 12-14 show mass spectra for these samples.

Pooling of test samples has application not only to the healthy databases discussed herein, but also to use in gathering data for entry into any database of subjects and genotypic information, including typical databases derived from diseased populations. What is demonstrated herein, is the finding that the results achieved are statistically the same as the results that would be achieved if each sample is analyzed separately. Analysis of pooled samples by a method, such as the mass spectrometric methods provided herein, permits resolution of such data and quantitation of the results.

For factor VII the R53Q acid polymorphism was assessed. In Figure 9, the "individual" data represent allelic frequency observed in 92 individuals reactions. The pooled data represent the allelic frequency of the same 92 individuals pooled into a single probe reaction. The concentration of DNA in the samples of individual donors is 250 nanograms. The total concentration of DNA in the pooled samples, is also 250 nanograms, where the concentration of any individual DNA is 2.7 nanograms.

It also was shown that it is possible to reduce the DNA concentration of individuals in a pooled samples from 2.7 nanograms to 0.27 nanograms without any change in the quality of the spectrum or the ability to quantitate the amount of sample detected. Hence low concentrations of sample may be used in the pooling methods.

### Identification of markers and genes for disorders is of great interest.

### Model systems which do not fall within the scope of the invention

Several gene systems, p21, p53 and Lipoprotein Lipase polymorphism (N291 S), were selected. The p53 gene is a tumor suppressor gene that is mutated in diverse tumor types. One common allelic variant occurs at codon 72. A polymorphism that has been identified in the p53 gene, i.e., the R72P allele, results in an amino acid exchange, arginine to proline, at codon 72 of the gene.

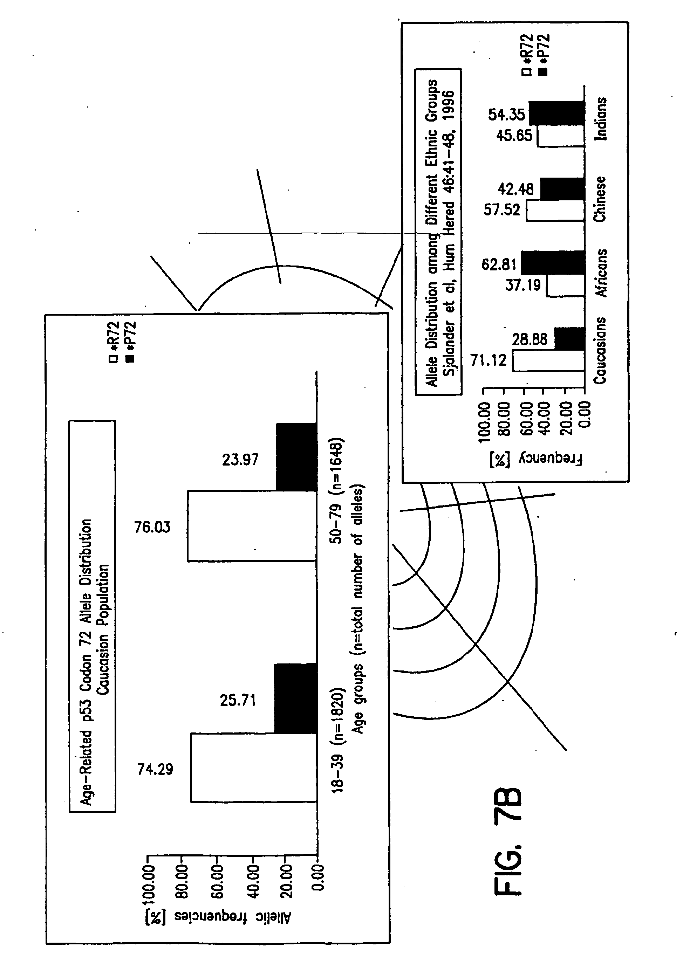

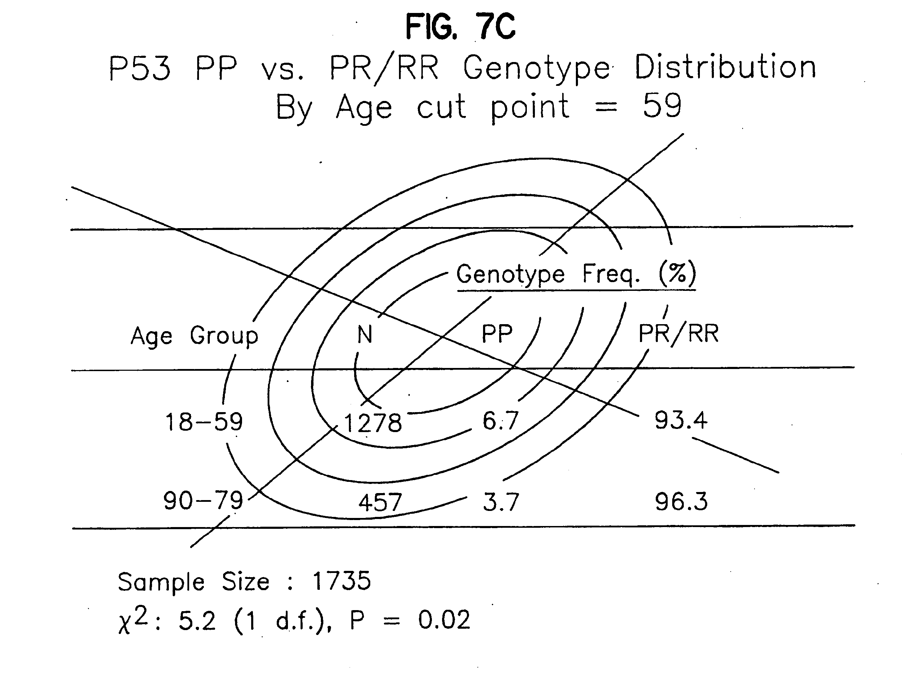

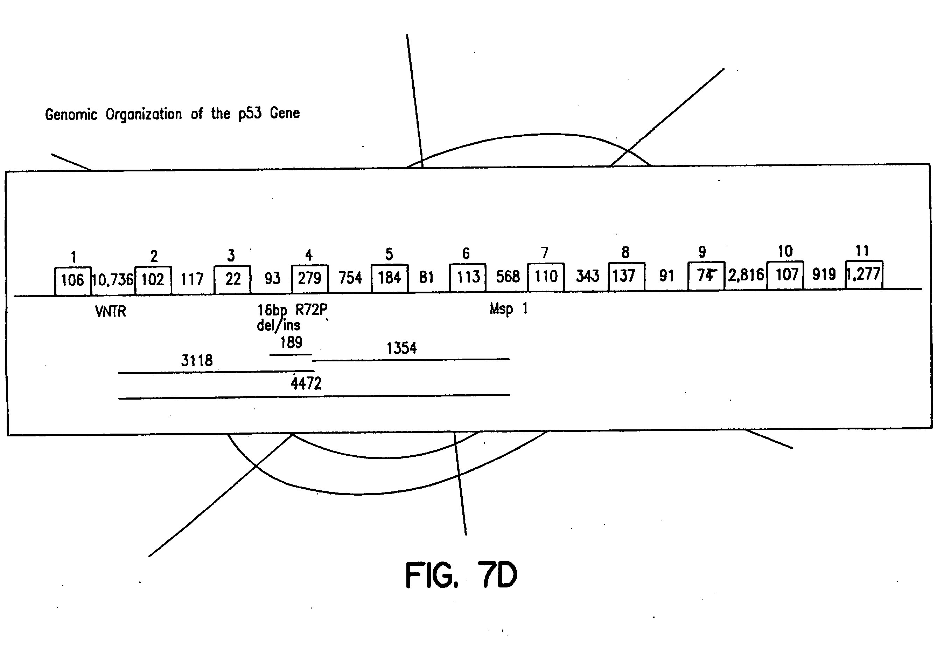

Using diseased populations, it has been shown that there are ethnic differences in the allelic distribution of these alleles among African-Americans and Caucasians in the U.S. The results here support this finding and also demonstrate that the results obtained with a healthy database are meaningful (see, Figure 7B).

The 291 S allele leads to reduced levels of high density lipoprotein cholesterol (HDL-C) that is associated with an increased risk of males for arteriosclerosis and in particular myocardial infarction (see, Reymer et al. (1995) Nature Genetics 10:28-34).

Both genetic polymorphisms were profiled within a part of the Caucasian population-based sample bank. For the polymorphism located in the lipoprotein lipase gene a total of 1025 unselected individuals (436 males and 589 female's) were tested. Genomic DNA was isolated from blood samples obtained from the individuals.

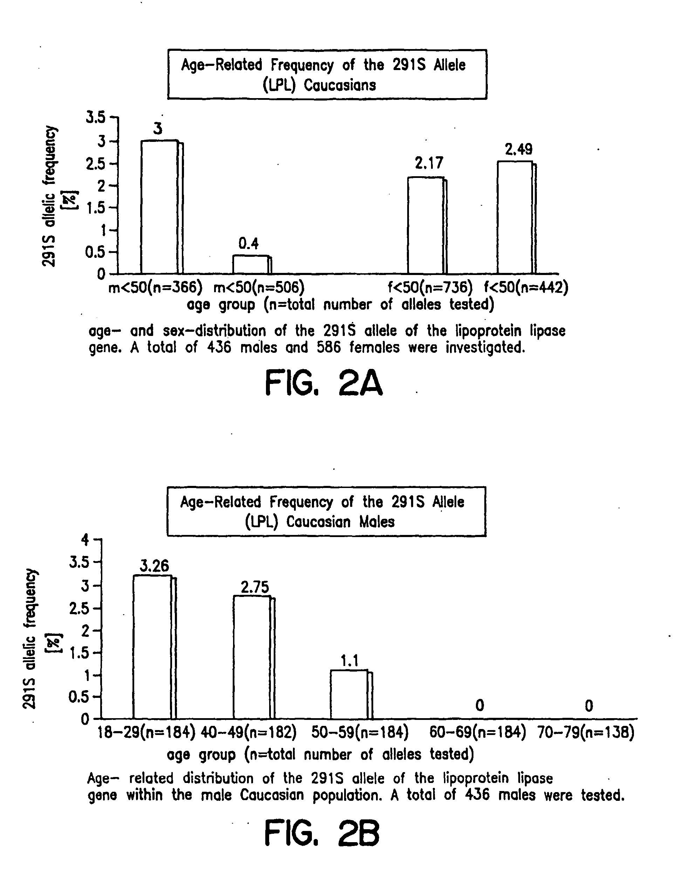

As shown in the Examples and figures, an exemplary database containing about 5000 subjects, answers to the questionnaire (see Figure 3), and genotypic information has been stratified. A particular known allele has been selected, and the samples tested for the marker using mass spectrometric analyses, particularly PROBE (see the EXAMPLES) to identify polymorphisms in each sample. The population in the database has been sorted according to various parameters and correlations have been observed. For example, FIGURES 2A-C, show sorting of the data by age and sex for the Lipoprotein Lipase gene in the Caucasian population in the database. The results show a decrease in the frequency of the allele with age in males but no such decrease in females. Other alleles that have been tested against the database, include, alleles of p53, p21 and factor VII. Results when sorted by age are shown in the figures.

These examples demonstrate an effect of altered frequency of disease causing genetic factors within the general population. The scientific interpretation of those results allows prediction of medical relevance of polymorphic genetic alterations. In addition, conclusions can be drawn with regard to their penetrance, diagnostic specificity, positive predictive value, onset of disease, most appropriate onset of preventive strategies, and the general applicability of genetic alterations identified in isolated populations to panmixed populations.

Therefore, an age- and sex-stratified population-based sample bank that is ethnically homogenous is a suitable tool for rapid identification and validation of genetic factors regarding their potential medical utility.

### Exemplary computer system for creating, storing and processing the databases Systems

Systems, including computers, containing the databases are discussed herein but do not form part of the invention. The computers and databases can be used in conjunction, for example, with the APL system (see, US 2002-0009394), which is an automated system for analyzing biopolymers, particularly nucleic acids. Results from the APL system can be entered into the database.

Any suitable computer system may be used. The computer system may be integrated into systems for sample analysis, such as the automated process line described herein (see, *e.g.*, copending U.S. application Serial No. 09/285,4811).

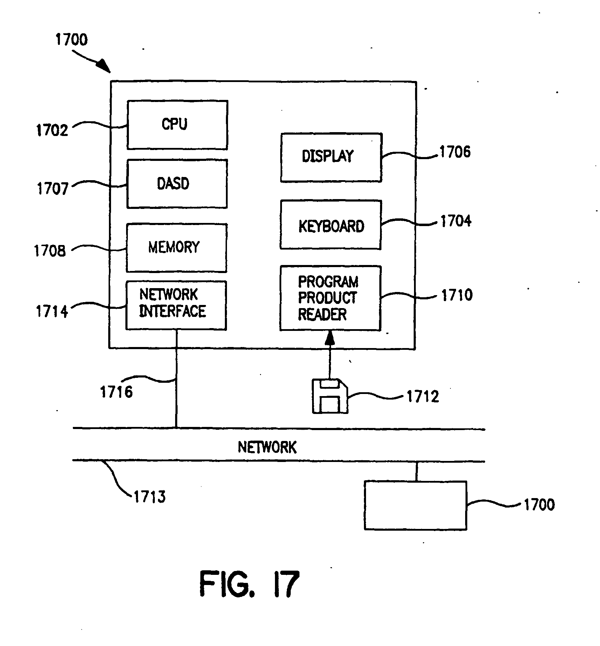

Figure 17 is a block diagram of a computer constructed in to provide and process the databases discussed herein. The processing that maintains the database and performs the methods and procedures may be performed on multiple computers all having a similar construction, or may be performed by a single, integrated computer. For example, the computer through which data is added to the database may be separate from the computer through which the database is sorted, or may be integrated with it. In either arrangement, the computers performing the processing may have a construction as illustrated in Figure 17.

Figure 17 is a block diagram of an exemplary computer 1700 that maintains the database described above and performs the methods and procedures. Each computer 1700 operates under control of a central processor unit (CPU) 1702, such as a "Pentium" microprocessor and associated integrated circuit chips, available from Intel Corporation of Santa Clara, California, USA. A computer user can input commands and data from a keyboard and display mouse 1704 and can view inputs and computer output at a display 1706. The display is typically a video monitor or flat panel display device. The computer 1700 also includes a direct access storage device (DASD) 1707, such as a fixed hard disk drive. The memory 1708 typically comprises volatile semiconductor random access memory (RAM). Each computer preferably includes a program product reader 1710 that accepts a program product storage device 1712, from which the program product reader can read data (and to which it can optionally write data). The program product reader can comprise, for example, a disk drive, and the program product storage device can comprise removable storage media such as a magnetic floppy disk, an optical CD-ROM disc, a CD-R disc, a CD-RW disc, or a DVD data disc. If desired, the computers can be connected so they can communicate with each other, and with other connected computers, over a network 1 7.1 3. Each computer 1700 can communicate with the other connected computers over the network 1713 through a network interface 177144 that enables communication over a connection 1716 between the network and the computer.

The computer 1700 operates under control of programming steps that are temporarily stored in the memory 1708 in accordance with conventional computer construction. When the programming steps are executed by the CPU 1702, the pertinent system components perform their respective functions. Thus, the programming steps implement the functionality of the system as described above. The programming steps can be received from the DASD 1707, through the program product reader 1712, or through the network connection 1716. The storage drive 1710 can receive a program product, read programming steps recorded thereon and transfer the programming steps into the memory 1708 for execution by the CPU 1702. As noted above, the program product storage device 1710 can comprise any one of multiple removable media having recorded computer-readable instructions, including magnetic floppy disks and CD-ROM storage discs. Other suitable program product storage devices can include magnetic tape and semiconductor memory chips. In this way, the processing steps necessary for operation can be embodied on a program product.

Alternatively, the program steps can be received into the operating memory 1708 over the network 1713. In the network method, the computer receives data including program steps into the memory 1708 through the network interface 1714 after network communication has been established over the network connection 1716 by well-known methods that will be understood by those skilled in the art without further explanation. The program steps are then executed by the CPU 1702 to implement the processing of the Garment Database system.

It should be understood that all of the computers of the system preferably have a construction similar to that shown in Figure 17, so that details described with respect to the Figure 17 computer 1700 will be understood to apply to all computers of the system 1700. This is indicated by multiple computers 1700 shown connected to the network 1713. Any one of the computers 1700 can have an alternative construction, so long as they can communicate with the other computers and support the functionality described herein.

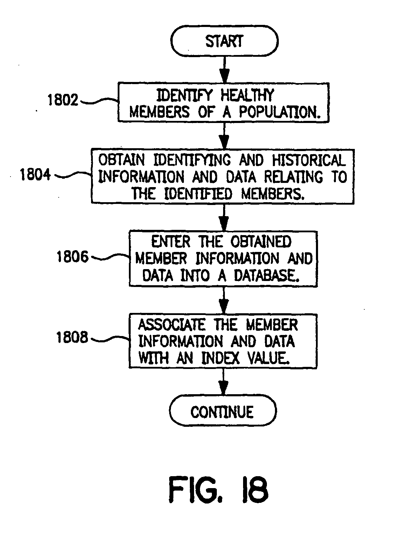

Figure 18 is a flow diagram that illustrates the processing steps performed using the computer illustrated in Figure 17, to maintain and provide access to the databases, such as for identifying polymorphic genetic markers. In particular, the information contained in the database is stored in computers having a construction similar to that illustrated in Figure 17. The first step for maintaining the database, as indicated in Figure 18, is to identify healthy members of a population. As noted above, the population members are subjects that are selected only on the basis of being healthy, and where the subjects are mammals, such as humans, they are preferably selected based upon apparent health and the absence of detectable infections. The step of identifying is represented by the flow diagram box numbered 1802.

The next step, represented by the flow diagram box numbered 1804, is to obtain identifying and historical information and data relating to the identified members of the population. The information and data comprise parameters for each of the population members, such as member age, ethnicity, sex, medical history, and ultimately genotypic information. Initially, the parameter information is obtained from a questionnaire answered by each member, from whom a body tissue or body fluid sample also is obtained. The step of entering and storing these parameters into the database of the computer is represented by the flow diagram box numbered 1806. As additional information about each population member and corresponding sample is obtained, this information can be inputted into the database and can serve as a sorting parameter.

In the next step, represented by the flow diagram box numbered 1808, the parameters of the members are associated with an indexer. This step may be executed as part of the database storage operation, such as when a new data record is stored according to the relational database structure and is automatically linked with other records according to that structure. The step 1806 also may be executed as part of a conventional data sorting or retrieval process, in which the database entries are searched according to an input search or indexing key value to determine attributes of the data. For example, such search and sort techniques may be used to follow the occurrence of known genetic markers and then determine if there is a correlation with diseases for which they have been implicated. Examples of this use are for assessing the frequencies of the p53 and Lipoprotein Lipase polymorphisms.

Such searching of the database also may be valuable for identifying one or more genetic markers whose frequency changes within the population as a function of age, ethnic group, sex, or some other criteria. This can allow the identification of previously unknown polymorphisms and, ultimately, identification of a gene or pathway involved in the onset and progression of disease.

In addition, the database can be used for taking an identified polymorphism and ascertaining whether it changes in frequency when the data is sorted according to a selected parameter.

In this way, the databases and methods provided herein permit, among other things, identification of components, particularly key components, of a disease process by understanding its genetic underpinnings, and also an understanding of processes, such as individual drug responses. The databases and methods provided herein also can be used in methods involving elucidation of pathological pathways, in developing new diagnostic assays, identifying new potential drug targets, and in identifying new drug candidates.

### Morbidity and/or early mortality associated polymorphisms

A database containing information provided by a population of healthy blood donors who were not selected for any particular disease to can be used to identify polymorphisms and the alleles in which they are present, whose frequency decreases with age. These may represent morbidity susceptibility markers and genes. This does not form part of the current invention.

Polymorphisms of the genome can lead to altered gene function, protein function or genome instability. To identify those polymorphisms which have a clinical relevance/utility is the goal of a world-wide scientific effort. It can be expected that the discovery of such polymorphisms will have a fundamental impact on the identification and development of novel drug compounds to cure diseases. However, the strategy to identify valuable polymorphisms is cumbersome and dependent upon the availability of many large patient and control cohorts to show disease association. In particular, genes that cause a general risk of the population to suffer from any disease (morbidity susceptibility genes) will escape these case/control studies entirely.

Here described is a screening strategy to identify morbidity susceptibility genes underlying a variety of different diseases. The definition of a morbidity susceptibility gene is a gene that is expressed in many different cell types or tissues (housekeeping gene) and its altered function can facilitate the expression of a clinical phenotype caused by disease-specific susceptibility genes that are involved in a pathway specific for this disorder. In other words, these morbidity susceptibility genes predispose people to develop a distinct disease according to their genetic make-up for this disease.

Candidates for morbidity susceptibility genes can be found at the bottom level of pathways involving transcription, translation, heat-shock proteins, protein trafficking, DNA repair, assembly systems for subcellular structures (e.g. mitochondria, peroxysomes and other cellular microbodies), receptor signaling cascades, immunology, etc. Those pathways control the quality of life at the cellular level as well as for the entire organism. Mutations/polymorphisms located in genes encoding proteins for those pathways can reduce the fitness of cells and make the organism more susceptible to express the clinical phenotype caused by the action of a disease-specific susceptibility gene. Therefore, these morbidity susceptibility genes can be potentially involved in a whole variety of different complex diseases if not in all. Disease-specific susceptibility genes are involved in pathways that can be considered as disease-specific pathways like glucose-, lipid, hormone metabolism, etc.

The exemplified method permit, among other things, identification of genes and/or gene products involved in a man's general susceptibility to morbidity and/or mortality; use of these genes and/or gene products in studies to elucidate the genetic underpinnings of human diseases; use of these genes and/or gene products in combinatorial statistical analyses without or together with disease-specific susceptibility genes; use of these genes and/or gene products to predict penetrance of disease susceptibility genes; use of these genes and/or gene products in predisposition and/or acute medical diagnostics and use of these genes and/or gene products to develop drugs to cure diseases and/or to extend the life span of humans.

### SCREENING PROCESS

The healthy population stratified by age, gender and ethnicity, etc. is a very efficient and a universal screening tool for morbidity associated genes. Changes of allelic frequencies in the young compared to the old population are expected to indicate putative morbidity susceptibility genes. Individual samples of this healthy population base can be pooled to further increase the throughput. In a proof of principle experiment pools not forming part of the invention of young and old Caucasian females and males were applied to screen more than 400 randomly chosen single nucleotide polymorphisms located in many different genes. Candidate polymorphisms were identified if the allelic difference was greater than 8% between young and old for both or only one of the genders. The initial results were assayed again in at least one independent subsequent experiments. Repeated experiments are necessary to recognize unstable biochemical reactions, which occur with a frequency of about 2-3% and can mimic age-related allelic frequency differences. Average frequency differences and standard deviations are calculated after successful reproducibility of initial results. The final allelic frequency is then compared to a reference population of Caucasian CEPH sample pool. The result should show similar allelic frequencies in the young Caucasian population. Subsequently, the exact allele frequencies of candidates including genotype information were obtained by analyzing all individual samples. This procedure is straight forward with regard to time and cost. It enables the screening of an enormous number of SNPs. So far, several markers with a highly significant association to age were identified and descried below.

In general at least 5 individuals in a stratified population need to be screened to produce statistically significant results. The frequency of the allele is determined for an age stratified population. Chi square analysis is then performed on the allelic frequencies to determine if the difference between age groups is statistically significant. A p value less than of 0.1 is considered to represent a statistically significant difference. More preferably the p value should be less than 0.05.

### Clinical Trials

The identification of markers whose frequency in a population decreases with age also allows for better designed and balanced clinical trials. Currently, if a clinical trial utilizes a marker as a significant endpoint in a study and the marker disappears with age, then the results of the study may be inaccurate. This information can be considered and controlled when designing the study. For, example, an age independent marker could be substituted in its place.

The following examples are included for illustrative purposes only and are not intended to limit the scope of the invention.

### COMPARATIVE EXAMPLE 1

This example which does not fall within the scope of the invention describes the use of a database containing information. provided by a population of healthy blood donors who were not selected for any particular disease to determine the distribution of allelic frequencies of known genetic markers with age and by sex in a Caucasian subpopulation of the database. The results described in this example demonstrate that a disease-related genetic marker or polymorphism can be identified by sorting a healthy database by a parameter or parameters, such as age, sex and ethnicity.

### Generating a database

Blood was obtained by venous puncture from human subjects who met blood bank criteria for donating blood. The blood samples were preserved with EDTA at pH 8.0 and labeled. Each donor provided information such as age, sex, ethnicity, medical history and family medical history. Each sample was labeled with a barcode representing identifying information. A database was generated by entering, for each donor, the subject identifier and information corresponding to that subject into the memory of a computer storage medium using commercially available software, e.g.. Microsoft Access.

### Model genetic markers

The frequencies of polymorphisms known to be associated at some level with disease were determined in a subpopulation of the subjects represented in the database. These known polymporphisms occur in the p21, p53 and Lipoprotein Lipase genes. Specifically, the N291S polymorphism (N291 S) of the Lipoprotein Lipase gene, which results in a substitution of a serine for an asparagine at amino acid codon 291, leads to reduced levels of high density lipoprotein cholesterol (HDL-C) that is associated with an increased risk of males for arteriosclerosis and in particular myocardial infarction (see, Reymer et al. (1995) Nature Genetics 10:28-34).

The p53 gene encodes a cell cycle control protein that assesses DNA damage and acts as a transcription factor regulating genes that control cell growth, DNA repair and apoptosis (programmed cell death). Mutations in the p53 gene have been found in a wide variety of different cancers, including different types of leukemia, with varying frequency. The loss of normal p53 function results in genomic instability an uncontrolled cell growth. A polymorphism that has been identified in the p53 gene, i.e., the R72P allele, results in the substitution of a proline for an arginine at amino acid codon 72 of the gene.

The p21 gene encodes a cyclin-dependent kinase inhibitor associated with G1 phase arrest of normal cells. Expression of the p21 gene triggers apoptosis. Polymorphisms of the p21 gene have been associated with Wilms' tumor, a pediatric kidney cancer. One polymorphism of the p21 gene, the S31R polymorphism, results in a substitution of an arginine for a serine at amino acid codon 31.

### Database analysis

Sorting of subjects according to specific parameters The genetic polymorphisms were profiled within segments of the Caucasian subpopulation of the sample bank. For p53 profiling, the genomic DNA isolated from blood from a total of 1277 Caucasian subjects age 18-59 years and 457 Caucasian subjects age 60-79 years was analyzed. For p21 profiling, the genomic DNA isolated from blood from a total of 910 Caucasian subjects age 18-49 years and 824 Caucasian subjects age 50-79 years was analyzed. For lipoprotein lipase gene profiling, the genomic DNA from a total of 1464 Caucasian females and 1470 Caucasian males under 60 years of age and a total of 478 Caucasian females and 560 Caucasian males over 60 years of age was analyzed.

### Isolation and analysis of genomic DNA

Genomic DNA was isolated from blood samples obtained from the individuals. Ten milliliters of whole blood from each individual was centrifuged at 2000 x g. One milliliter of the buffy coat was added to 9 ml of 155 mM NH₄Cl, 10 mM KHCO₃, and 0.1 mM Na₂EDTA, incubated 10 min at room temperature and centrifuged for 10 min at 2000 x g. The supernatant was removed, and the white cell pellet was washed in 155 mM NH₄Cl, 10 mM KHCO₃ and 0.1 mM Na₂EDTA and resuspended in 4.5 ml of 50 mM Tris, 5 mM EDTA and 1 % SDS. Proteins were precipitated from the cell lysate by 6 mM ammonium acetate, pH 7.3, and then separated from the nucleic acids by centrifugation at 3000 x g. The nucleic acid was recovered from the supernatant by the addition of an equal volume of 100% isopropanol and centrifugation at 2000 x g. The dried nucleic acid pellet was hydrated in 10 mM Tris, pH 7.6, and 1 mM Na₂EDTA and stored at 4° C.

Assays of the genomic DNA to determine the presence or absence of the known genetic markers were developed using the BiomassPROBE^{™} detection method (primer oligo base extension) reaction. This method uses a single detection primer followed by an oligonucleotide extension step to give products, which can be readily resolved by mass spectrometry, and, in particular, MALDITOF mass spectrometry. The products differ in length depending on the presence or absence of a polymorphism. In this method, a detection primer anneals adjacent to the site of a variable nucleotide or sequence of nucleotides and the primer is extended using a DNA polymerase in the presence of one or more dideoxyNTPs and, optionally, one or more deoxyNTPs. The resulting products are resolved by MALDI-TOF mass spectrometry. The mass of the products as measured by MALDI-TOF mass spectrometry makes possible the determination of the nucleotide(s) present at the variable site.

First, each of the Caucasian genomic DNA samples was subjected to nucleic acid amplification using primers corresponding to sites 5' and 3' of the polymorphic sites of the p21 (S31R allele), p53 (R72P allele) and Lipoprotein Lipase (N291 S allele) genes. One primer in each primer pair was biotinylated to permit immobilization of the amplification product to a solid support. Specifically, the polymerase chain reaction primers used for amplification of the relevant segments of the p21, p53 and lipoprotein lipase genes are shown below: US4p21c31-2F (SEQ ID NO: 9) and US5p21-2R (SEQ ID NO: 10) for p21 gene amplification; US4-p53-ex4-F (also shown as p53-ex4US4 (SEQ ID NO: 2)) and US5-p53/2-4R (also shown as US5P53/4R (SEQ ID NO: 3)) for p53 gene amplification; and US4-LPL-F2 (SEQ ID NO: 16) and US5-LPL-R2 (SEQ ID NO: 17) for lipoprotein lipase gene amplification.

Amplification of the respective DNA sequences was conducted according to standard protocols. For example, primers may be used in a concentration of 8 pmol. The reaction mixture (e.g., total volume 50 µl) may contain Taq-polymerase including 10x buffer and dTNPs. Cycling conditions for polymerase chain reaction amplification may typically be initially 5 min. at 95°C, followed by 1 min. at 94°C, 45 sec at 53°C, and 30 sec at 72°C for 40 cycles with a final extension time of 5 min at 72°C. Amplification products may be purified by using Qiagen's PCR purification kit (No. 28106) according to manufacturer's instructions. The elution of the purified products from the column can be done in 50 µl TE-buffer (10mM Tris, 1 mM EDTA, pH 7.5).

The purified amplification products were immobilized via a biotin-avidin linkage to streptavidin-coated beads and the double-stranded DNA was denatured. A detection primer was then annealed to the immobilized DNA using conditions such as, for example, the following: 50 µl annealing buffer (20 mM Tris, 10 mM KCl, 10 mM (NH₄)₂SO₄, 2 mM MgSO₂, 1 % Triton X-100, pH 8) at 50°C for 10 min, followed by washing of the beads three times with 200 µl washing buffer (40 mM Tris, 1 mM EDTA, 50 mM NaCl, 0.1 % Tween 20, pH 8.8) and once in 200 µl TE buffer.

The PROBE extension reaction was performed, for example, by using some components of the DNA sequencing kit from USB (No. 70770) and dNTPs or ddNTPs from Pharmacia. An exemplary protocol could include a total reaction volume of 45 µl, containing of 21 µl water, 6 µl Sequenase-buffer, 3 µl 10 mM DTT solution, 4.5 µl, 0.5 mM of three dNTPs, 4.5 µl, 2 mM the missing one ddNTP, 5.5 µl glycerol enzyme dilution buffer, 0.25 µl Sequenase 2.0, and 0.25 pyrophosphatase. The reaction can then by pipetted on ice and incubated for 15 min at room temperature and for 5 min at 37 °C. The beads may be washed three times with 200 µl washing buffer and once with 60 µl of a 70 mM NH₄-Citrate solution.

The DNA was denatured to release the extended primers from the immobilized template. Each of the resulting extension products was separately analyzed by MALDI-TOF mass spectrometry using 3-hydroxypicolinic acid (3-HPA) as matrix and a UV laser.

Specifically, the primers used in the PROBE reactions are as shown below: P21/31-3 (SEQ ID NO: 12) for PROBE analysis of the p21 polymorphic site; P53/72 (SEQ ID NO: 4) for PROBE analysis of the p53 polymorphic site; and LPL-2 for PROBE analysis of the lipoprotein lipase gene polymorphic site. In the PROBE analysis of the p21 polymorphic site, the extension reaction was performed using dideoxy-C. The products resulting from the reaction conducted on a "wild-type" allele template (wherein codon 31 encodes a serine) and from the reaction conducted on a polymorphic S31 R allele template (wherein codon 31 encodes an arginine) are shown below and designated as P21 /31-3 Ser (wt) (SEQ ID NO: 13) and P21 /31-3 Arg (SEQ ID NO: 14), respectively. The masses for each product as can be measured by MALDI-TOF mass spectrometry are also provided (i.e., 4900.2 Da for the wild-type product and 5213.4 Da for the polymorphic product).

In the PROBE analysis of the p53 polymorphic site, the extension reaction was performed using dideoxy-C. The products resulting from the reaction conducted on a "wild-type" allele template (wherein codon 72 encodes an arginine) and from the reaction conducted on a polymorphic R72P allele template (wherein codon 72 encodes a proline) are shown below and designated as Cod72 G Arg (wt) and Cod72 C Pro, respectively. The masses for each product as can be measured by MALDI-TOF mass spectrometry are also provided (i.e., 5734.8 Da for the wild-type product and 5405.6 Da for the polymorphic product).

In the PROBE analysis of the lipoprotein lipase gene polymorphic site, the extension reaction was performed using a mixture of ddA and ddT. The products resulting from the reaction conducted on a "wild-type" allele template (wherein codon 291 encodes an asparagine) and from the reaction conducted on a polymorphic N291S allele template (wherein codon 291 encodes a serine) are shown below and designated as 291 Asn and 291Ser, respectively. The masses for each product as can be measured by MALDI-TOF mass spectrometry are also provided (i.e., 6438.2 Da for the wild-type product and 6758.4 Da for the polymorphic product). **P53-1 (R72P)**

PCR Product length: 407 bp (SEQ ID NO: 1)
US4-p53-ex4-F
ctg aggacctggt cctctgactg
ctcttttcac ccatctacag tcccccttgc cgtcccaagc aatggatgat ttgatgctgt
ccccggacga tattgaacaa tggttcactg aagacccagg tccagatgaa gctcccagaa
P53/72 72R
tgccagaggc tgctccccgc gtggcccctg caccagcagc tcctacaccg gcggcccctg
c 72P
caccagcccc ctcctggccc ctgtcatctt ctgtcccttc ccagaaaacc taccagggca
gctacggttt ccgtctgggc ttcttgcatt ctgggacagc caagtctgtg acttgcacgg
tcagttgccc tgaggggctg gcttccatga gacttcaa
US5-p53/2-4R

### Primers (SEQ ID NOs: 2-4)

p53-ex4FUS4 ccc agt cac gac gtt gta aaa cgc tga gga cct ggt cct ctg ac
US5P53/4R agc gga taa caa ttt cac aca ggt tga agt ctc atg gaa gcc
P53/72 gcc aga ggc tgc tcc cc

### Masses

| Allele | Product Termination: ddC | SEQ # | Length | Mass |
|---|---|---|---|---|
| P53/72 | gccagaggctgctcccc | 5 | 17 | 5132.4 |
| Cod72 G Arg (wt) | gccagaggctgctccccgc | 6 | 19 | 5734.8 |
| Cod72 C Pro | gccagaggctgctccccc | 7 | 18 | 5405.6 |

| | | | | |
|---|---|---|---|---|
| Biotinylated US5 primer is used in the PCR amplification. | | | | |

### LPL-1 (N291S)

Amino acid exchange asparagine to serine at codon 291 of the lipoprotein lipase gene.

### PCR Product length: 251 bp (SEQ ID NO: 15)

US4-LPL-F2 (SEQ ID NO: 16)

gcgctccatt catctcttca tcgactctct gttgaatgaa gaaaatccaa gtaaggccta
caggtgcagt tccaaggaag cctttgagaa agggctctgc ttgagttgta gaaagaaccg
LPL-2 291N
ctgcaacaat ctgggctatg agatcaataa agtcagagcc aaaagaagca gcaaaatgta
g 291S
cctgaagact cgttctcaga tgccc
US4-LPL-R2

### Primers (SEQ ID NOs: 16-18):

US4-LPL-F2 ccc agt cac gac gtt gta aaa cgg cgc tcc att cat ctc ttc
US5-LPL-R2 agc gga taa caa ttt cac aca ggg ggc atc tga gaa cga gtc
LPL-2 caa tct ggg cta tga gat ca

### Masses

| **Allele** | **Product Termination: ddA, ddT** | **SEQ #** | **Length** | **Mass** |
|---|---|---|---|---|
| LPL-2 | caatctgggctatgagatca | 19 | 20 | 6141 |
| 291 Asn | caatctgggctatgagatcaa | 20 | 21 | 6438.2 |
| 291 Ser | caatctgggctatgagatcagt | 21 | 22 | 6758.4 |

| | | | | |
|---|---|---|---|---|
| Biotinylated US5 primer is used in the PCR amplification. | | | | |

### P21-1 (S31R)

Amino acid exchange serine to arginine at codon 31 of the tumor suppressor gene p21. Product length: 207 bp (SEQ ID NO: 8)

gtcc gtcagaaccc atgcggcagc
p21/31-3 31S
aaggcctgcc gccgcctctt cggcccagtg gacagcgagc agctgagccg cgactgtgat
a 31R
gcgctaatgg cgggctgcat ccaggaggcc cgtgagcgat ggaacttcga ctttgtcacc
gagacaccac tggaggg
US5p21-2R

### Primers (SEQ ID NOs: 9-11)

US4p21c31-2F ccc agt cac gac gtt gta aaa cgg tcc gtc aga acc cat gcg g
US5p21-2R agc gga taa caa ttt cac aca ggc tcc agt ggt gtc tcg gtg ac
P21/31-3 cag cga gca gct gag

### Masses

| **Allele** | **Product Termination: ddC** | **SEQ #** | **Length** | **Mass** |
|---|---|---|---|---|
| p21/31-3 | cagcgagcagctgag | 12 | 15 | 4627 |
| P21/31-3 Ser (wt) | cagcgagcagctgagc | 13 | 16 | 4900.2 |
| P21/31-3 Arg | cagcgagcagctgagac | 14 | 17 | 5213.4 |

| | | | | |
|---|---|---|---|---|
| Biotinylated US5 primer is used in the PCR amplification. | | | | |

Each of the Caucasian subject DNA samples was individually analyzed by MALDI-TOF mass spectrometry to determine the identity of the nucleotide at the polymorphic sites. The genotypic results of each assay can be entered into the database. The results were then sorted according to age and/or sex to determine the distribution of allelic frequencies by age and/or sex. As depicted in the Figures showing histograms of the results, in each case, there was a differential distribution of the allelic frequencies of the genetic markers for the p21, p53 and lipoprotein lipase gene polymorphisms.

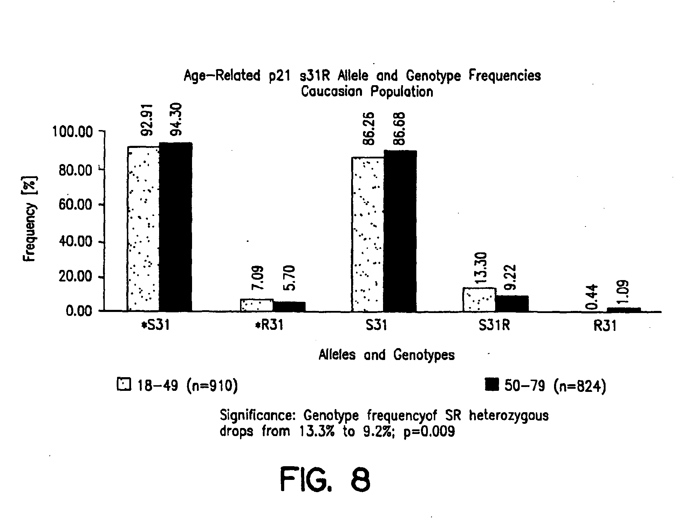

Figure 8 shows the results of the p21 genetic marker assays reveals a statistically significant decrease (from 13.3% to 9.2%) in the frequency of the heterozygous genotype (S31 R) in Caucasians with age (18-49 years of age compared to 50-79 years of age). The frequencies of the homozygous (S31 and R31) genotypes for the two age groups are also shown, as are the overall frequencies of the S31 and R31 alleles in the two age groups (designated as *S31 and *R31, respectively in the Figure).

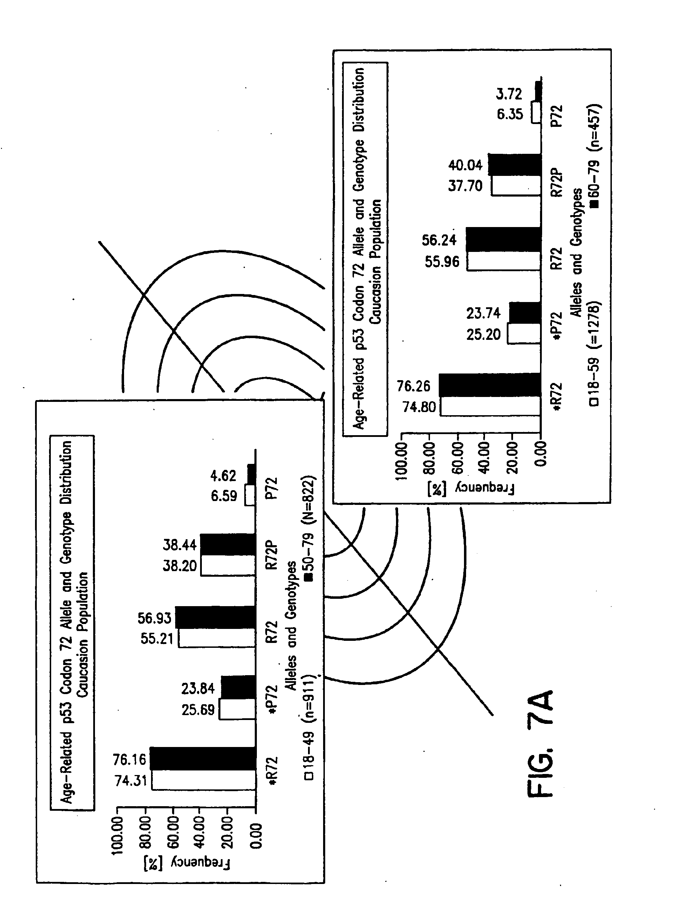

Figures 7A-C shows the results of the p53 genetic marker assays and reveals a statistically significant decrease (from 6.7% to 3.7%) in the frequency of the homozygous polymorphic genotype (P72) in Caucasians with age (18-59 years of age compared to 60-79 years of age). The frequencies of the homozygous "wild-type" genotype (R72) and the heterozygous genotype (R72P) for the two age groups are also shown, as are the overall frequencies of the R72 and P72 alleles in the two age groups (designated as *R72 and *P72, respectively in the Figure). These results are consistent with the observation that allele is not benign, as p53 regulates expression of a second protein, p21, which inhibits cyclin-dependent kinases (CDKs) needed to drive cells through the cell-cycle (a mutation in either gene can disrupt the cell cycle leading to increased cell division).

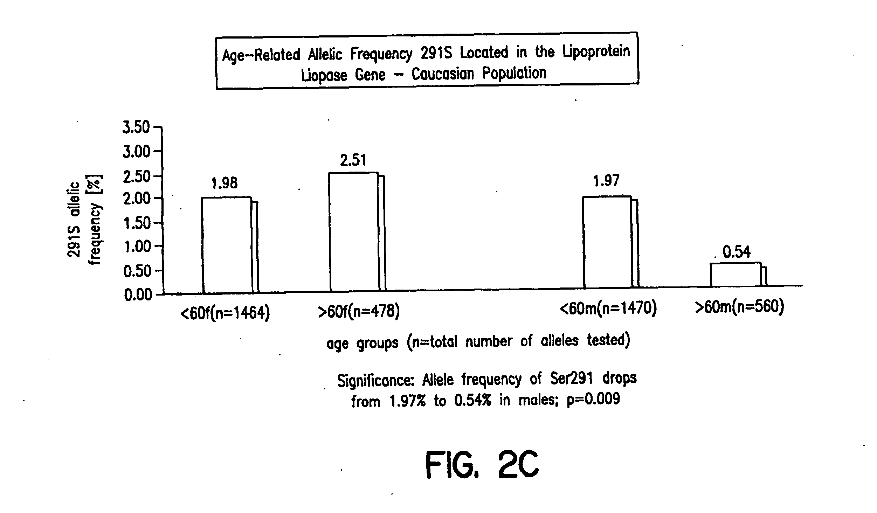

Figure 2C shows the results of the lipoprotein lipase gene genetic marker assays reveals a statistically significant decrease (from 1.97% to 0.54%) in the frequency of the polymorphic allele (S291) in Caucasian males with age (see also Reymer et al. (1995) Nature Genetics 10:28-34). The frequencies of this allele in Caucasian females of different age groups are also shown.

### COMPARATIVE EXAMPLE 2

This example describes the use of MALDI-TOF mass spectrometry to analyze DNA samples of a number of subjects as individual samples and as pooled samples of multiple subjects to assess the presence or absence of a polymorphic allele (the 353Q allele) of the Factor VII gene and determine the frequency of the allele in the group of subjects. The results of this study show that essentially the same allelic frequency can be obtained by analyzing pooled DNA samples as by analyzing each sample separately and thereby demonstrate the quantitative nature of MALDI-TOF mass spectrometry in the analysis of nucleic acids.

### Factor VII

Factor VII is a serine protease involved in the extrinsic blood coagulation cascade. This factor is activated by thrombin and works with tissue factor (Factor III) in the processing of Factor X to Factor Xa. There is evidence that supports an association between polymorphisms in the Factor VII gene and increased Factor VII activity which can result in an elevated risk of ischemic cardiovascular disease, including myocardial infarction. The polymorphism investigated in this study is R353Q (i.e., a substitution of a glutamic acid residue for an arginine residue at codon 353 of the Factor VII gene) (see Table 5).

### Analysis of DNA samples for the presence or absence of the 353Q allele of the Factor VII gene

Genomic DNA was isolated from separate blood samples obtained from a large number of subjects divided into multiple groups of 92 subjects per group. Each sample of genomic DNA was analyzed using the BiomassPROBE^{™} assay as described in Example 1 to determine the presence or absence of the 353Q polymorphism of the Factor VII gene.

First, DNA from each sample was amplified in a polymerase chain reaction using primers F7-353FUS4 (SEQ ID NO: 24) and F7-353RUS5 (SEQ ID NO: 26) as shown below and using standard conditions, for example, as described in Example 1. One of the primers was biotinylated to permit immobilization of the amplification product to a solid support. The purified amplification products were immobilized via a biotin-avidin linkage to streptavidin-coated beads and the double-stranded DNA was denatured. A detection primer was then annealed to the immobilized DNA using conditions such as, for example, described in Example 1. The detection primer is shown as F7-353-P (SEQ ID NO: 27) below. The PROBE extension reaction was carried out using conditions, for example, such as those described in Example 1. The reaction was performed using ddG.

The DNA was denatured to release the extended primers from the immobilized template. Each of the resulting extension products was separately analyzed by MALDI-TOF mass spectrometry. A matrix such as 3-hydroxypicolinic acid (3-HPA) and a UV laser could be used in the MALDI-TOF mass spectrometric analysis. The products resulting from the reaction conducted on a "wild-type" allele template (wherein codon 353 encodes an arginine) and from the reaction conducted on a polymorphic 353Q allele template (wherein codon 353 encodes a glutamic acid) are shown below and designated as 353 CGG and 353 CAG, respectively. The masses for each product as can be measured by MALDI-TOF mass spectrometry are also provided (i.e., 5646.8 Da for the wild-type product and 5960 Da for the polymorphic product).

The MALDI-TOF mass spectrometric analyses of the PROBE reactions of each DNA sample were first conducted separately on each sample (250 nanograms total concentration of DNA per analysis). The allelic frequency of the 353Q polymorphism in the group of 92 subjects was calculated based on the number of individual subjects in which it was detected.

Next, the samples from 92 subjects were pooled (250 nanograms total concentration of DNA in which the concentration of any individual DNA is 2.7 nanograms) and the pool of DNA was subjected to MALDITOF mass spectrometric analysis. The area under the signal corresponding to the mass of the 353Q polymorphism PROBE extension product in the resulting spectrum was integrated in order to quantitate the amount of DNA present. The ratio of this amount to total DNA was used to determine the allelic frequency of the 353Q polymorphism in the group of subjects. This type of individual sample vs. pooled sample analysis was repeated for numerous different groups of 92 different samples.

The frequencies calculated based on individual MALDI-TOF mass spectrometric analysis of the 92 separate samples of each group of 92 are compared to those calculated based on MALDI-TOF mass spectrometric analysis of pools of DNA from 92 samples in Figure 9. These comparisons are shown as "pairs" of bar graphs in the Figure, each pair being labeled as a separate "pool" number, e.g., P1, P16, P2, etc. Thus, for example, for P1, the allelic frequency of the polymorphism calculated by separate analysis of each of the 92 samples was 11.41 % and the frequency calculated by analysis of a pool of all of the 92 DNA samples was 12.09%.

The similarity in frequencies calculated by analyzing separate DNA samples individually and by pooling the DNA samples demonstrates that it is possible, through the quantitative nature of MALDI-TOF mass spectrometry, to analyze pooled samples and obtain accurate frequency determinations. The ability to analyze pooled DNA samples significantly reduces the time and costs involved in the use of the non-selected, healthy databases as described herein. It has also been shown that it is possible to decrease the DNA concentration of the individual samples in a pooled mixture from 2.7 nanograms to 0.27 nanograms without any change in the quality of the spectrum or the ability to quantitate the amount of sample detected.

### Factor VII R353Q PROBE Assay

PROBE Assay for cod353 CGG > CAG (Arg > Gln), Exon 9 G > A. **PCR fragment: 134 bp (incl. US tags; SEQ ID Nos. 22 and 23)** Frequency of A allele: Europeans about 0.1, Japanese/Chinese about 0.03-0.05 (Thromb. Haemost. 1995, 73:617-22; Diabetologia 1998, 41:760-6) :

F7-353FUS4>
1201 GTGCCGGCTA CTCGGATGGC AGCAAGGACT CCTGCAAGGG GGACAGTGGA
GGCCCACATG
F7-353-P>      A              <F7-353RUS5
1261 CCACCCACTA CCGGGGCACG TGGTACCTGA CGGGCATCGT CAGCTGGGGC
CAGGGCTGCG

| Primers (SEQ ID NOs: 24-26) | | Tm^{gs} |
|---|---|---|
| F7-353FUS4 | CCC **AGT CAC GAC GTT GTA AAA** CGA TGG CAG CAA GGA CTC CTG | 64°C |
| F7-353-P | CAC ATG CCA CCC ACT ACC | |
| F7-353RUS5 | AGC **GGA TAA CAA TTT CAC ACA GGT** GAC GAT GCC CGT CAG GTA C | 64°C |

### Masses

| **Allele** | **Product Termination: ddG** | **SEQ #** | **Length** | **Mass** |
|---|---|---|---|---|
| F7-353-P | atgccacccactacc | 27 | 18 | 5333.6 |
| 353 CGG | cacatgccacccactaccg | 28 | 19 | 5646.8 |
| 353 CAG | cacatgccacccactaccag | 29 | 20 | 5960 |
| US5-bio bio- | agcggataacaatttcacacagg | 30 | 23 | 7648.6 |

### Conclusion

The above examples demonstrate an effect of altered frequency of disease causing genetic factors within the general population. Interpretation of those results allows prediction of the medical relevance of polymorphic genetic alterations. In addition, conclusions can be drawn with regard to their penetrance, diagnostic specificity, positive predictive value, onset of disease, most appropriate onset of preventive strategies, and the general applicability of genetic alterations identified in isolated populations to panmixed populations. Therefore, an age- and sex-stratified population-based sample bank that is ethnically homogenous is a suitable tool for rapid identification and validation of genetic factors regarding their potential medical utility.

### EXAMPLE 3

### MORBIDITY AND MORTALITY MARKERS

### Sample Band and Initial Screening

Healthy samples were obtained through the blood bank of San Bernardino, CA. Donors signed prior to the blood collection a consent form and agreed that their blood will be used in genetic studies with regard to human aging. All samples were anomymized. Tracking back of samples is not possible.

### Isolation of DNA from blood samples of a healthy donor population

Blood is obtained from a donor by venous puncture and preserved with 1mM EDTA pH 8.0. Ten milliliters of whole blood from each donor was centrifuged at 2000x g. One milliliter of the buffy coat was added to 9 milliters of 155mM NH₄Cl, 10mM KHCO₃, and 0.1mM Na₂EDTA, incubated 10 minutes at room temperature and centrifuged for 10 minutes at 2000x g. The supernatant was removed, and the white cell pellet was washed in 155mM NH₄Cl, 10mM KHCO₃, and 0.1 mM Na₂EDTA and resuspended in 4.5 milliliters of 50mM Tris, 5mM EDTA, and 1 % SDS. Proteins were precipitated from the cell lysate by 6M Ammonium Acetate, pH 7.3, and separated from the nucleic acid by centrifugation 3000x g. The nucleic acid was recovered from the supernatant by the addition of an equal volume of 100% isopropanol and centrifugation at 2000x g. The dried nucleic acid pellet was hydrated in IOmM Tris pH 7.6 and 1mM Na2EDTA and stored at 4C.

In this study, samples were pooled as shown in Table 1. Both parents of the blood donors were of Caucasian origin.

**Table 1.**

| **Pool ID** | **Sex** | **Age-range** | **# individuals** |
|---|---|---|---|
| SP1 | Female | 18-39 years | 276 |
| SP2 | Males | 18-39 years | 276 |
| SP3 | Females | 60-69 years | 184 |
| SP4 | Males | 60-79 years | 368 |

More than 400 SNPs were tested using all four pools. After one test run 34 assays were selected to be re-assayed at least once. Finally, 1O assays showed repeatedly differences in allele frequencies of several percent and, therefore, fulfilled the criteria to be tested using the individual samples. Average allele frequency and standard deviation is tabulated in Table 2.

**Table 2**

| **Assay ID** | **SP1** | **SP1-STD** | **SP2** | **SP2-STD** | **SP3** | **SP3-STD** | **SP4** | **SP4-STD** |
|---|---|---|---|---|---|---|---|---|
| **47861** | **0.457** | 0.028 | 0.433 | 0.042 | **0.384** | 0.034 | **0.380** | 0.015 |
| **47751** | **0.276** | 0.007 | 0.403 | 0.006 | **0.428** | 0.052 | **0.400** | 0.097 |
| **48319** | **0.676** | 0.013 | 0.627 | 0.018 | **0.755** | 0.009 | **0.686** | 0.034 |
| **48070** | **0.581** | 0.034 | 0.617 | 0.045 | **0.561** | n.a. | **0.539** | 0.032 |
| **49807** | **0.504** | 0.034 | 0.422 | 0.020 | **0.477** | 0.030 | **0.556** | 0.005 |
| **49534** | **0.537** | 0.017 | 0.503 | n.a. | **0.623** | 0.023 | **0.535** | 0.009 |
| **49733** | **0.560** | 0.006 | 0.527 | 0.059 | **0.546** | 0.032 | **0.436** | 0.016 |
| **49947** | **0.754** | 0.008 | 0.763 | 0.047 | **0.736** | 0.052 | **0.689** | 0.025 |
| **50128** | **0.401** | 0.022 | 0.363 | 0.001 | **0.294** | 0.059 | **0.345** | 0.013 |
| 63306 | 0.697 | 0.012 | 0.674 | 0.013 | 0.712 | 0.017 | 0.719 | 0.005 |

So far, 7 out of the 10 potential morbidity markers were fully analyzed. Additional information about genes in which these SNPs are located was gathered through publicly databases like Genbank.

### AKAPS

Candidate morbidity and mortality markers include housekeeping genes, such as genes involved in signal transduction. Among such genes are the A-kinase anchoring proteins (AKAPs) genes, which participate in signal transduction pathways involving protein phosphorylation. Protein phosphorylation is an important mechanism for enzyme regulation and the transduction of extracellular signals across the cell membrane in eukaryotic cells. A wide variety of cellular substrates, including enzymes, membrane receptors, ion channel and transcription factors, can be phosphorylated in response to extracellular signals that interact with cells. A key enzyme in the phosphorylation of cellular proteins in response to hormones and neurotransmitters is cyclic AMP (cAMP)-dependent protein kinase (PKA). Upon activation by cAMP, PKA thus mediates a variety of cellular responses to such extracellular signals. An array of PKA isozymes are expressed in mammalian cells. The PKAs usually exist as inactive tetramers containing a regulatory (R) subunit dimer and two catalytic (C) subunits. Genes encoding three C subunits (Cα, Cβ and Cγ) and four R subunits (RIα, RIβ, RIIα and RIIβ) have been identified [see Takio et al. (1982) Proc. Natl. Acad. Sci. U.S. A. 79:2544-2548; Lee et al. (1983) Proc. Natl. Acad. Sci. U.S. A. 80:3608-3612; Jahnsen et al. (1996) J. Biol. Chem. 261:12352-12361; Clegg et al. (1988) Proc. Natl. Acad. Sci. U. S. A. 85:3703-3707; and Scott (1991) Pharmacol. Ther. 50:123-145]. The type I (RI) α and type II (RII) α subunits are distributed ubiquitously, whereas RIβ and RIIβ are present mainly in brain [see. *e.g.*, Miki and Eddy (1999) J. Biol. Chem. 274:29057-29062]. The type I PKA holoenzyme (RIα and RIβ) is predominantly cytoplasmic, whereas the majority of type II PKA (RIIα and RIIβ) associates with cellular structures and organelles [Scott (1991) Pharmacol Ther. 50:123-145]. Many hormones and other signals act through receptors to generate cAMP which binds to the R subunits of PKA and releases and activates the C subunits to phosphorylate proteins. Because protein kinases and their substrates are widely distributed throughout cells, there are mechanisms in place in cells to localize protein kinase-mediated responses to different signals. One such mechanism involves subcellular targeting of PKAs through association with anchoring proteins, referred to as A-kinase anchoring proteins (AKAPs), that place PKAs in close proximity to specific organelles or cytoskeletal components and particular substrates thereby providing for more specific PKA interactions and localized responses [see, *e.g.,* Scott et al. (1990) J. Biol. Chem. 265:21561-21566; Bregman et al. (1991) J. Biol. Chem. 266:7207-72.13; and Miki and Eddy (1999) J. Biol. Chem. 274:29057-29062]. Anchoring not only places the kinase close to preferred substrates, but also positions the PKA holoenzyme at sites where it can optimally respond to fluctuations in the second messenger cAMP [Mochly-Rosen (1995) Science 268:247-251; Faux and Scott (1996) Trends Biochem. Sci. 21:312-315; Hubbard and Cohen (1993) Trends Biochem. Sci. 18:172-177].

Up to 75% of type II PKA is localized to various intracellular sites through association of the regulatory subunit (RII) with AKAPs [see, *e.g.,* Hausken et al. (1996) J. Biol. Chem. 271:29016-29022]. RII subunits of PKA bind to AKAPs with nanomolar affinity [Carr et al. (1992) J. Biol. Chem. 267:13376-13382], and many AKAP-RII complexes have been isolated from cell extracts. RI subunits of PKA bind to AKAPs with only micromolar affinity [Burton et al. (1997) Proc. Natl. Acad. Sci. U.S.A. 94:11067-1 1072]. Evidence of binding of a PKA RI subunit to an AKAP has been reported [Miki and Eddy (1998) J. Biol. Chem 273:34384-34390] in which RIα-specific and RIa/RIIa dual specificity PKA anchoring domains were identified on FSC1/AKAP82. Additional dual specific AKAPs, referred to as D-AKAP1 and D-AKAP2, which interact with the type I and type II regulatory subunits of PKA have also been reported [Huang et al. (1997) J. Biol. Chem. 272:8057-8064; Huang et al. (1997) Proc. Natl. Acad. Sci. U.S.A. 94:11184-11189].

More than 20 AKAPs have been reported in different tissues and species. Complementary DNAs (cDNAs) encoding AKAPs have been isolated from diverse species, ranging from *Caenorhabditis elegans* and *Drosophilia* to human [see, *e.g.,* Colledge and Scott (1999) Trends Cell Biol. 9:216-221]. Regions within AKAPs that mediate association with RII subunits of PKA have been identified. These regions of approximately 10-18 amino acid residues vary substantially in primary sequence, but secondary structure predictions indicate that they are likely to form an amphipathic helix with hydrophobic residues aligned along one face of the helix and charged residues along the other [Carr et al. (1991) J. Bio/. Chem. 266:14188-14192; Carr et al. (1992) J. Biol. Chem. 267:13376-13382]. Hydrophobic amino acids with a long aliphatic side chain, e.g., valine, leucine or isoleucine, may participate in binding to RII subunits [Glantz et al. (1993) J. Biol. Chem. 268:12796-12804].

Many AKAPs also have the ability to bind to multiple proteins, including other signaling enzymes. For example, AKAP79 binds to PKA, protein kinase C (PKC) and the protein phosphatase calcineurin (PP2B) [Coghlan et al. (1995) Science 267:108-112 and Klauck et al. (1996) Science 271:1589-1592]. Therefore, the targeting of AKAP79 to neuronal postsynaptic membranes brings together enzymes with opposite catalytic activities in a single complex.

AKAPs thus serve as potential regulatory mechanisms that increase the selectivity and intensity of a cAMP-mediated response. There is a need, therefore, to identify and elucidate the structural and functional properties of AKAPs in order to gain a complete understanding of the important role these proteins play in the basic functioning of cells.

### AKAP10

The sequence of a human AKAP10 cDNA (also referred to as D-AKAP2) is available in the GenBank database, at accession numbers AF037439 (SEQ ID NO: 31) and NM 007202. The AKAP10 gene is located on chromosome 17.

The sequence of a mouse D-AKAP2 cDNA is also available in the GenBank database (see accession number AF021833). The mouse D-AKAP2 protein contains an RGS domain near the amino terminus that is characteristic of proteins that interact with Gα subunits and possess GTPase activating protein-like activity [Huang et al. (1997) Proc. Natl. Acad. Sci. U.S.A. 94:11184-11189]. The human AKAP10 protein also has sequences homologous to RGS domains. The carboxy-terminal 40 residues of the mouse D-AKAP2 protein are responsible for the interaction with the regulatory subunits of PKA. This sequence is fairly well conserved between the mouse D-AKAP2 and human AKAP10 proteins. **Polymorphisms of the human AKAP10 gene and polymorphic AKAP10 proteins**

Polymorphisms of AKAP genes that alter gene expression, regulation, protein structure and/or protein function are more likely to have a significant effect on the regulation of enzyme (particularly PKA) activity, cellular transduction of signals and responses thereto and on the basic functioning of cells than polymorphisms that do not alter gene and/or protein function. Included in the polymorphic AKAPs provided herein are human AKAP10 proteins containing differing amino acid residues at position number 646.

Amino acid 646 of the human AKAP10 protein is located in the carboxy-terminal region of the protein within a segment that participates in the binding of R-subunits of PKAs. This segment includes the carboxy-terminal 40 amino acids.

The amino acid residue reported for position 646 of the human AKAP10 protein is an isoleucine. Polymorphic human AKAP10 proteins provided herein have the amino acid sequence but contain residues other than isoleucine at amino acid position 646 of the protein. In particular embodiments of the polymorphic human AKAP10 proteins provided herein, the amino acid at position 646 is a valine, leucine or phenylalanine residue.

### An A to G transition at nucleotide 2073 of the human AKAP10 coding sequence

As described herein, an allele of the human AKAP10 gene that contains a specific polymorphism at position 2073 of the coding sequence and thereby encodes a valine at position 646 has been detected in varying frequencies in DNA samples from younger and older segments of the human population. In this allele, the A at position 2073 of the AKAP10 gene coding sequence is changed from an A to a G, giving rise to an altered sequence in which the codon for amino acid 646 changes from ATT, coding for isoleucine, to GTT, coding for valine.

### Morbidity marker 1: human protein kinase A anchoring protein (AKAP10-1)

### PCR Amplification and BiomassPROBE assay detection of AKAP10-1 in a healthy donor population

### PCR Amplification of donor population for AKAP 10

PCR primers were synthesized by OPERON using phosphoramidite chemistry. Amplification of the AKAP10 target sequence was carried out in single 50µl PCR reaction with 100ng-1ug of pooled human genomic DNAs in a 50µl PCR reaction. Individual DNA concentrations within the pooled samples were present in equal concentration with the final concentration ranging from 1-25ng. Each reaction containing IX PCR buffer (Qiagen, Valencia, CA), 200uM dNTPs, 1 U Hotstar Taq polymerase (Qiagen, Valencia, CA), 4mM MgCl₂, and 25pmol of the forward primer containing the universal primer sequence and the target specific sequence 5'-TCTCAATCATGTGCATTGAGG-3'(SEQ ID NO: 45), 2pmol of the reverse primer
5'-AGCGGATAACAATTTCACACAGGGATCACACAGCCATCAGCAG-3' (SEQ ID NO: 46), and 10pmol of a biotinylated universal primer complementary to the 5' end of the PCR amplicon
5'-AGCGGATAACAATTTCACACAGG-3'(SEQ ID NO: 47). After an initial round of amplification with the target with the specific forward and reverse primer, the 5' biotinylated universal primer then hybridized and acted as a reverse primer thereby introducing a 3' biotin capture moiety into the molecule. The amplification protocol results in a 5'-biotinylated double stranded DNA amplicon and dramatically reduces the cost of high throughput genotyping by eliminating the need to 5' biotin label each forward primer used in a genotyping. Thermal cycling was performed in 0.2mL tubes or 96 well plate using an MJ Research Thermal Cycler (calculated temperature) with the following cycling parameters: 94° C for 5 min; 45 cycles: 94° C for 20 sec, 56° C for 30 sec, 72° C for 60 sec; 72° C 3min.

### Immobilization of DNA

The 50µl PCR reaction was added to 25µl of streptavidin coated magnetic bead (Dynal) prewashed three times and resuspended in 1M NH₄Cl, 0.06M NH₄OH. The PCR amplicons were allowed to bind to the beads for 15 minutes at room temperature. The beads were then collected with a magnet and the supernatant containing unbound DNA was removed. The unbound strand was release from the double stranded amplicons by incubation in 100mM NaOH and washing of the beads three times with 10mM Tris pH 8.0.

### BiomassPROBE assay analysis of donor population for AKAP10-1 (clone 48319)

Genotyping using the BiomassPROBE assay methods was carried out by resuspending the DNA coated magnetic beads in 26mM Tris-HCl pH 9.5, 6.5 mM MgCl₂ and 50mM each of dTTP and 50mM each of ddCTP, ddATP, ddGTP, 2.5U of a thermostable DNA polymerase (Ambersham) and 20pmol of a template specific oligonucleotide PROBE primer 5'-CTGGCGCCCACGTGGTCAA-3' (SEQ ID NO: 48) (Operon). Primer extension occurs with three cycles of oligonucleotide primer hybridization and extension. The extension products were analyzed after denaturation from the template with 50mM NH₄Cl and transfer of 150nL each sample to a silicon chip preloaded with 150nL of H3PA matrix material. The sample material was allowed to crystallize and was analyzed by MALDI-TOF (Bruker, PerSeptive). The SNP that is present in AKAP10-1 is a T to C transversion at nucleotide number 156277 of the sequence of a genomic clone of the AKAP10 gene (GenBank Accession No. AC005730) (SEQ ID NO: 36). SEQ ID NO: 35: represents the nucleotide sequence of human chromosome 17, which contains the genomic nucleotide sequence of the human AKAP10 gene, and SEQ ID NO: represents the nucleotide sequence of human chromosome 17, which contains the genomic nucleotide sequence of the human AKAP10-1 allele. The mass of the primer used in the BioMass probe reaction was 5500.6 daltons. In the presence of the SNP, the primer is extended by the addition of ddC, which has a mass of 5773.8. The wildtype gene results in the addition of dT and ddG to the primer to produce an extension product having a mass of 6101 daltons.

The frequency of the SNP was measured in a population of age selected healthy individuals. Five hundred fifty-two (552) individuals between the ages of 18-39 years (276 females, 276 males) and 552 individuals between the ages of 60-79 (184 females between the ages of 60-69, 368 males between the age of 60-79) were tested for the presence of the polymorphism localized in the non-translated 3'region of AKAP 10. Differences in the frequency of this polymorphism with increasing age groups were observed among healthy individuals. Statistical analysis showed that the significance level for differences in the allelic frequency for alleles between the "younger" and the "older" populations was p = 0.0009 and for genotypes was p = 0.003. Differences between age groups are significant. For the total population allele significance is p = 0.0009, and genotype significance is p = 0.003.

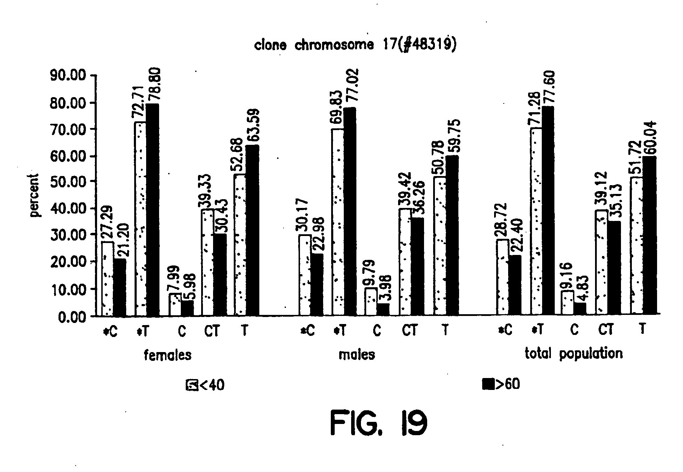

This marker led to the best significant result with regard to allele and genotype frequencies in the age-stratified population. Figure 19 shows the allele and genotype frequency in both genders as well as in the entire population. For latter the significance for alleles was p=0.0009 and for genotypes was p = 0.003. The young and old populations were in Hardy-Weinberg equilibrium. A preferential change of one particular genotype was not seen.

The polymorphism is localized in the non-translated 3'-region of the gene encoding the human protein kinase A anchoring protein (AKAP10). The gene is located on chromosome 17. Its structure includes 15 exons and 14 intervening sequences (introns). The encoded protein is responsible for the sub-cellular localization of the cAMP-dependent protein kinase and, therefore, plays a key role in the G-protein mediated receptor-signaling pathway (Huang et al. PNAS (1007) 94:11184-11189). Since its localization is outside the coding region, this polymorphism is most likely in linkage disequilibrium (LD) with other non-synonymous polymorphisms that could cause amino acid substitutions and subsequently alter the function of the protein. Sequence comparison of different Genbank database entries concerning this gene revealed further six potential polymorphisms of which two are supposed to change the respective amino acid (see Table 3).

**Table 3**

| Exon | Codon | Nucleotides | Amino acid |
|---|---|---|---|
| 3 | 100 | GCT > GCC | Ala > Ala |
| 4 | 177 | AGT>GTG | Met>Val |
| 8 | 424 | GGG > GGC | Gly > Gly |
| 10 | 524 | CCG > CTG | Pro > Leu |
| 12 | 591 | GTG > G.TC | Val > Val |
| 12 | 599 | CGC > CGA | Arg > Arg |

| | | | |
|---|---|---|---|
| **Morbitity marker 2: human protein kinase A anchoring protein** **(AKAP10-5)** | | | |

### Discovery of AKAP10-5 Allele (SEQ ID NO: 33)

Genomic DNA was isolated from blood (as described above) of seventeen (17) individuals with a genotype CC at the AKAP10-1 gene locus and a single heterozygous individual (CT) (as described). A target sequence in the AKAP10-1 gene which encodes the C-terminal PKA binding domain was amplified using the polymerase chain reaction. PCR primers were synthesized by OPERON using phosphoramidite chemistry. Amplification of the AKAP10-1 target sequence was carried out in individual 50µl PCR reaction with 25ng of human genomic DNA templates. Each reaction containing I X PCR buffer (Qiagen, Valencia, CA), 200µM dNTPs, IU Hotstar Taq polymerase (Qiagen, Valencia, CA), 4mM MgCl₂, 25pmol of the forward primer (Ex13F) containing the universal primer sequence and the target specific sequence 5'-TCC CAA AGT GCT GGA ATT AC-3' (SEQ ID NO: 53), and 2pmol of the reverse primer (Ex14R) 5'-GTC CAA TAT ATG CAA ACA GTT G-3' (SEQ ID NO: 54). Thermal cycling was performed in 0.2mL tubes or 96 well plate using an MJ Research Thermal Cycler (MJ Research, Waltham, MA) (calculated temperature) with the following cycling parameters: 94° C for 5 min; 45 cycles; 94° C for 20 sec, 56° C for 30 sec, 72° C for 60 sec; 72° C 3min. After amplification the amplicons were purified using a chromatography (Mo Bio Laboratories (Solana Beach, CA)).

The sequence of the 18 amplicons, representing the target region, was determined using a standard Sanger cycle sequencing method with 25nmol of the PCR amplicon, 3.2uM DNA sequencing primer 5'-CCC ACA GCA GTT AAT CCT TC-3'(SEQ ID NO: 55), and chain terminating dRhodamine labeled 2', 3' dideoxynucleotides (PE Biosystems, Foster City, CA) using the following cycling parameters: 96° C for 15 seconds; 25 cycles: 55° C for 15 seconds, 60° C for 4 minutes. The sequencing products precipitated by 0.3M NaOAc and ethanol. The precipitate was centrifuged and dried. The pellets were resuspended in deionized formamide and separated on a 5% polyacrylimide gel. The sequence was determined using the "Sequencher" software (Gene Codes, Ann Arbor, MI).

The sequence of all 17 of the amplicons, which are homozygous for the AKAP10-1 SNP of the amplicons, revealed a polymorphism at nucleotide position 152171 (numbering for GenBank Accession No. AC005730 for AKAP10 genomic clone (SEQ ID NO: 35)) with A replaced by G. This SNP can also be designated as located at nucleotide 2073 of a cDNA clone of the wildtype AKAP10 (GenBank Accession No. AF037439) (SEQ ID NO: 31). The amino acid sequence of the human AKAP10 protein is provided as SEQ ID NO: 32. This single nucleotide polymorphism was designated as AKAP10-5 (SEQ ID NO: 33) and resulted in a substitution of a valine for an isoleucine residue at amino acid position 646 of the amino acid sequence of human AKAP10 (SEQ ID NO: 32).

### PCR Amplification and BiomassPROBE assay detection of AKAP10-5 in a healthy donor population

The healthy population stratified by age is a very efficient and a universal screening tool for morbidity associated genes by allowing for the detection of changes of allelic frequencies in the young compared to the old population. Individual samples of this healthy population base can be pooled to further increase the throughput.

Healthy samples were obtained through the blood bank of San Bernardino, CA. Both parents of the blood donors were of Caucasian origin. Practically a healthy subject, when human, is defined as human donor who passes blood bank criteria to donate blood for eventual use in the general population. These criteria are as follows: free of detectable viral, bacterial, mycoplasma, and parasitic infections; not anemic; and then further selected based upon a questionnaire regarding history (see Figure 3). Thus, a healthy population represents an unbiased population of sufficient health to donate blood according to blood bank criteria, and not further selected for any disease state. Typically such individuals are not taking any medications.

PCR primers were synthesized by OPERON using phosphoramidite chemistry. Amplification of the AKAP10 target sequence was carried out in a single 50µl PCR reaction with 100ng- 1µg of pooled human genomic DNAs in a 50µl PCR reaction. Individual DNA concentrations within the pooled samples were present in equal concentration with the final concentration ranging from 1-25ng. Each reaction contained 1X PCR buffer (Qiagen, Valencia, CA), 200µM dNTPs, 1 U Hotstar Taq polymerase (Qiagen, Valencia, CA), 4mM MgCl₂, and 25pmol of the forward primer containing the universal primer sequence and the target specific sequence 5'-AGCGGATAACAATTTCACACAGGGAGCTAGCTTGGAAGAT TGC-3' (SEQ ID NO: 41), 2pmol of the reverse primer
5'-GTCCAATATATGCAAACAGTTG-3' (SEQ ID NO: 54), and 10pmol of a biotinylated universal primer complementary to the 5' end of the PCR amplicon BIO:5'-AGCGGATAACAATTTCACACAGG-3' (SEQ ID NO: 43). After an initial round of amplification with the target with the specific forward and reverse primer, the 5' biotinylated universal primer can then be hybridized and acted as a forward primer thereby introducing a 5' biotin capture moiety into the molecule. The amplification protocol resulted in a 5'-biotinylated double stranded DNA amplicon and dramatically reduced the cost of high throughput genotyping by eliminating the need to 5' biotin label every forward primer used in a genotyping.

Themal cycling was performed in 0.2mL tubes or 96 well-plate using an MJ Research Thermal Cycler (calculated temperature) with the following cycling parameters: 94° C for 5 min; 45 cycles: 94° C for 20 sec, 56° C for 30 sec; 72° C for 60 sec; 72° C 3min.

### Immobilization of DNA

The 50 µl PCR reaction was added to 25µL of streptavidin coated magnetic beads (Dynal, Oslo, Norway), which were prewashed three times and resuspended in 1M NH₄Cl, 0.06M NH₄OH. The 5' end of one strand of the double stranded PCR amplicons were allowed to bind to the beads for 15 minutes at room temperature. The beads were then collected with a magnet and the supernatant containing unbound DNA was removed. The hybridized but unbound strand was released from the double stranded amplicons by incubation in 100mM NaOH and washing of the beads three times with 10mM Tris pH 8.0.

### Detection of AKAP10-5 using BiomassPROBE^{™} Assay

BiomassPROBE^{™} assay of primer extension analysis (see, U.S. Patent No. 6,043,031) of donor population for AKAP 10-5 (SEQ ID NO: 33) was performed. Genotyping using these methods was carried out by resuspending the DNA coated magnetic beads in 26mM Tris-HCL pH 9.5, 6.5 mM MgCl₂, 50mM dTTP, 50mM each of ddCTP, ddATP, ddGTP, 2.5U of a thermostable DNA polymerase (Ambersham), and 20pmol of a template specific oligonucleotide PROBE primer 5'-ACTGAGCCTGCTGCATAA-3' (SEQ ID NO: 44) (Operon). Primer extension occurs with three cycles of oligonucleotide primer with hybridization and extension. The extension products were analyzed after denaturation from the template with 50 mM NH₄Cl and transfer of 150 nL of each sample to a silicon chip preloaded with 150 nl of H3PA matrix material. The sample material was allowed to crystallize and analyzed by MALDI-TOF (Bruker, PerSeptive). The primer has a mass of 5483.6 daltons. The SNP results in the additional of a ddC to the primer, giving a mass of 5756.8 daltons for the extended product. The wild type results in the addition a T and ddG to the primer giving a mass of 6101 daltons.

The frequency of the SNP was measured in a population of age selected healthy individuals. Seven hundred thirteen (713) individuals under 40 years of age (360 females, 353 males) and 703 individuals over 60 years of age (322 females, 381 males) were tested for the presence of the SNP, AKAP10-5 (SEQ ID NO: 33). Results are presented below in

**Table 1.**

| **TABLE 1** | | | | | |
|---|---|---|---|---|---|
| **AKAP10-5 (2073V) frequency comparison in 2 age groups** | | | | | |
| | | | **< 40** | **> 60** | **delta G allele** |
| **Female** | Alleles | *G | 38.6 | 34.6 | 4.0 |
| | | *A | 61.4 | 65.4 | |
| | Genotypes | G | 13.9 | 11.8 | 2.1 |
| | | GA | 49.4 | 45.7 | |
| | | A | 36.7 | 42.5 | |
| | | | | | |
| **Male** | Alleles | *G | 41.4 | 37.0 | 4.4 |
| | | *A | 58.6 | 63.0 | |
| | Genotypes | G | 18.4 | 10.8 | 7.7 |
| | | GA | 45.9 | 52.5 | |
| | | A | 35.7 | 36.7 | |
| | | | | | |
| **Total** | Alleles | *G | 40.0 | 35.9 | 4.1 |
| | | *A | 60.0 | 64.1 | |
| | Genotypes | G | 16.1 | 11.2 | 4.9 |
| | | GA | 47.7 | 49.4 | |
| | | A | 36.2 | 39.4 | |

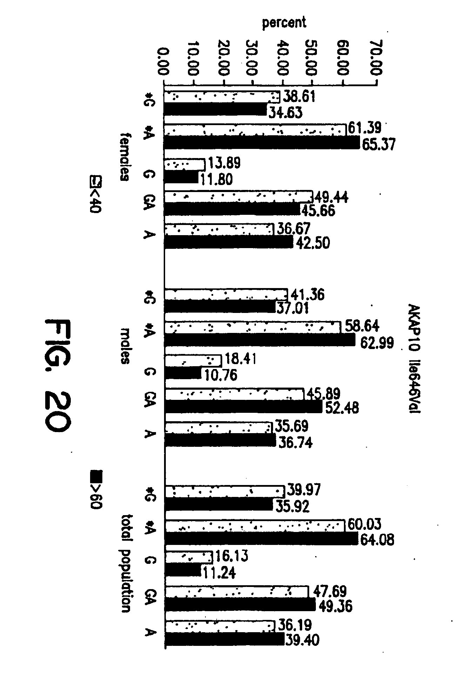

Figure 20 graphically shows these results of allele and genotype distribution in the age and sex stratified Caucasian population.

### Morbidity marker 3: human methionine sulfoxide reductase A (msrA)

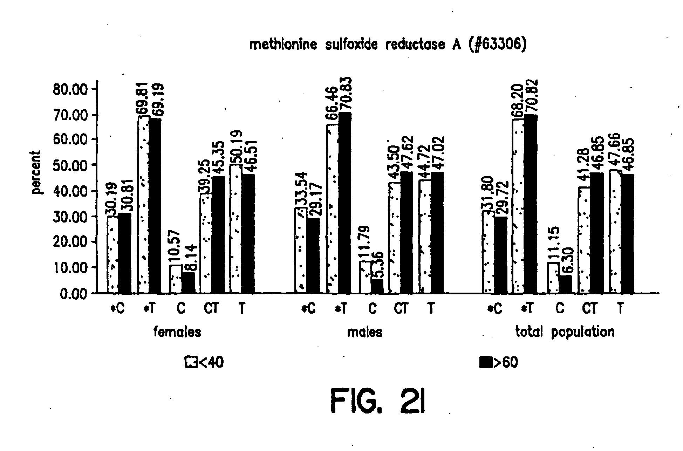

The age-related allele and genotype frequency of this marker in both genders and the entire population is shown in Figure 21. The decrease of the homozygous CC genotype in the older male population is highly significant.

### Methionine sulfoxide reductase A (#63306)

PCR Amplification and BiomassPROBE assay detection of the human methioine sulfoxid reductase A (h-msr-A) in a healthy donor population

### PCR Amplification of donor population for h-msr-A

PCR primers were synthesized by OPERON using phosphoramidite chemistry. Amplification of the AKAP10 target sequence was carried out in single 50µl PCR reaction with 100ng-1ug of pooled human genomic DNA templates in a 50µl PCR reaction. Individual DNA concentrations within the pooled samples were present in an equal concentration with the final concentration ranging from 1-25ng. Each reaction containing I X PCR buffer (Qiagen, Valencia, CA), 200µM dNTPs, 1 U Hotstar Taq polymerase (Qiagen, Valencia, CA), 4mM MgCl₂, 25pmol of the forward primer containing the universal primer sequence and the target specific sequence 5'-TTTCTCTGCACAGAGAGGC-3' (SEQ ID NO: 49), 2pmol of the reverse primer
5'-AGCGGATAACAATTTCACACAGGGCTGAAATCCTTCGCTTTACC-3' (SEQ ID NO: 50), and 10pmol of a biotinylated universal primer complementary to the 5' end of the PCR amplicon
5'-AGCGGATAACAATTTCACACAGG-3' (SEQ ID NO: 51). After an initial round of amplification of the target with the specific forward and reverse primers, the 5' biotinylated universal primer was then hybridized and acted as a reverse primer thereby introducing a 3' biotin capture moiety into the molecule. The amplification protocol results in a 5'-biotinylated double stranded DNA amplicon and and dramatically reduces the cost of high throughput genotyping by eliminating the need to 5' biotin label each forward primer used in a genotyping. Thermal cycling was performed in 0.2mL tubes or 96 well plate using an MJ Research Thermal Cycler (calculated temperature) with the following cycling parameters: 94° C for 5 min; 45 cycles: 94° C for 20 sec, 56° C for 30 sec, 72° C for 60 sec; 72° C 3min.

### Immobilization of DNA

The 50µl PCR reaction was added to 25µl of streptavidin coated magnetic bead (Dynal) prewashed three times and resuspended in 1 M NH₄Cl, 0.06M NH₄OH. The PCR amplicons were allowed to bind to the beads for 15 minutes at room temperature. The beads were then collected with a magnet and the supernatant containing unbound DNA was removed. The unbound strand was release from the double stranded amplicons by incubation in 100mM NaOH and washing of the beads three times with 10mM Tris pH 8.0.

### BiomassPROBE assay analysis of donor population for h-msr A

Genotyping using the BiomassPROBE assay methods was carried out by resuspending the he DNA coated magnetic beads in 26mM Tris-HCl pH 9.5, 6.5 mM MgCl₂, 50mM of dTTPs and 50mM each of ddCTP, ddATP, ddGTP, 2.5U of a thermostable DNA polymerase (Ambersham), and 20pmol of a template specific oligonucleotide PROBE primer 5'-CTGAAAAGGGAGAGAAAG-3' (Operon) (SEQ ID NO: 52). Primer extension occurs with three cycles of oligonucleotide primer with hybridization and extension. The extension products were analyzed after denaturation from the template with 50mM NH₄Cl and transfer of 150nl each sample to a silicon chip preloaded with 150nl of H3PA matrix material. The sample material was allowed to crystallize and analyzed by MALDI-TOF (Bruker, PerSeptive). The SNP is represented as a T to C tranversion in the sequence of two ESTs. The wild type is represented by having a T at position 128 of GenBank Accession No. AW 195104, which represents the nucleotide sequence of an EST which is a portion of the wild type human msrA gene (SEQ ID NO: 39 ). The SNP is presented as a C at position 129 of GenBank Accession No. AW 874187, which represents the nucleotide sequence of an EST which is a portion of an allele of the human msrA gene (SEQ ID NO: 40).

In a genomic sequence the SNP is represented as an A to G transversion. The primer utilized in the BioMass probe reaction had a mass of 5654.8 daltons. In the presence of the SNP the primer is extended by the incorporation of a ddC and has a mass of 5928. In the presence of the wildtype the primer is extended by adding a dT and a DDC to produce a mass of 6232.1 daltons.

The frequency of the SNP was measured in a population of age selected healthy individuals. Five hundred fifty-two (552) individuals between the ages of 18-39 years (276 females, 276 males and 552 individuals between the age of 60-79 (184 females between the ages of 60-69, 368 males between the age of 60-79) were tested for the presence of the polymorphism localized in the nontranslated 3'region of h-msr-A.

Genotype difference between male age group among healthy individuals is significant. For the male population allele significance is p = 0.0009 and genotype significance is p = 0.003. The age-related allele and genotype frequency of this marker in both genders and the entire population is shown in Figure 21. The decrease of the homozygous CC genotype in the older male population is highly significant.

The polymorphism is localized in the non-translated 3'-region of the gene encoding the human methionine sulfoxide reductase (h-msrA). The exact localization is 451 base pairs downstream the stop codon (TAA). It is very likely that this SNP is in linkage disequilibrium (LD) with another polymorphism more upstream in the coding or promoter region; thus, it is not directly cause morbidity. The enzyme methionine sulfoxide reductase has been proposed to exhibit multiple biological functions. It may serve to repair oxidative protein damage but also play an important role in the regulation of proteins by activation or inactivation of their biological functions (Moskovitz et al. (1990) PNAS 95:14071-14075). It has also been shown that its activity is significantly reduced in brain tissues of Alzheimer patients (Gabbita et al., (1999) J. Neurochem 73:1660-1666). It is scientifically conceivable that proteins involved in the metabolism of reactive oxygen species are associated to disease.

### CONCLUSION

The use of the healthy population provides for the identification of morbidity markers. The identification of proteins involved in the G-protein coupled signaling transduction pathway or in the detoxification of oxidative stress can be considered as convincing results. Further confirmation and validation of other potential polymorphisms already identified in *silico* in the gene encoding the human protein kinase A anchoring protein could even provide stronger association to morbidity and demonstrate that this gene product is a suitable pharmaceutical or diagnostic target.

### EXAMPLE 4

### MALDI-TOF Mass Spectrometry Analysis

All of the products of the enzyme assays listed below were analyzed by MALDI-TOF mass spectrometry. A diluted matrix solution (0.15µL) containing of 10:1 3-hydroxypicolinic acid:ammonium citrate in 1:1 water:acetonitrile diluted 2.5-fold with water was pipetted onto a SpectroChip (Sequenom, Inc.) and was allowed to crystallize. Then, 0.15µL of sample was added. A linear PerSeptive Voyager DE mass spectrometer or Bruker Biflex MALDI-TOF mass spectrometer, operating in positive ion mode, was used for the measurements. The sample plates were kept at 18.2 kV for 400 nm after each UV laser shot (approximate 250 laser shots total), and then the target voltage was raised to 20 kV. The original spectra were digitized at 500 MHz.

### EXAMPLE 5

### Sample Conditioning

Where indicated in the examples below, the products of the enzymatic digestions were purified with ZipTips (Millipore, Bedford, MA). The ZipTips were pre-wetted with 10µL 50% acetonitrile and equilibrated 4 times with 10 µl 0.1 M TEAAc. The oligonucleotide fragments were bound to the C 18 in the ZipTip material by continuous aspiration and dispension of each sample into the ZipTip. Each digested oligonucleotide was conditioned by washing with 10 µL 0.1 M TEAAc, followed by 4 washing steps with 10 µL H₂O. DNA fragments were eluted from the Ziptip with 7 µL 50% acetonitrile.

Any method for condition the samples may be employed. Methods for conditioning, which generally is used to increase peak resolution, are well known (see, *e.g.*, International PCT application No. WO 98/20019).

### COMPARATIVE EXAMPLE 6

### DNA Glycosylase-Mediated Sequence Analysis

DNA Glycosylases modifies DNA at each position that a specific nucleobase resides in the DNA, thereby producing abasic sites. In a subsequent reaction with another enzyme, a chemical, or heat, the phosphate backbone at each abasic site can be cleaved.

The glycosylase utilized in the following procedures was uracil-DNA glycosylase (UDG). Uracil bases were incorporated into DNA fragments in each position that a thymine base would normally occupy by amplifying a DNA target sequence in the presence of uracil. Each uracil substituted DNA amplicon was incubated with UDG, which cleaved each uracil base in the amplicon, and was then subjected to conditions that effected backbone cleavage at each abasic site, which produced DNA fragments. DNA fragments were subjected to MALDI-TOF mass spectrometry analysis. Genetic variability in the target DNA was then assessed by analyzing mass spectra.

Glycosylases specific for nucleotide analogs or modified nucleotides, as described herein, can be substituted for UDG in the following procedures. The glycosylase methods described hereafter, in conjunction with phosphate backbone cleavage and MALDI, can be used to analyze DNA fragments for the purposes of SNP scanning, bacteria typing, methylation analysis, microsatellite analysis, genotyping, and nucleotide sequencing and re-sequencing.

### A. Genotyping

A glycosylase procedure was used to genotype the DNA sequence encoding UCP-2 (Uncoupling Protein 2). The sequence for UCP-2 is deposited in GenBank under accession number AF096289. The sequence variation genotyped in the following procedure was a cytosine (C-allele) to thymine (T-allele) variation at nucleotide position 4790, which results in a alanine to valine mutation at position 55 in the UCP-2 polypeptide.

DNA was amplified using a PCR procedure with a 50 µL reaction volume containing of 5 pmol biotinylated primer having the sequence 5'-TGCTTATCCCTGTAGCTACCCTGTCTTGGCCTTGCAGATCCAA-3' (SEQ ID NO: 91), 15 pmol non-biotinylated primer having the sequence 5'-AGCGGATAACAATTTCACACAGGCCATCACACCGCGGTACTG-3' (SEQ ID NO: 92), 200 µM dATP, 200 µM dCTP, 200 µM dGTP, 600 µM dUTP (to fully replace dTTP), 1.5 mM to 3 mM MgCl₂, 1 U of HotStarTaq polymerase, and 25 ng of CEPH DNA. Amplification was effected with 45 cycles at an annealing temperature of 56°C.

The amplification product was then immobilized onto a solid support by incubating 50µL of the amplification reaction with 5 µL of prewashed Dynabeads for 20 minutes at room temperature. The supernatant was removed, and the beads were incubated with 50 µL of 0.1 M NaOH for 5 minutes at room temperature to denature the double-stranded PCR product in such a fashion that single-stranded DNA was linked to the beads. The beads were then neutralized by three washes with 50 µL 10 mM TrisHCl (pH 8). The beads were resuspended in 10 µL of a 60mM TrisHCl/1 mM EDTA (pH 7.9) solution, and 1 U uracil DNA glycosylase was added to the solution for 45 minutes at 37°C to remove uracil nucleotides present in the single-stranded DNA linked to the beads. The beads were then washed two times with 25 µL of 10 mM TrisHCl (pH 8) and once with 10 µL of water. The biotinylated strands were then eluted from the beads with 12 µL of 2 M NH₄OH at 60°C for 10 minutes. The backbone of the DNA was cleaved by incubating the samples for 10 min at 95 °C (with a closed lid), and ammonia was evaporated from the samples by incubating the samples for 11 min at 80°C.

The cleavage fragments were then analyzed by MALDI-TOF mass spectrometry as described in Example 4. The T-allele generated a unique fragment of 3254 Daltons. The C-allele generated a unique fragment of 4788 Daltons. These fragements were distinguishable in mass spectra. Thus, the above-identified procedure was successfully utilized to genotype individuals heterozygous for the C-allele and T-allele in UCP-2.

### B. Glycosylase Analysis Utilizing Pooled DNA Samples

The glycosylase assay was conducted using pooled samples to detect genetic variability at the UCP-2 locus. DNA of known genotype was pooled from eleven individuals and was diluted to a fixed concentration of 5 ng/µL. The procedure provided in Example 3A was followed using 2 pmol of forward primer having a sequence of 5'-CCCAGTCACGACGTTGTAAAACGTCTTGGCCTTGCAGATCCAAG- 3' (SEQ ID NO: 93) and 15 pmol of reverse primer having the sequence 5'-AGCGGATAACAATTTCACACAGGCCATCACACCGCGGTACTG-3' (SEQ ID NO: 94). In addition, 5 pmol of biotinylated primer having the sequence 5'bioCCCAGTCACGACGTTGTAAAACG 3' (SEQ ID NO: 97) may be introduced to the PCR reaction after about two cycles. The fragments were analyzed via MALDI-TOF mass spectroscopy (Example 4). As determined in Example 3A, the T-allele, which generated a unique fragment of 3254 Daltons, could be distinguished in mass spectra from the C-allele, which generated a unique fragment of 4788 Daltons. Allelic frequency in the pooled samples was quantified by integrating the area under each signal corresponding to an allelic fragment. Integration was accomplished by hand calculations using equations well known to those skilled in the art. In the pool of eleven samples, this procedure suggested that 40.9% of the individuals harbored the T allele and 59.09% of the individuals harbored the C allele.

### C. Glycosylase-Mediated Microsatellite Analysis

A glycosylase procedure was utilized to identify microsatellites of the Bradykinin Receptor 2 (BKR-2) sequence. The sequence for BKR-2 is deposited in GenBank under accession number X86173. BKR-2 includes a SNP in the promoter region, which is a C to T variation, as well as a SNP in a repeated unit, which is a G to T variation. The procedure provided in Example 3A was utilized to identify the SNP in the promotor region, the SNP in the microsattelite repeat region, and the number of repeated units in the microsattelite region of BKR-2. Specifically, a forward PCR primer having the sequence 5'-CTCCAGCTGGGCAGGAGTGC-3' (SEQ ID NO: 95) and a reverse primer having the sequence 5'-CACTTCAGTCGCTCCCT-3' (SEQ ID NO: 96) were utilized to amplify BKR-2 DNA in the presence of uracil. The amplicon was fragmented by UDG followed by backbone cleavage. The cleavage fragments were analyzed by MALDI-TOF mass spectrometry as described in Example 4.

With regard to the SNP in the BKR-2 promotor region having a C to T variation, the C-allele generated a unique fragment having a mass of 7342.4 Daltons and the T-allele generated a unique fragment having a mass of 7053.2 Daltons. These fragments were distinguishable in mass spectra. Thus, the above-identified procedure was successfully utilized to genotype individuals heterozygous for the C-allele and T-allele in the promotor region of BKR-2.

With regard to the SNP in the BKR-2 repeat region having a G to T variation, the T-allele generated a unique fragment having a mass of 1784 Daltons, which was readily detected in a mass spectrum. Hence, the presence of the T-allele was indicative of the G to T sequence variation in the repeat region of BKR-2.

In addition, the number of repeat regions was distinguished between individuals having two repeat sequences and individuals having three repeat sequences in BKR-2. The DNA of these individuals did not harbor the G to T sequence variation in the repeat sequence as each repeat sequence contained a G at the SNP locus. The number of repeat regions was determined in individual samples by calculating the area under a signal corresponding to a unique DNA fragment having a mass of 2771.6 Daltons. This signal in spectra generated from individuals having two repeat regions had an area that was thirty-three percent less than the area under the same signal in spectra generated from individuals having three repeat regions. Thus, the procedures discussed above can be utilized to genotype individuals for the number of repeat sequences present in BKR-2.

### D. Bisulfite Treatment Coupled with Glycosylase Digestion

Bisulfite treatment of genomic DNA can be utilized to analyze positions of methylated cytosine residues within the DNA. Treating nucleic acids with bisulfite deaminates cytosine residues to uracil residues, while methylated cytosine remains unmodified. Thus, by comparing the sequence of a PCR product generated from genomic DNA that is not treated with bisulfite with the sequence of a PCR product generated from genomic DNA that is treated with bisulfite, the degree of methylation in a nucleic acid as well as the positions where cytosine is methylated can be deduced.

Genomic DNA (2 µg) was digested by incubation with 1 µL of a restriction enzyme at 37°C for 2 hours. An aliquot of 3 M NaOH was added to yield a final concentration of 0.3M NaOH in the digestion solution. The reaction was incubated at 37°C for 15 minutes followed by treatment with 5.35M urea, 4.44M bisulfite, and 10mM hydroquinone, where the final concentration of hydroquinone is 0.5 mM.

The sample that was treated with bisulfite (sample A) was compared to the same digestion sample that had not undergone bisulfite treatment (sample B). After sample A was treated with bisulfite as described above, sample A and sample B were amplified by a standard PCR procedure. The PCR procedure included the step of overlaying each sample with mineral oil and then subjecting the sample to thermocycling (20 cycles of 15 minutes at 55°C followed by 30 seconds at 95°C). The PCR reaction contained four nucleotide bases, C, A, G, and U. The mineral oil was removed from each sample, and the PCR products were purified with glassmilk. Sodium iodide (3 volumes) and glassmilk (5 µL) were added to samples A and B. The samples were then placed on ice for 8 minutes, washed with 420 µL cold buffer, centrifuged for 10 seconds, and the supernatant fractions were removed. This process was repeated twice and then 25 µL of water was added. Samples were incubated for 5 minutes at 37 °C, were centrifuged for 20 seconds, and the supernatant fraction was collected, and then this incubation/centrifugation/supernatant fraction collection procedure was repeated. 50 µL 0.1 M NaOH was then added to the samples to denature the DNA. The samples were incubated at room temperature for 5 minutes, washed three times with 50 µL of 10 mM TrisHCl (pH 8), and resuspended in 10 µL 60mM TrisHCl/1mM EDTA, pH 7.9.

The sequence of PCR products from sample A and sample B were then treated with 2U of UDG (MBI Fermentas) and then subjected to backbone cleavage, as described herein. The resulting fragments from each of sample A and sample B were analyzed by MALDI-TOF mass spectroscopy as described in Example 4. Sample A gave rise to a greater number of fragments than the number of fragments arising from sample B, indicative that the nucleic acid harbored at least one methylated cytosine moiety.

### EXAMPLE 7

### Fen-Ligase-Mediated Haplotyping

Haplotyping procedures permit the selection of a fragment from one of an individual's two homologous chromosomes and to genotype linked SNPs on that fragment. The direct resolution of haplotypes can yield increased information content, improving the diagnosis of any linked disease genes or identifying linkages associated with those diseases. In previous studies, haplotypes were typically reconstructed indirectly through pedigree analysis (in cases where pedigrees were available) through laborious and unreliable allele-specific PCR or through single-molecule dilution methods well known in the art.

A haplotyping procedure was used to determine the presence of two SNPs, referred to as SNP1 and SNP2, located on one strand in a DNA sample. The haplotyping procedure used in this assay utilized Fen-1, a site-specific "flap" endonuclease that cleaves DNA "flaps" created by the overlap of two oligonucleotides hybridized to a target DNA strand. The two overlapping oligonucleotides in this example were short arm and long arm allele-specific adaptors. The target DNA was an amplified nucleic acid that had been denatured and contained SNP1 and SNP2.

The short arm adaptor included a unique sequence not found in the target DNA. The 3' distal nucleotide of the short arm adaptor was identical to one of the SNP1 alleles. Moreover, the long arm adaptor included two regions: a 3' region complementary to the short arm and a 5'gene-specific region complementary to the fragment of interest adjacent to the SNP. If there was a match between the adaptor and one of the homologues, the Fen enzyme recognized and cleaved the overlapping flap. The short arm of the adaptor was then ligated to the remainder of the target fragment (minus the SNP site). This ligated fragment was used as the forward primer for a second PCR reaction in which only the ligated homologue was amplified. The second PCR product (PCR2) was then analyzed by mass spectrometry. If there was no match between the adaptors and the target DNA, there was no overlap, no cleavage by Fen-1, and thus no PCR2 product of interest.

If there was more than one SNP in the sequence of interest, the second SNP (SNP2) was found by using an adaptor that was specific for SNP2 and hybridizing the adaptor to the PCR2 product containing the first SNP. The Fen-ligase and amplification procedures were repeated for the PCR2 product containing the first SNP. If the amplified product yielded a second SNP, then SNP1 and SNP2 were on the same fragment.

If the SNP is unknown, then four allele-specific adaptors (e.g. C, G, A, and T) can be used to hybridize with the target DNA. The substrates are then treated with the Fen-ligase protocol, including amplification. The PCR2 products may be analyzed by PROBE, as described herein, to determine which adaptors were hybridized to the DNA target and thus identify the SNPs in the sequence.

A Fen-ligase assay was used to detect two SNPs present in Factor VII. These SNPs are located 814 base pairs apart from each other. SNP1 was located at position 8401 (C to T), and SNP2 was located at 9215 (G. to A) (SEQ ID #).

### A. First Amplification Step

A PCR product (PCR1) was generated for a known heterozygous individual at SNP1, a short distance from the 5' end of the SNP. Specifically, a 10 µL PCR reaction was performed by mixing 1.5 mM MgCl₂, 200 µM of each dNTP, 0.5 U HotStar polymerase, 0.1µM of a forward primer having the sequence 5'-GCG CTC CTG TCG GTG CCA (SEQ ID NO: 56), 0.1µM of a reverse primer having the sequence 5'-GCC TGA CTG GTG GGG CCC (SEQ ID NO: 57), and 1 ng of genomic DNA. The annealing temperature was 58°C, and the amplification process yielded fragments that were 861 bp in length.

The PCR1 reaction mixture was divided in half and was treated with an exonuclease 1/SAP mixture (0.22 µL mixture/5 µL PCR1 reaction) which contained 1.0µL SAP and 0.1 µL exon1. The exonuclease treatment was done for 30 minutes at 37 °C and then 20 minutes at 85°C to denature the DNA.

### B. Adaptor Oligonucleotides

A solution of allele-specific adaptors (C and T), containing of one long and one short oligonucleotide per adaptor, was prepared. The long arm and short arm oligonucleotides of each adaptor (10µM) were mixed in a 1:1 ratio and heated for 30 seconds at 95°C. The temperature was reduced in 2°C increments to 37°C for annealing. The C-adaptor had a short arm sequence of 5'-CAT GCA TGC ACG GTC (SEQ ID NO: 58) and a long arm sequence of 5'-CAG AGA GTA TCC CTC GAC CGT GCA TGC ATG (SEQ ID NO: 59). Hence, the long arm of the adaptor was 30 bp (15 bp gene-specific), and the short arm was 15bp. The T-adaptor had a short arm sequence of 5'-CAT GCA TGC ACG GTT (SEQ ID NO: 60) and a long arm sequence of 5'-GTA CGT ACG TGC CAA CTC CCC ATG AGA GAC (SEQ ID NO: 61). The adaptor could also have a hairpin structure in which the short and long arm are separated by a loop containing of 3 to 10 nucleotides (SEQ ID NO: 118).

### C. FEN-ligase reaction

In two tubes (one tube for each allele-specific adaptor per sample) was placed a solution (Solution A) containing of 3.5 µl 10 mM 16%PEG/50 mM MOPS, 1.2 µl 25 mM MgCl₂, 1.5 µl 10X Ampligase Buffer, and 2.5 µl PCR1. Each tube containing Solution A was incubated at 95°C for 5 minutes to denature the PCR1 product. A second solution (Solution B) containing of 1.65 µl Ampligase (Thermostable ligase, Epicentre Technologies), 1.65 µl 200ng/µl MFEN (from *Methanocuccus jannaschii*), and 3.0 µl of an allele specific adaptor (C or T) was prepared. Thus, different variations of Solution B, each variation containing of different allele-specific adaptors, were made. Solution B was added to Solution A at 95°C and incubated at 55°C for 3 hours. The total reaction volume was 15.0 µl per adaptor-specific reaction. For a bi-allelic system, 2 x 15.0 µl reactions were required.

The Fen-ligase reaction in each tube was then deactivated by adding 8.0 µl 10 mM EDTA. Then, 1.0 µl exoIII/Buffer (70%/30%) solution was added to each sample and incubated 30 minutes at 37°C, 20 minutes at 70°C (to deactivate exoIII), and 5 minutes at 95°C (to denature the sample and dissociate unused adaptor from template). The samples were cooled in an ice slurry and purified on UItraClean PCR Clean-up (MoBio) spin columns which removed all fragments less than 100 base pairs in length. The fragments were eluted with 50 µl H₂O.

### D. Second Amplification Step

A second amplification reaction (PCR2) was conducted in each sample tube using the short arm adaptor (C or T) sequence as the forward primer (minus the SNP1 site). Only the ligated homologue was amplified. A standard PCR reaction was conducted with a total volume of 10.0 µl containing of 1X Buffer (final concentration), 1.5 mM final concentration MgCl₂, 200 µM final concentration dNTPs, 0.5 U HotStar polymerase, 0.1 µM final concentration forward primer 5'-CAT GCA TGC ACG GT (SEQ ID NO: 62), 0.1µM final concentration reverse primer 5'-GCC TGA CTG GTG GGG CCC (SEQ ID NO: 63), and 1.0 µl of the purified FEN-ligase reaction solution. The annealing temperature was 58°C. The PCR2 product was analyzed by MALDI TOF mass spectroscopy as described in Example 4. The mass spectrum of Fen SNP1 showed a mass of 6084.08 Daltons, representing the C allele.

### E. Genotyping Additional SNPs

The second SNP (SNP2) can be found by using an adaptor that is specific for SNP2 and hybridizing that adaptor to the PCR2 product containing the first SNP. The Fen-ligase and amplification procedures are repeated for the PCR2 product containing the first SNP. If the amplified product yields a second SNP, then SN1 and SN2 are on the same fragment. The mass spectrum of SNP2, representing the T allele, showed a mass of 6359.88 Daltons.

This assay can also be performed upon pooled DNA to yield haplotype frequencies as described herein. The Fen-ligase assay can be used to analyze multiplexes as described herein.

### COMPARATIVE EXAMPLE 8

### Nickase-Mediated Sequence Analysis

A DNA nickase, or DNase, was used to recognize and cleave one strand of a DNA duplex. Two nickases usd were NY2A nickase and NYS1 nickase (Megabase) which cleave DNA at the following sites:
NY2A: 5'...R AG...3'
   3'...Y ↓ TCC...5' where R = A or G and Y = C or T
NYS1: 5'...↓ CC[A/G/T]...3'
   3'... GG[T/C/A]...5'.

### A. Nickase Digestion

Tris-HCl (10 mM), KCl (10 mM, pH 8.3), magnesium acetate (25 mM), BSA (1 mg/mL), and 6 U of Cvi NY2A or Cvi NYS1 Nickase (Megabase Research) were added to 25 pmol of double-stranded oligonucleotide template having a sequence of 5'-CGC AGG GTT TCC TCG TCG CAC TGG GCA TGT G-3' (SEQ ID NO: 90, Operon, Alameda, CA) synthesized using standard phosphoramidite chemistry . With a total volume of 20µL, the reaction mixture was incubated at 37°C for 5 hours, and the digestion products were purified using ZipTips (Millipore, Bedford, MA) as described in Example 5. The samples were analyzed by MALTY-TOM mass spectroscopy as described in Example 1. The nickase Cvi NY2A yielded three fragments with masses 4049.76 Daltons, 5473.14 Daltons, and 9540.71 Daltons. The Cvi NYS1 nickase yielded fragments with masses 2063.18 Daltons, 3056.48 Daltons, 6492.81 Daltons, and 7450.14 Daltons.

### B. Nickase Digestion of Pooled Samples

DQA (HLA ClassII-DQ Alpha, expected fragment size = 225bp) was amplified from the genomic DNA of 100 healthy individuals. DQA was amplified using standard PCR chemistry in a reaction having a total volume of 50 µL containing of 10 mM Tris-HCl, 10 mM KCI (pH 8.3), 2.5 mM MgCl₂, 200 µM of each dNTP, 10 pmol of a forward primer having the sequence 5'-GTG CTG CAG GTG TAA ACT TGT ACC AG-3'(SEQ. ID NO: 64), 10 pmol of a reverse primer having the sequence 5'-CAC GGA TCC GGT AGC AGC GGT AGA GTT G-3'(SEQ ID NO: 65), 1 U DNA polymerase (Stoffel fragment, Perkin Elme r), and 200ng human genomic DNA (2ng DNA/individual). The template was denatured at 94°C for 5 minutes. Thermal cycling was continued with a touch-down program that included 45 cycles of 20 seconds at 94°C, 30 seconds at 56°C, 1 minute at 72°C, and a final extension of 3 minutes at 72°C. The crude PCR product was used in the subsequent nickase reaction.

The unpurified PCR product was subjected to nickase digestion. Tris-HCl (10 mM), KCI (10 mM, pH 8.3), magnesium acetate (25mM), BSA (1 mg/mL), and 5 U of Cvi NY2A or Cvi NYS1 Nickase (Megabase Research) were added to 25 pmol of the amplified template with a total reaction volume of 20µL. The mixture was then incubated at 37°C for 5 hours. The digestion products were purified with either ZipTips (Millipore, Bedford, MA) as described in Example 5. The samples were analyzed by MALDI-TOF mass spectroscopy as described in Example 4. This assay can also be used to do multiplexing and standardless genotyping as described herein.

To simplify the nickase mass spectrum, the two complementary strands can be separated after digestion by using a single-stranded undigested PCR product as a capture probe. This probe (preparation shown below in Example 8C) can be hybridized to the nickase fragments in hybridization buffer containing 200 mM sodium citrate and 1 % blocking reagent (Boehringer Mannheim). The reaction is heated to 95°C for 5 minutes and cooled to room temperature over 30 minutes by using a thermal cycler (PTC-200 DNA engine, MJ Research, Waltham, MA). The capture probe-nickase fragment is immobilized on 140 µg of streptavidin-coated magnetic beads. The beads are subsequently washed three times with 70 mM ammonium citrate. The captured single-stranded nickase fragments are eluted by heating to 80°C for 5 minutes in 5 µL of 50 mM ammonium hydroxide.

### C. Preparation of Capture Probe

The capture probe is prepared by amplifying the human β-globin gene (3' end of intron 1 to 5' end of exon 2) via PCR methods in a total volume of 50 µL containing of GeneAmp 1XPCR Buffer II, 10 mM Tris-HCl, pH 8.3, 50 mM KCI, 2 mM MgCl₂, 0.2 mM dNTP mix, 10pmol of each primer (forward primer 5'-ACTGGGCATGTGGAGACAG-3'(SEQ ID NO: 66) and biotinylated reverse primer bio5'-GCACTTTCTTGCCATGAG-3'(SEQ ID: 67), 2 U of AmpliTaq Gold, and 200 ng of human genomic DNA. The template is denatured at 94°C for 8 minutes. Thermal cycling is continued with a touch-down program that included 11 cycles of 20 seconds at 94°C, 30 seconds at 64°C, 1 minute at 72°C; and a final extension of 5 minutes at 72°C. The amplicon is purified using UltraClean" PCR clean-up kit (MO Bio Laboratories, Solano Beach, CA).

### COMPARATIVE EXAMPLE 9

### Multiplex Type IIS SNP Assay

A Type IIS assay was used to identify human gene sequences with known SNPs. The Type IIS enzyme used in this assay was Fok I which effected double-stranded cleavage of the target DNA. The assay involved the steps of amplification and Fok I treatment of the amplicon. In the amplification step, the primers were designed so that each PCR product of a designated gene target was less than 100 bases such that a Fok I recognition sequence was incorporated at the 5' and 3' end of the amplicon. Therefore, the fragments that were cleaved by Fok I included a center fragment containing the SNP of interest.

Ten human gene targets with known SNPs were analyzed by this assay. Sequences of the ten gene targets, as well as the primers used to amplify the target regions, are found in Table 5. The ten targets were lipoprotein lipase, prothrombin, factor V, cholesterol ester transfer protein (CETP), factor VII, factor XIII, HLA-H exon 2, HLA-H exon 4, methylenetetrahydrofolate reductase (MTHR), and P53 exon 4 codon 72.

Amplification of the ten human gene sequences were carried out in a single 50 µL volume PCR reaction with 20 ng of human genomic DNA template in 5 PCR reaction tubes. Each reaction vial contained 1X PCR buffer (Qiagen), 200µM dNTPs, 1 U Hotstar Taq polymerase (Qiagen), 4 mM MgCl₂, and 10pmol of each primer. US8, having sequence of 5'TCAGTCACGACGTT3'(SEQ ID NO: 68), and US9, having sequence of 5'CGGATAACAATTTC3'(SEQ ID NO: 69), were used for the forward and reverse primers respectively. Moreover, the primers were designed such that a Fok I recognition site was incorporated at the 5' and 3' ends of the amplicon. Thermal cycling was performed in 0.2 mL tubes or a 96 well plate using a MJ Research Thermal Cycler (calculated temperature) with the following cycling parameters: 94°C for 5 minutes; 45 cycles: 94°C for 20 seconds, 56°C for 20 seconds, 72°C for 60 seconds; and 72°C for 3 minutes.

Following PCR, the sample was treated with 0.2 U Exonuclease I (Amersham Pharmacia) and S Alkaline Phosphotase (Amersham Pharmacia) to remove the unincorporated primers and dNTPs. Typically, 0.2 U of exonuclease I and SAP were added to 5 µL of the PCR sample. The sample was then incubated at 37°C for 15 minutes. Exonuclease I and SAP were then inactivated by heating the sample up to 85°C for 15 minutes. Fok I digestion was performed by adding 2 U of Fok I (New England Biolab) to the 5 uL PCR sample and incubating at 37°C for 30 minutes. Since the Fok I restriction sites are located on both sides of the amplicon, the 5' and 3' cutoff fragments have higher masses than the center fragment containing the SNP. The sample was then purified by anion exchange and analyzed by MALDI-TOF mass spectrometry as described in Example 4. The masses of the gene fragments from this multiplexing experiment are listed in Table 6. These gene fragments were resolved in mass spectra thereby allowing multiplex analysis of sequence variability in these genes.

**Table 5 Genes for Multiplex Type IIS Assay**

| Gene | Sequence | Seq. ID No. | | Seq. ID No. |
|---|---|---|---|---|
| Lipoprotein Lipase (Asn291Ser) | cctttgagaa agggctctgc ttgagttgta gaaagaaccg ctgcaacaat ctgggctatg agatca[a▸g]taa agtcagagcc aaaagaagca gcaaaatgta | 98-99 | 5' caatttcatcgctggatgcaatct gggctatgagatc 3' 5' caatttcacacagcggatgcttct tttggctctgact 3' | 70 71 |
| Prothrombin | 26731 gaattatttt tgtgtttcta aaactatggt tcccaataaa agtgactctc 26781 agc[g▸a]agcctc aatgctccca gtgctattca tgggcagctc tctgggctca | 100-101 | 5' tcagtcacgacgttggatgccaa taaaagtgactctcagc 3' 5' cggataacaatttcggatgcact gggagcattgaggc 3' | 72 73 |
| Factor V (Arg506Gln) | taataggact acttctaatc tgtaagagca gatccctgga caggc[g>a]agga | 102-103 | 5' tcagtcacgacgttggatgagca gatccctggacaggc 3' 5' cggataacaatttcggatggaca aaatacctgtattcc 3' | 74 75 |
| Cholesterol ester transfer protein (CETP) (I405V) | 1261 ctcaccatgg gcatttgatt gcagagcagc tccgagtcc[g>a] tccagagctt 1311 cctgcagtca atgatcaccg ctgtgggcat ccctgaggtc atgtctcgta | 104-105 | 5′ tcagtcacgacgttggatgcaga gcagctccgagtc 3′ 5′ cagcggtgatcattggatgcagg aagctctgg 3′ | 76 77 |
| Factor VII (R353Q) | 1221 agcaaggact cctgcaaggg ggacagtgga ggcccacatg ccacccacta 1271 cc[a▸g]gggcacg tggtacctga cgggcatcgt cagctggggc cagggctgcg | 106-107 | 5' tcagtcacgacgttggatgccca catgccacccactac 3' 5' cggataacaatttcggatgcccg tcaggtaccacg 3' | 78 79 |
| Factor XIII (V34L) | 111 caataactct aatgcagcgg aagatgacct gcccacagtg gagcttcagg 161 gc[g►t]tggtgcc ccggggcgtc aacctgcaag gtatgagcat acccccttc | 108-109 | 5' tcagtcacgacgttggatgccca cagtggagcttcag 3' 5' gctcataccttgcaggatgacg 3' | 80 81 |
| HLA-H exon 2 (His63Asp) | 361 ttgaagcttt gggctacgtg gatgaccagc tgttcgtgtt ctatgat(c▸g)at 411 gagagtcgcc gtgtggagcc ccgaactcca tgggtttcca gtagaatttc | 110-111 | 5' tcagtcacgacgttggatgacca gctgttcgtgttc 3' 5' tacatggagttcggggatgcaca cggcgactctc 3' | 82 83 |
| HLA-H exon 4 (Cys282Tyr) | 1021 ggataacctt ggctgtaccc cctggggaag agcagagata tacgt[g▸a]ccag 1071 gtggagcacc caggcctgga tcagcccctc attgtgatct gggagccctc | 112-113 | 5' tcagtcacgacgttggatgggga agagcagagatatacgt 3' 5' gaggggctgatccaggatgggt gctccac 3' | 84 85 |
| Methylentetrahy drofolateredctas e (MTHR) (Ala222Val) | 761 tgaagcactt gaagga gaag gtgtctgcgg gag[c▸t]cgattt catcatcacg 811 cagcttttct ttgaggctga cacattcttc | 114-115 | 5' tcagtcacgacgttggatggggga agagcagagatatacgt 3' 5' gaggggctgatccaggatgggt gctccac 3' | 86 87 |
| P53 Exon4 Codon 72 (Arg72Pro) | 12101 tccagatgaa gctcccagaa tgccagaggc tgctcccc[g►c]c gtggcccctg 12151 caccagcagc tcctacaccg gcggcccctg | 116-117 | 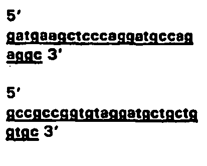 | 88 89 |

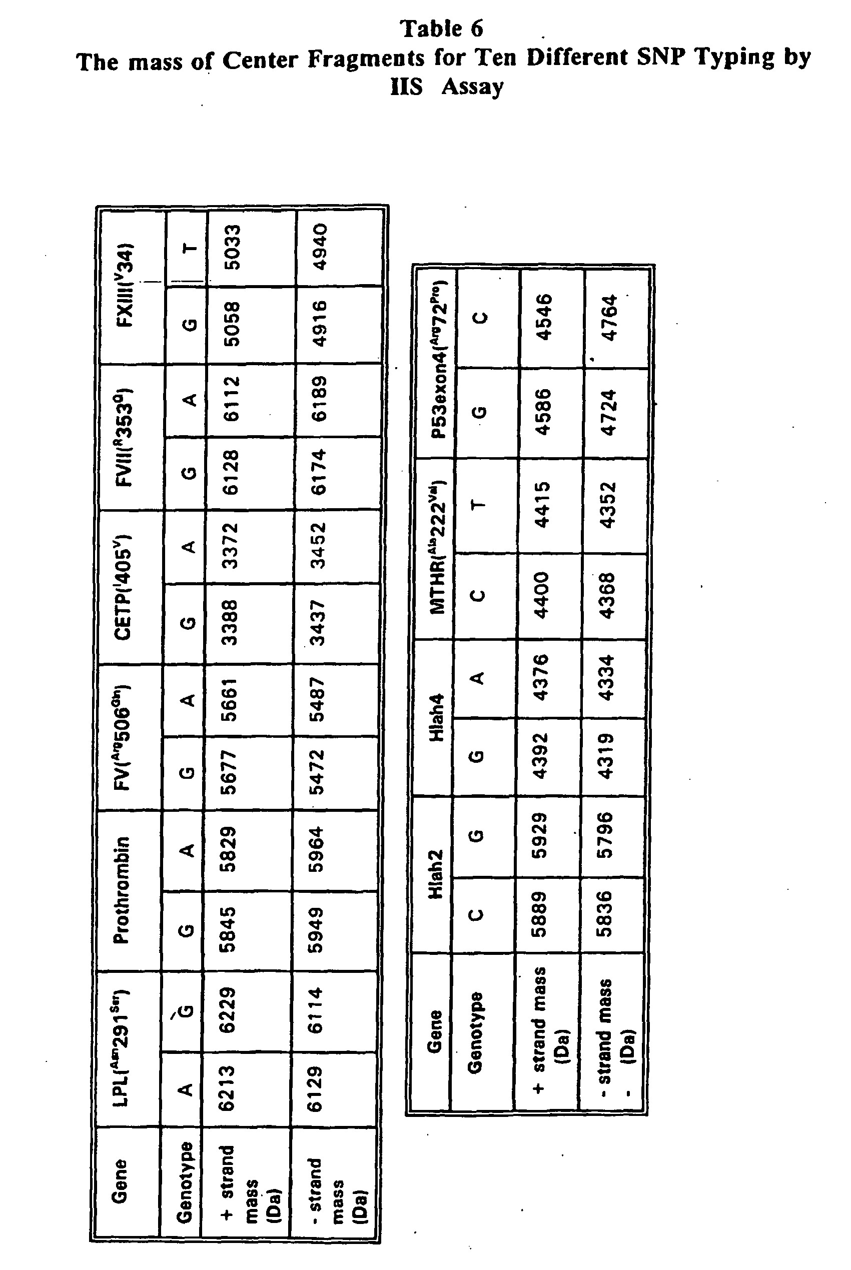

### COMPARATIVE EXAMPLE 10

### Exemplary use of parental medical history parameter for stratification of healthy database

A healthy database can be used to associate a disease state with a specific allele (SNP) that has been found to show a strong association between age and the allele, in particular the homozygous genotype. The method involves using the same healthy database used to identify the age dependent association, however stratification is by information given by the donors about common disorders from which their parents suffered (the donor's familial history of disease). There are three possible answers a donor could give about the health status of their parents: neither were affected, one was affected or both were affected. Only donors above a certain minimum age, depending on the disease, are utilized, as the donors parents must be old enough to to have exhibited clinical disease phenotypes. The genotype frequency in each of these groups is determined and compared with each other. If there is an association of the marker in the donor to a disease the frequency of the heterozyous genotype will be increased. The frequency of the homozygous genotype should not increase, as it should be significantly underrepresented in the healthy population.

### COMPARATIVE EXAMPLE 11

### Method and Device for Identifying a Biological Sample

### Description

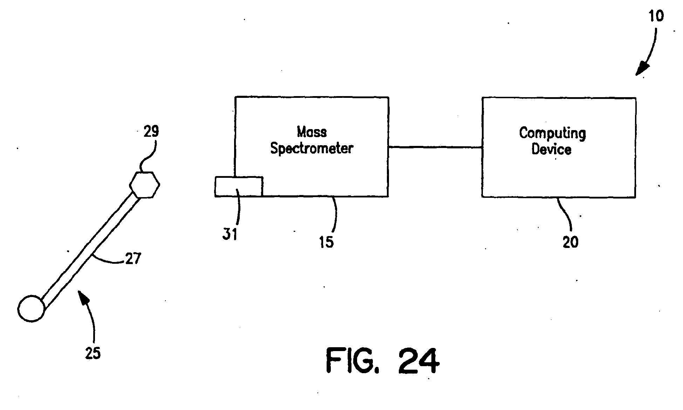

Referring now to FIG. 24, an apparatus 10 for identifying a biological sample is disclosed. The apparatus 10 for identifying a biological sample generally comprises a mass spectrometer 15 communicating with a computing device 20. The mass spectrometer may be a MALDI-TOF mass spectrometer manufactured by Bruker-Franzen Analytik GmbH; however, it will be appreciated that other mass spectrometers can be substituted. The computing device 20 is preferably a general purpose computing device. However, it will be appreciated that the computing device could be alternatively configured, for example, it may be integrated with the mass spectrometer or could be part of a computer in a larger network system.

The apparatus 10 for identifying a biological sample may operate as an automated identification system having a robot 25 with a robotic arm 27 configured to deliver a sample plate 29 into a receiving area 31 of the mass spectrometer 15. In such a manner, the sample to be identified may be placed on the plate 29 and automatically received into the mass spectrometer 15. The biological sample is then processed in the mass spectrometer to generate data indicative of the mass of DNA fragments in the biological sample. This data may be sent directly to computing device 20, or may have some preprocessing or filtering performed within the mass spectrometer. In a preferred embodiment, the mass spectrometer 15 transmits unprocessed and unfiltered mass spectrometry data to the computing device 20. However, it will be appreciated that the analysis in the computing device may be adjusted to accommodate preprocessing or filtering performed within the mass spectrometer.

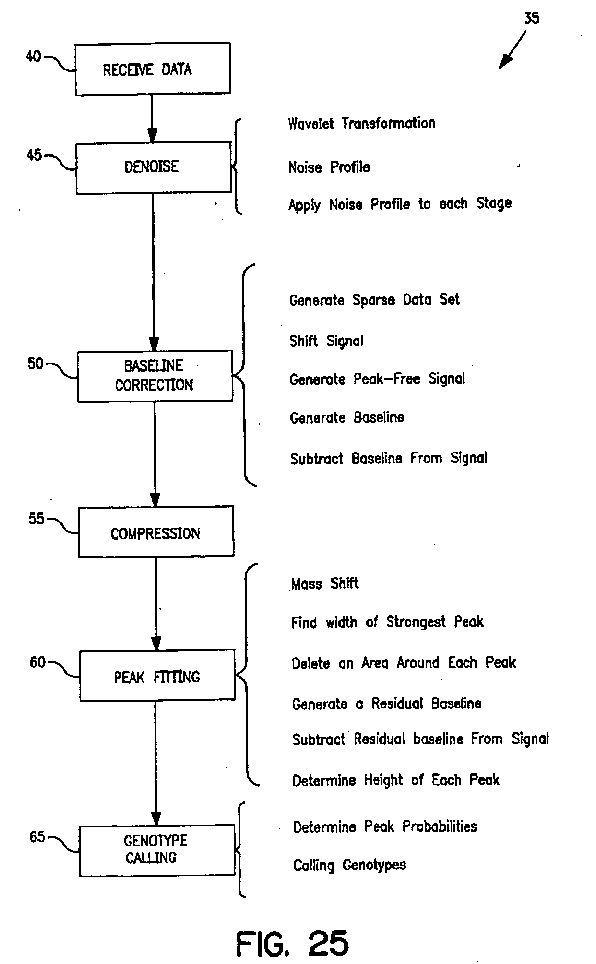

Referring now to FIG. 25, a general method 35 for identifying a biological sample is shown. In method 35, data is received into a computing device from a test instrument in block 40. Preferably the data is received in a raw, unprocessed and unfiltered form, but alternatively may have some form of filtering or processing applied. The test instrument of a preferred embodiment is a mass spectrometer as described above. However, it will be appreciated that other test instruments could be substituted for the mass spectrometer.

The data generated by the test instrument, and in particular the mass spectrometer, includes information indicative of the identification of the biological sample. More specifically, the data is indicative of the DNA composition of the biological sample. Typically, mass spectrometry data gathered from DNA samples obtained from DNA amplification techniques are noisier than, for example, those from typical protein samples. This is due in part because protein samples are more readily prepared in more abundance, and protein samples are more easily ionizable as compared to. DNA samples. Accordingly, conventional mass spectrometer data analysis techniques are generally ineffective for DNA analysis of a biological sample. To improve the analysis capability so that DNA composition data can be more readily discerned, a preferred embodiment uses wavelet technology for analyzing the DNA mass spectrometry data. Wavelets are an analytical tool for signal processing, numerical analysis, and mathematical modeling. Wavelet technology provides a basic expansion function which is applied to a data set. Using wavelet decomposition, the data set can be simultaneously analyzed in the time and frequency domains. Wavelet transformation is the technique of choice in the analysis of data that exhibit complicated time (mass) and frequency domain information, such as MALDI-TOF DNA data. Wavelet transforms as described herein have superior denoising properties as compared to conventional Fourier analysis techniques. Wavelet transformation has proven to be particularly effective in interpreting the inherently noisy MALDI-TOF spectra of DNA samples. In using wavelets, a "small wave" or "scaling function" is used to transform a data set into stages, with each stage representing a frequency component in the data set. Using wavelet transformation, mass spectrometry data can be processed, filtered, and analyzed with sufficient discrimination to be useful for identification of the DNA composition for a biological sample.

Referring again to FIG. 25, the data received in block 40 is denoised in block 45. The denoised data then has a baseline correction applied in block 50. A baseline correction is generally necessary as data coming from the test instrument, in particular a mass spectrometer instrument, has data arranged in a generally exponentially decaying manner. This generally exponential decaying arrangement is not due to the composition of the biological sample, but is a result of the physical properties and characteristics of the test instrument, and other chemicals involved in DNA sample preparation. Accordingly, baseline correction substantially corrects the data to remove a component of the data attributable to the test system, and sample preparation characteristics.

After denoising in block 45 and the baseline correction in block 50, a signal remains which is generally indicative of the composition of the biological sample. However, due to the extraordinary discrimination required for analyzing the DNA composition of the biological sample, the composition is not readily apparent from the denoised and corrected signal. For example, although the signal may include peak areas, it is not yet clear whether these "putative" peaks actually represent a DNA composition, or whether the putative peaks are result of a systemic or chemical aberration. Further, any call of the composition of the biological sample would have a probability of error which would be unacceptable for clinical or therapeutic purposes. In such critical situations, there needs to be a high degree of certainty that any call or identification of the sample is accurate. Therefore, additional data processing and interpretation is necessary before the sample can be accurately and confidently identified.

Since the quantity of data resulting from each mass spectrometry test is typically thousands of data points, and an automated system may be set to perform hundreds or even thousands of tests per hour, the quantity of mass spectrometry data generated is enormous. To facilitate efficient transmission and storage of the mass spectrometry data, block 55 shows that the denoised and baseline corrected data is compressed.

In one example, the biological sample is selected and processed to have only a limited range of possible compositions. Accordingly, it is therefore known where peaks indicating composition should be located, if present. Taking advantage of knowing the location of these expected peaks, in block 60 the method 35 matches putative peaks in the processed signal to the location of the expected peaks. In such a manner, the probability of each putative peak in the data being an actual peak indicative of the composition of the biological sample can be determined. Once the probability of each peak is determined in block 60, then in block 65 the method 35 statistically determines the composition of the biological sample, and determines if confidence is high enough to calling a genotype.

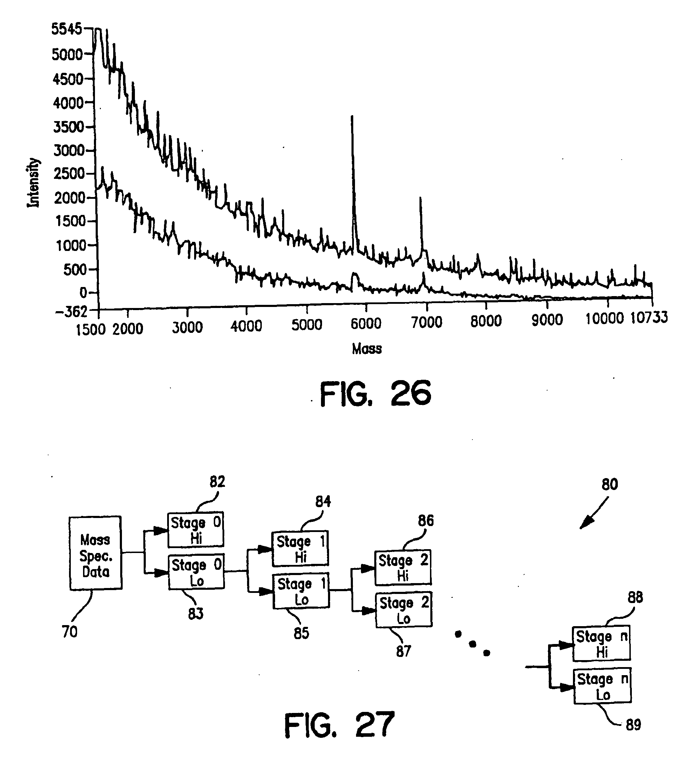

Referring again to block 40, data is received from the test instrument, which is preferably a mass spectrometer. In a specific illustration, FIG. 26 shows an example of data from a mass spectrometer. The mass spectrometer data 70 generally comprises data points distributed along an x-axis 71 and a y-axis 72. The x-axis 71 represents the mass of particles detected, while the y-axis 72 represents a numerical concentration of the particles. As can be seen in FIG. 26, the mass spectrometry data 70 is generally exponentially decaying with data at the left end of the x-axis 73 generally decaying in an exponential manner toward data at the heavier end 74 of the x-axis 71. However, the general exponential presentation of the data is not indicative of the composition of the biological sample, but is more reflective of systematic error and characteristics. Further, as described above and illustrated in FIG. 26, considerable noise exists in the mass spectrometry DNA data 70.

Referring again to block 45, where the raw data received in block 40 is denoised, the denoising process will be described in more detail. As illustrated in FIG. 25, the denoising process generally entails 1) performing a wavelet transformation on the raw data to decompose the raw data into wavelet stage coefficients; 2) generating a noise profile from the highest stage of wavelet coefficients; and 3) applying a scaled noise profile to other stages in the wavelet transformation. Each step of the denoising process is further described below.

Referring now to FIG. 27, the wavelet transformation of the raw mass spectrometry data is generally diagramed. Using wavelet transformation techniques, the mass spectrometry data 70 is sequentially transformed into stages. In each stage the data is represented in a high stage and a low stage, with the low stage acting as the input to the next sequential stage. For example, the mass spectrometry data 70 is transformed into stage 0 high data 82 and stage 0 low data 83. The stage 0 low data 83 is then used as an input to the next level transformation to generate stage 1 high data 84 and stage 1 low data 85. In a similar manner, the stage 1 low data 85 is used as an input to be transformed into stage 2 high data 86 and stage 2 low data 87. The transformation is continued until no more useful information can be derived by further wavelet transformation. For example, in the preferred embodiment a 24-point wavelet is used. More particularly a wavelet commonly referred to as the Daubechies 24 is used to decompose the raw data. However, it will be appreciated that other wavelets can be used for the wavelet transformation. Since each stage in a wavelet transformation has one-half the data points of the previous stage, the wavelet transformation can be continued until the stage n low data 89 has around 50 points. Accordingly, the stage n high 88 would contain about 100 data points. Since the preferred wavelet is 24 points long, little data or information can be derived by continuing the wavelet transformation on a data set of around 50 points.

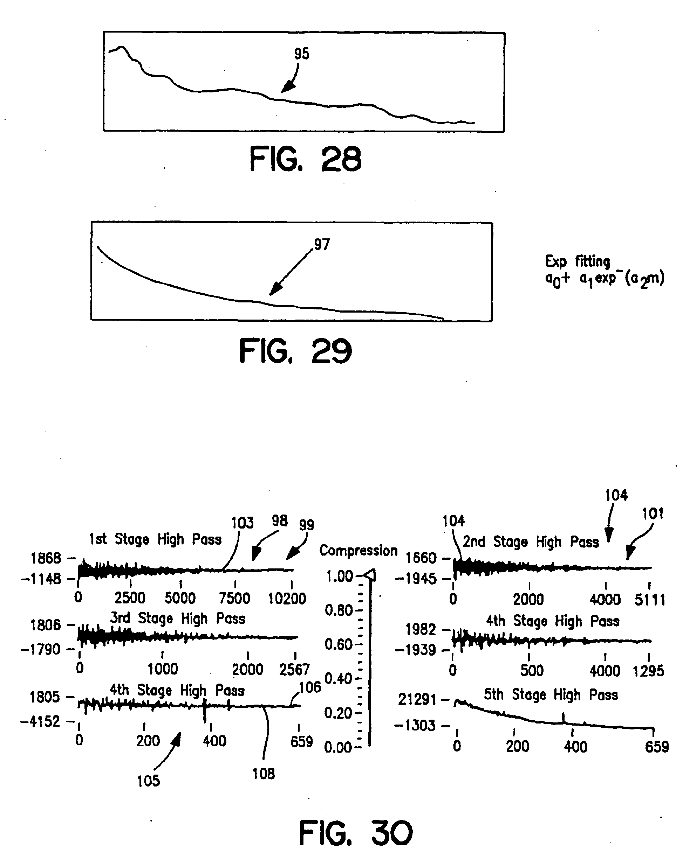

FIG. 28 shows an example of stage 0 high data 95. Since stage 0 high data 95 is generally indicative of the highest frequencies in the mass spectrometry data, stage 0 high data 95 will closely relate to the quantity of high frequency noise in the mass spectrometry data. In FIG. 29, an exponential fitting formula has been applied to the stage 0 high data 95 to generate a stage O noise profile 97. In particular, the exponential fitting formula is in the format A₀ + A₁ EXP (-A₂ m). It will be appreciated that other expediential fitting formulas or other types of curve fits may be used.

Referring now to FIG. 30, noise profiles for the other high stages are determined. Since the later data points in each stage will likely be representative of the level of noise in each stage, only the later data points in each stage are used to generate a standard deviation figure that is representative of the noise content in that particular stage. More particularly, in generating the noise profile for each remaining stage, only the last five percent of the data points in each stage are analyzed to determined a standard deviation number. It will be appreciated that other numbers of points, or alternative methods could be used to generate such a standard deviation figure.

The standard deviation number for each stage is used with the stage 0 noise profile (the exponential curve) 97 to generate a scaled noise profile for each stage. For example, FIG. 30 shows that stage 1 high data 98 has stage 1 high data 103 with the last five percent of the data points represented by area 99. The points in area 99 are evaluated to determine a standard deviation number indicative of the noise content in stage 1 high data 103. The standard deviation number is then used with the stage 0 noise profile 97 to generate a stage 1 noise profile.

In a similar manner, stage 2 high 100 has stage 2 high data 104 with the last five percent of points represented by area 101. The data points in area 101 are then used to calculate a standard deviation number which is then used to scale the stage 0 noise profile 97 to generate a noise profile for stage 2 data. This same process is continued for each of the stage high data as shown by the stage n high 105. For stage n high 105, stage n high data 108 has the last five percent of data points indicated in area 106. The data points in area 106 are used to determine a standard deviation number for stage n. The stage n standard deviation number is then used with the stage 0 noise profile 97 to generate a noise profile for stage n. Accordingly, each of the high data stages has a noise profile.

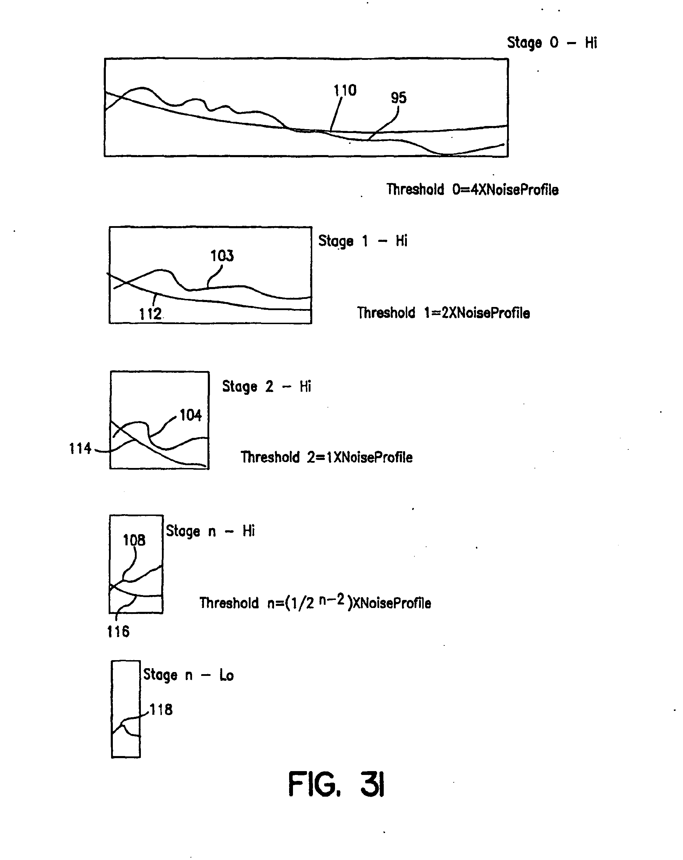

FIG. 31 shows how the noise profile is applied to the data in each stage. Generally, the noise profile is used to generate a threshold which is applied to the data in each stage. Since the noise profile is already scaled to adjust for the noise content of each stage, calculating a threshold permits further adjustment to tune the quantity of noise removed. Wavelet coefficients below the threshold are ignored while those above the threshold are retained. Accordingly, the remaining data has a substantial portion of the noise content removed.

Due to the characteristics of wavelet transformation, the lower stages, such as stage 0 and 1, will have more noise content than the later stages such as stage 2 or stage n. Indeed, stage n low data is likely to have little noise at all. Therefore, in a preferred embodiment the noise profiles are applied more aggressively in the lower stages and less aggressively in the later stages. For example, FIG. 31 shows that stage 0 high threshold is determined by multiplying the stage 0 noise profile by a factor of four. In such a manner, significant numbers of data points in stage 0 high data 95 will be below the threshold and therefore eliminated. Stage 1 high threshold 112 is set at two times the noise profile for the stage 1 high data, and stage 2 high threshold 114 is set equal to the noise profile for stage 2 high. Following this geometric progression, stage n high threshold 116 is therefore determined by scaling the noise profile for each respective stage n high by a factor equal to (1/2ⁿ⁻²). It will be appreciated that other factors may be applied to scale the noise profile for each stage. For example, the noise profile may be scaled more or less aggressively to accommodate specific systemic characteristics or sample compositions. As indicated above, stage n low data does not have a noise profile applied as stage n low data 118 is assumed to have little or no noise content. After the scaled noise profiles have been applied to each high data stage, the mass spectrometry data 70 has been denoised and is ready for further processing. A wavelet transformation of the denoised signal results in the sparse data set 120 as shown in FIG. 31.

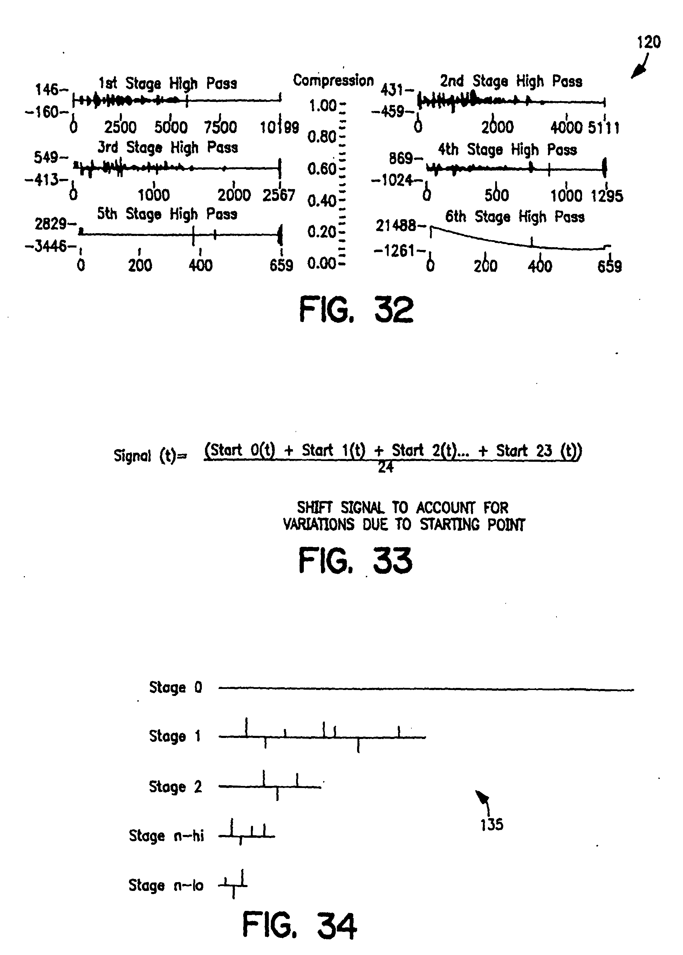

Referring again to FIG. 25, the mass spectrometry data received in block 40 has been denoised in block 45 and is now passed to block 50 for baseline correction. Before performing baseline correction, the artifacts introduced by the wavelet transformation procedure are preferably removed. Wavelet transformation results vary slightly depending upon which point of the wavelet is used as a starting point. For example, the preferred embodiment uses the 24-point Daubechies-24 wavelet. By starting the transformation at the 0 point of the wavelet, a slightly different result will be obtained than if starting at points 1 or 2 of the wavelet. Therefore, the denoised data is transformed using every available possible starting point, with the results averaged to determine a final denoised and shifted signal. For example, FIG. 33 shows that the wavelet coefficient is applied 24 different times and then the results averaged to generate the final data set. It will be appreciated that other techniques may be used to accommodate the slight error introduced due to wavelet shifting.

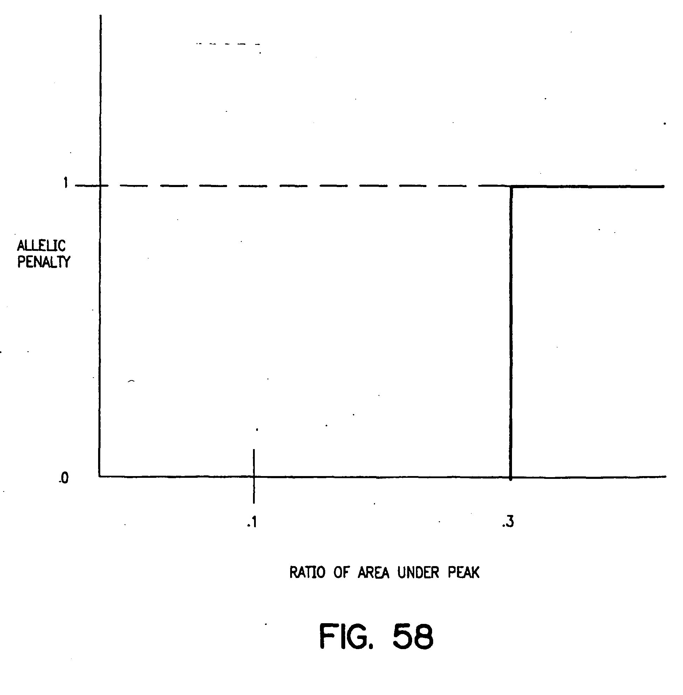

The formula 125 is generally indicated in FIG. 33. Once the signal has been denoised and shifted, a denoised and shifted signal 130 is generated as shown in FIG. 58. FIG. 34 shows an example of the wavelet coefficient 135 data set from the denoised and shifted signal 130.

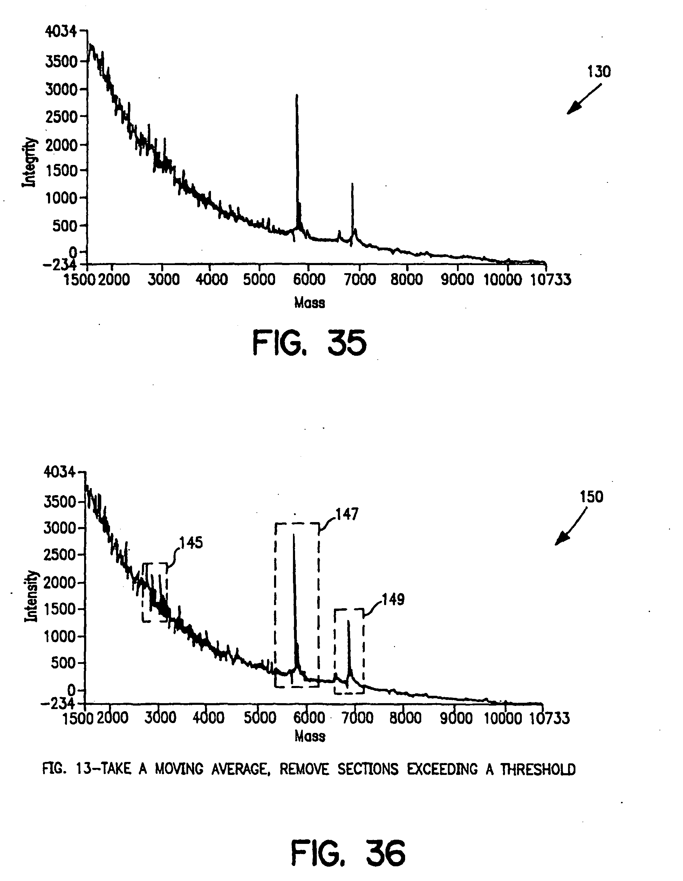

FIG. 36 shows that putative peak areas 145, 147, and 149 are located in the denoised and shifted signal 150. The putative peak areas are systematically identified by taking a moving average along the signal 150 and identifying sections of the signal 150 which exceed a threshold related to the moving average. It will be appreciated that other methods can be used to identify putative peak areas in the signal 150.

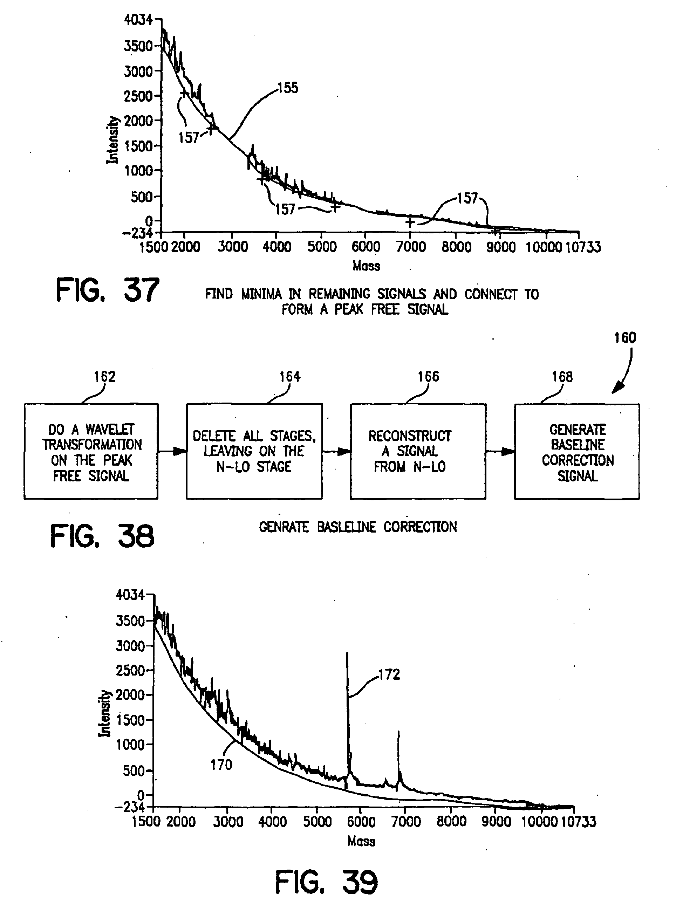

Putative peak areas 145, 147 and 149 are removed from the signal 150 to create a peak-free signal 155 as shown in FIG. 37. The peak-free signal 155 is further analyzed to identify remaining minimum values 157, and the remaining minimum values 157 are connected to generate the peak-free signal 155.

FIG. 38 shows a process of using the peak-free signal 155 to generate a baseline 170 as shown in FIG. 39. As shown in block 162, a wavelet transformation is performed on the peak-free signal 155. All the stages from the wavelet transformation are eliminated in block 164 except for the n low stage. The n low stage will generally indicate the lowest frequency component of the peak-free signal 155 and therefore will generally indicate the system exponential characteristics. Block 166 shows that a signal is reconstructed from the n low coefficients and the baseline signal 170 is generated in block 168.

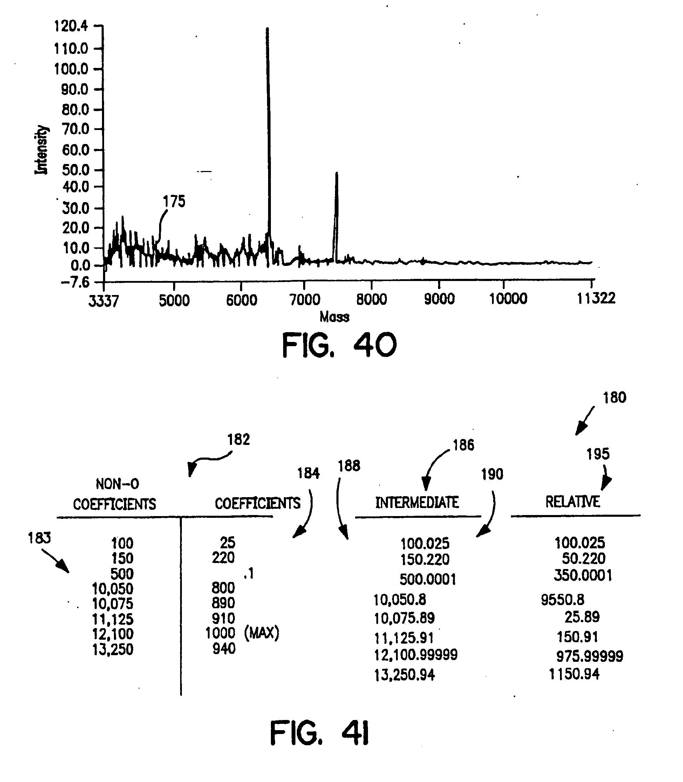

FIG. 39 shows a denoised and shifted data signal 172 positioned adjacent a correction baseline 170. The baseline correction 170 is subtracted from the denoised and shifted signal 172 to generate a signal 175 having a baseline correction applied as shown in FIG. 40. Although such a denoised, shifted, and corrected signal is sufficient for most identification purposes, the putative peaks in signal 175 are not identifiable with sufficient accuracy or confidence to call the DNA composition of a biological sample.

Referring again to FIG. 25, the data from the baseline correction 50 is now compressed in block 55, the compression technique used in a preferred embodiment is detailed in FIG. 41. In FIG. 41 the data in the baseline corrected data is presented in an array format 182 with x-axis points 183 having an associated data value 184. The x-axis is indexed by the non-zero wavelet coefficients, and the associated value is the value of the wavelet coefficient. In the illustrated data example in table 182, the maximum value 184 is indicated to be 1000. Although a particularly advantageous compression technique for mass spectrometry data is shown, it will be appreciated that other compression techniques can be used. Although not preferred, the data may also be stored without compression.

In compressing the data, an intermediate format 186 is generated. The intermediate format 186 generally comprises a real number having a whole number portion 188 and a decimal portion 190. The whole number portion is the x-axis point 183 while the decimal portion is the value data 184 divided by the maximum data value. For example, in the data 182 a data value "25" is indicated at x-axis point "100". The intermediate value for this data point would be "100.025".

From the intermediate compressed data 186 the final compressed data 195 is generated. The first point of the intermediate data file becomes the starting point for the compressed data. Thereafter each data point in the compressed data 195 is calculated as follows: the whole number portion (left of the decimal) is replaced by the difference between the current and the last whole number. The remainder (right of the decimal) remains intact. For example, the starting point of the compressed data 195 is shown to be the same as the intermediate data point which is "100.025". The comparison between the first intermediate data point "100.025" and the second intermediate data point "150.220" is "50.220". Therefore, "50.220" becomes the second point of the compressed data 195. In a similar manner, the second intermediate point is "150.220" and the third intermediate data point is "500.0001 ". Therefore, the third compressed data becomes "350.000". The calculation for determining compressed data points is continued until the entire array of data points is converted to a single array of real numbers.

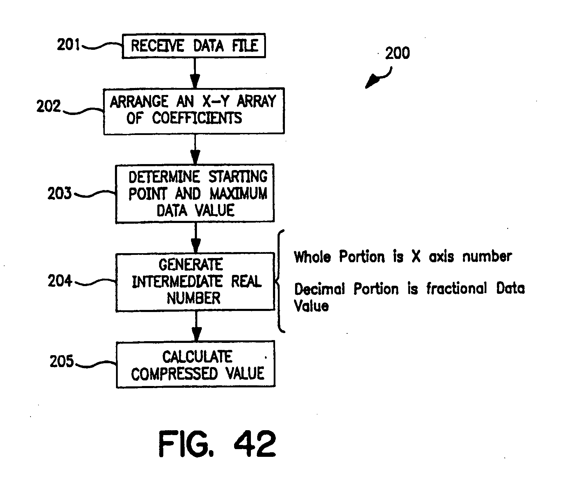

FIG. 42 generally describes the method of compressing mass spectrometry data, showing that the data file in block 201 is presented as an array of coefficients in block 202. The data starting point and maximum is determined as shown in block 203, and the intermediate real numbers are calculated in block 204 as described above. With the intermediate data points generated, the compressed data is generated in block 205. The described compression method is highly advantageous and efficient for compressing data sets such as a processed data set from a mass spectrometry instrument. The method is particularly useful for data, such as mass spectrometry data, that uses large numbers and has been processed to have occasional lengthy gaps in x-axis data. Accordingly, an x-y data array for processed mass spectrometry data may be stored with an effective compression rate of 10x or more. Although the compression technique is applied to mass spectrometry data, it will be appreciated that the method may also advantageously be applied to other data sets.

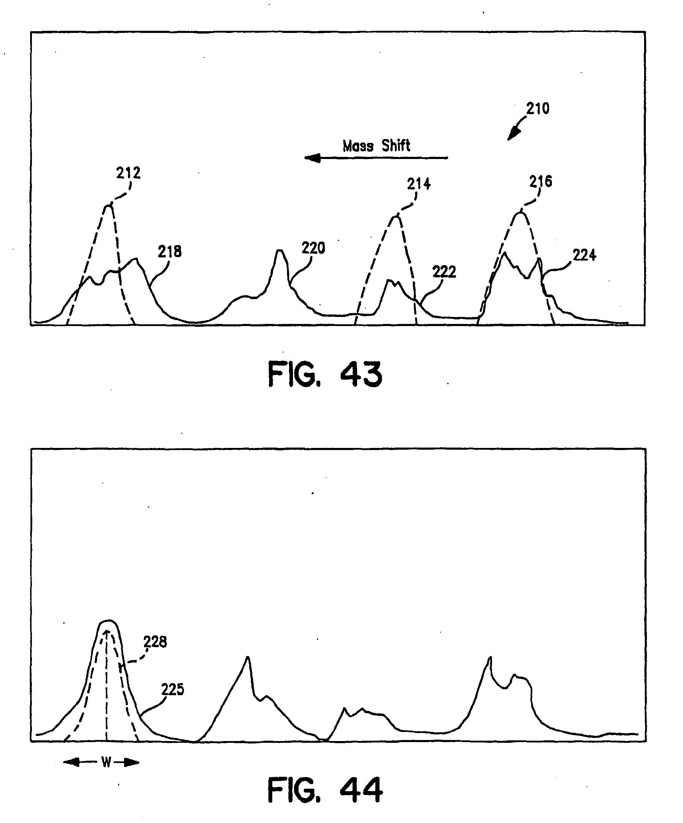

Referring again to FIG. 25, peak heights are now determined in block 60. The first step in determining peak height is illustrated in FIG. 43 where the signal 210 is shifted left or right to correspond with the position of expected peaks. As the set of possible compositions in the biological sample is known before the mass spectrometry data is generated, the possible positioning of expected peaks is already known. These possible peaks are referred to as expected peaks, such as expected peaks 212, 214, and 216. Due to calibration or other errors in the test instrument data, the entire signal may be shifted left or right from its actual position, therefore, putative peaks located in the signal, such as putative peaks 218, 222, and 224 may be compared to the expected peaks 212, 214, and 216, respectively. The entire signal is then shifted such that the putative peaks align more closely with the expected peaks.

Once the putative peaks have been shifted to match expected peaks, the strongest putative peak is identified in FIG. 44. The strongest peak is calculated as a combination of analyzing the overall peak height and area beneath the peak. For example, a moderately high but wide peak would be stronger than a very high peak that is extremely narrow. With the strongest putative peak identified, such as putative peak 225, a Gaussian 228 curve is fit to the peak 225. Once the Gaussian is fit, the width (W) of the Gaussian is determined and will be used as the peak width for future calculations.

As generally addressed above, the denoised, shifted, and baseline-corrected signal is not sufficiently processed for confidently calling the DNA composition of the biological sample. For example, although the baseline has generally been removed, there are still residual baseline effects present. These residual baseline effects are therefore removed to increase the accuracy and confidence in making identifications.

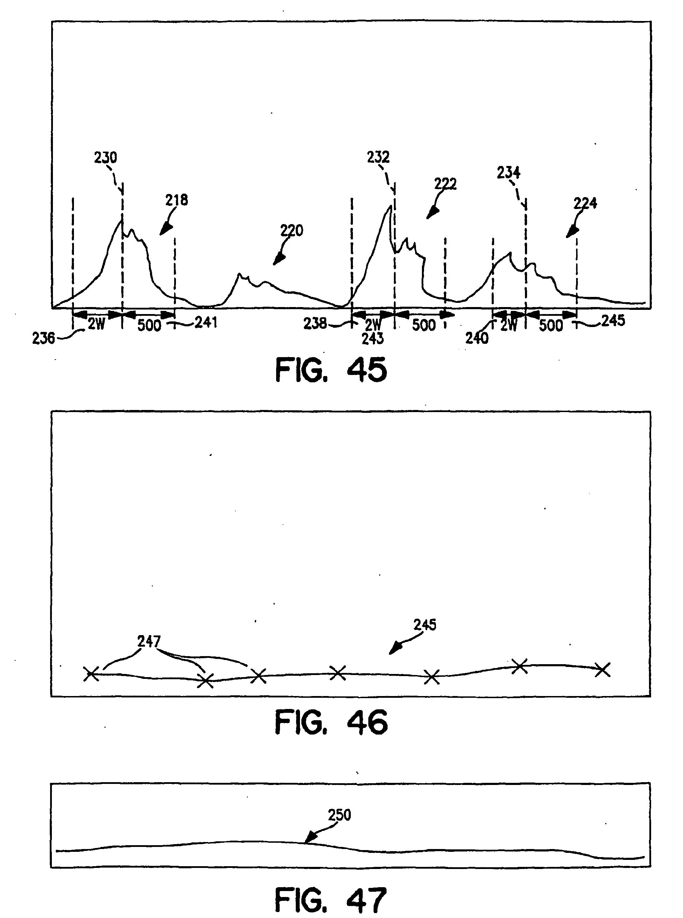

To remove the residual baseline effects, FIG. 45 shows that the putative peaks 218, 222, and 224 are removed from the baseline corrected signal. The peaks are removed by identifying a center line 230, 232, and 234 of the putative peaks 218, 222, and 224, respectively and removing an area to the left and to the right of the identified center line. For each putative peak, an area equal to twice the width (W) of the Gaussian is removed from the left of the center line, while an area equivalent to 50 daltons is removed from the right of the center line. It has been found that the area representing 50 daltons is adequate to sufficiently remove the effect of salt adducts which may be associated with an actual peak. Such adducts appear to the right of an actual peak and are a natural effect from the chemistry involved in acquiring a mass spectrum. Although a 50 Dalton buffer has been selected, it will be appreciated that other ranges or methods can be used to reduce or eliminate adduct effects.

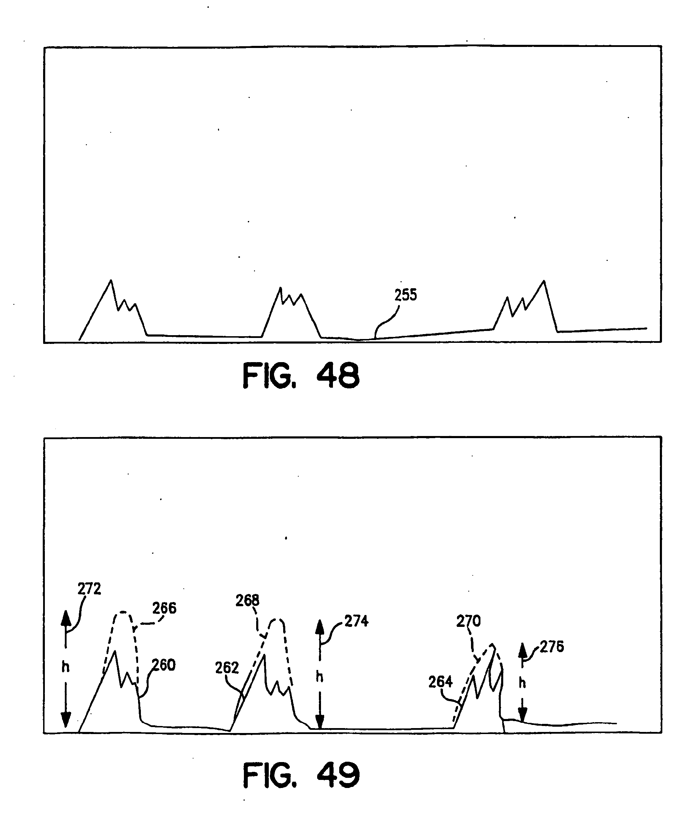

The peaks are removed and remaining minima 247 located as shown in FIG. 46 with the minima 247 connected to create signal 245. A quartic polynomial is applied to signal 245 to generate a residual baseline 250 as shown in FIG. 47. The residual baseline 250 is subtracted from the signal 225 to generate the final signal 255 as indicated in FIG. 48. Although the residual baseline is the result of a quartic fit to signal 245, it will be appreciated that other techniques can be used to smooth or fit the residual baseline.

To determine peak height, as shown in FIG. 49, a Gaussian such as Gaussian 266, 268, and 270 is fit to each of the peaks, such as peaks 260, 262, and 264, respectively. Accordingly, the height of the Gaussian is determined as height 272, 274, and 276. Once the height of each Gaussian peak is determined, then the method of identifying a biological compound 35 can move into the genotyping phase 65 as shown in FIG. 25.

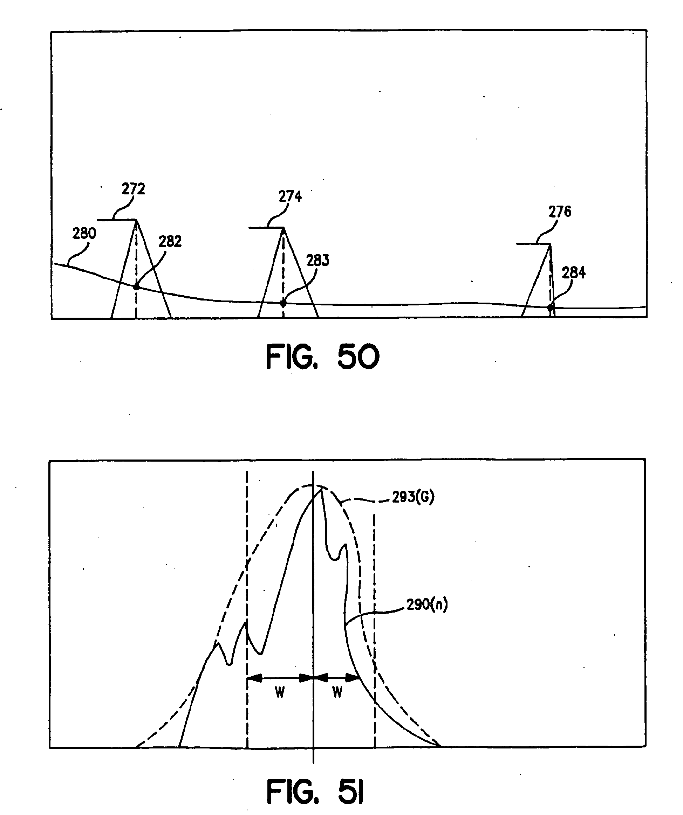

An indication of the confidence that each putative peak is an actual peak can be discerned by calculating a signal-to-noise ratio for each putative peak. Accordingly, putative peaks with a strong signal-to-noise ratio are generally more likely to be an actual peak than a putative peak with a lower signal-to-noise ratio. As described above and shown in FIG. 50, the height of each peak, such as height 272, 274, and 276, is determined for each peak, with the height being an indicator of signal strength for each peak. The noise profile, such as noise profile 97, is extrapolated into noise profile 280 across the identified peaks. At the center line of each of the peaks, a noise value is determined, such as noise value 282, 283, and 284. With a signal values and a noise values generated, signal-to-noise ratios can be calculated for each peak. For example, the signal-to-noise ratio for the first peak in FIG. 50 would be calculated as signal value 272 divided by noise value 282, and in a similar manner the signal-to-noise ratio of the middle peak in FIG. 50 would be determined as signal 274 divided by noise value 283.

Although the signal-to-noise ratio is generally a useful indicator of the presence of an actual peak, further processing has been found to increase the confidence by which a sample can be identified. For example, the signal-to-noise ratio for each peak is preferably adjusted by the goodness of fit between a Gaussian and each putative peak. It is a characteristic of a mass spectrometer that sample material is detected in a manner that generally complies with a normal distribution. Accordingly, greater confidence will be associated with a putative signal having a Gaussian shape than a signal that has a less normal distribution. The error resulting from having a non-Gaussian shape can be referred to as a "residual error".

Referring to FIG. 51, a residual error is calculated by taking a root mean square calculation between the Gaussian 293 and the putative peak 290 in the data signal. The calculation is performed on data within one width on either side of a center line of the Gaussian. The residual error is calculated as: √[(G-R)²/N] where G is the Gaussian signal value, R is the putative peak value, and N is the number of points from -W to +W. The calculated residual error is used to generate an adjusted signal-to-noise ratio, as described below.

An adjusted signal noise ratio is calculated for each putative peak using the formula (S/N)* EXP^{(-1*R)}, where S/N is the signal-to-noise ratio, and R is the residual error determined above. Although the example calculates an adjusted signal-to-noise ratio using a residual error for each peak, it will be appreciated that other techniques can be used to account for the goodness of fit between the Gaussian and the actual signal.

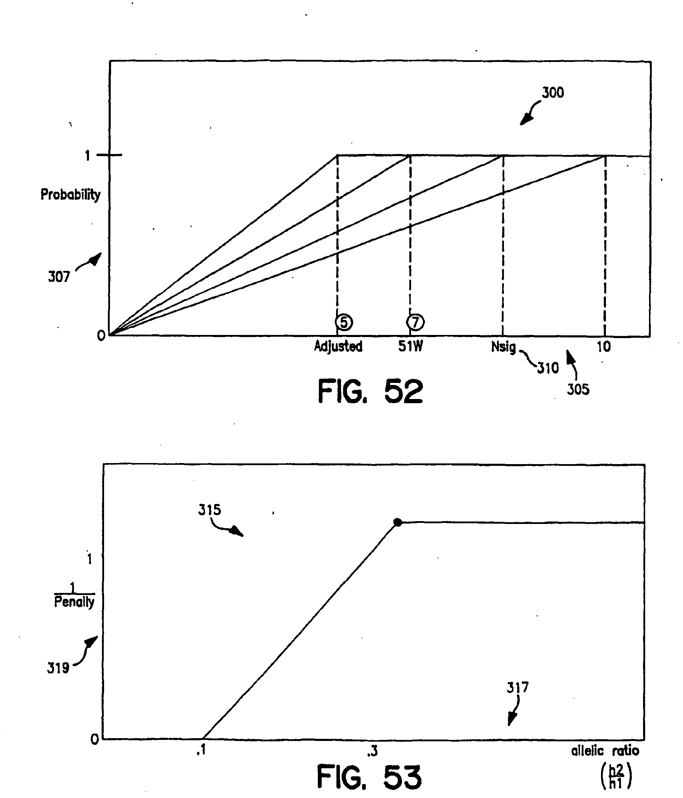

Referring now to FIG. 52, a probability is determined that a putative peak is an actual peak. In making the determination of peak probability, a probability profile 300 is generated where the adjusted signal-to-noise ratio is the x-axis and the probability is the y-axis. Probability is necessarily in the range between a 0% probability and a 100% probability, which is indicated as 1. Generally, the higher the adjusted signal-to-noise ratio, the greater the confidence that a putative peak is an actual peak.

At some target value for the adjusted signal-to-noise, it has been found that the probability is 100% that the putative peak is an actual peak and can confidently be used to identify the DNA composition of a biological sample. However, the target value of adjusted signal-to-noise ratio where the probability is assumed to be 100% is a variable parameter which is to be set according to application specific criteria. For example, the target signal-to-noise ratio will be adjusted depending upon trial experience, sample characteristics, and the acceptable error tolerance in the overall system. More specifically, for situations requiring a conservative approach where error cannot be tolerated, the target adjusted signal-to-noise ratio can be set to, for example, 10 and higher. Accordingly, 100% probability will not be assigned to a peak unless the adjusted signal-to-noise ratio is 10 or over.

In other situations, a more aggressive approach may be taken as sample data is more pronounced or the risk of error may be reduced. In such a situation, the system may be set to assume a 100% probability with a 5 or greater target signal-to-noise ratio. Of course, an intermediate signal-to-noise ratio target figure can be selected, such as 7, when a moderate risk of error can be assumed. Once the target adjusted signal-to-noise ratio is set for the method, then for any adjusted signal-to-noise ratio a probability can be determined that a putative peak is an actual peak.

Due to the chemistry involved in performing an identification test, especially a mass spectrometry test of a sample prepared by DNA amplifications, the allelic ratio between the signal strength of the highest peak and the signal strength of the second (or third and so on) highest peak should fall within an expected ratio. If the allelic ratio falls outside of normal guidelines, the example imposes an allelic ratio penalty to the probability. For example, FIG. 53 shows an allelic penalty 315 which has an x-axis 317 that is the ratio between the signal strength of the second highest peak divided by signal strength of the highest peak. The y-axis 319 assigns a penalty between 0 and 1 depending on the determined allelic ratio. It is assumed that allelic ratios over 30% are within the expected range and therefore no penalty is applied. Between a ratio of 10% and 30%, the penalty is linearly increased until at allelic ratios below 10% it is assumed the second-highest peak is not real. For allelic ratios between 10% and 30%, the allelic penalty chart 315 is used to determine a penalty 319, which is multiplied by the peak probability determined in FIG. 52 to determine a final peak probability. Although the example incorporates an allelic ratio penalty to account for a possible chemistry error, it will be appreciated that other techniques may be used. Similar treatment will be applied to the other peaks.

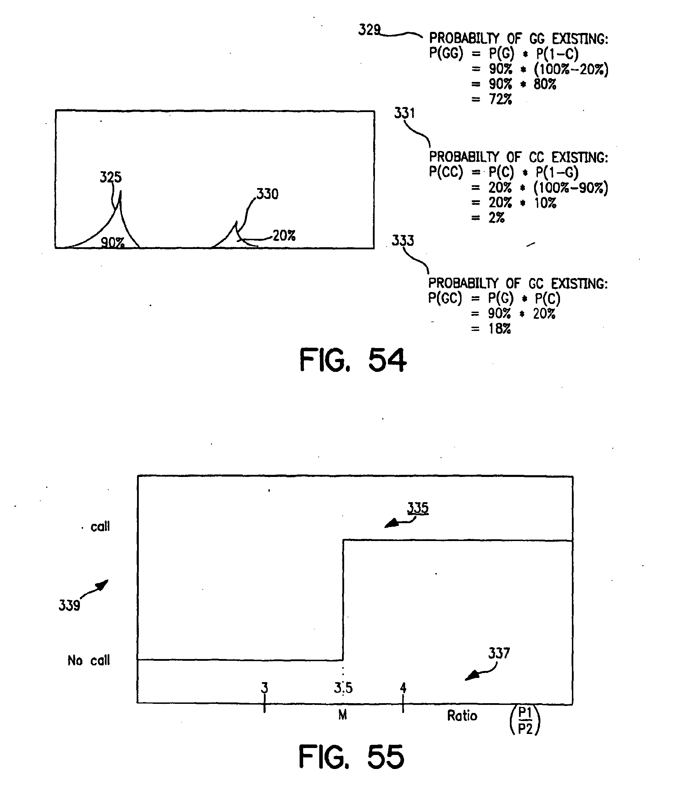

With the peak probability of each peak determined, the statistical probability for various composition components may be determined. As an example, in order to determine the probability of each of three possible combinations of two peaks, -- peak G, peak C and combinations GG, CC and GC. FIG. 54 shows an example where a most probable peak 325 is determined to have a final peak probability of 90%. Peak 325 is positioned such that it represents a G component in the biological sample. Accordingly, it can be maintained that there is a 90% probability that G exists in the biological sample. Also in the example shown in FIG. 54, the second highest probability is peak 330 which has a peak probability of 20%. Peak 330 is at a position associated with a C composition. Accordingly, it can be maintained that there is a 20% probability that C exists in the biological sample.

With the probability of G existing (90%) and the probability of C existing (20%) as a starting point, the probability of combinations of G and C existing can be calculated. For example, FIG. 54 indicates that the probability of GG existing 329 is calculated as 72%. This is calculated as the probability of GG is equal to the probability of G existing (90%) multiplied by the probability of C not existing (100% -20%). So if the probability of G existing is 90% and the probability of C not existing is 80%, the probability of GG is 72%.

In a similar manner, the probability of CC existing is equivalent to the probability of C existing (20%) multiplied by the probability of G not existing (100% - 90%). As shown in FIG. 54, the probability of C existing is 20% while the probability of G not existing is 10%, so therefore the probability of CC is only 2%. Finally, the probability of GC existing is equal to the probability of G existing (90%) multiplied by the probability of C existing (20%). So if the probability of G existing is 90% and the probability of C existing is 20%, the probability of GC existing is 18%. In summary form, then, the probability of the composition of the biological sample is:
- probability of GG:: 72%;
- probability of GC:: 18%; and
- probability of CC:: 2%.

Once the probabilities of each of the possible combinations has been determined, FIG. 55 is used to decide whether or not sufficient confidence exists to call the genotype. FIG. 55 shows a call chart 335 which has an x-axis 337 which is the ratio of the highest combination probability to the second highest combination probability. The y-axis 339 simply indicates whether the ratio is sufficiently high to justify calling the genotype. The value of the ratio may be indicated by M 340. The value of M is set depending upon trial data, sample composition, and the ability to accept error. For example, the value M may be set relatively high, such as to a value 4 so that the highest probability must be at least four times greater than the second highest probability before confidence is established to call a genotype. However, if a certain level of error may be acceptable, the value of M may be set to a more aggressive value, such as to 3, so that the ratio between the highest and second highest probabilities needs to be only a ratio of 3 or higher. Of course, moderate value may be selected for M when a moderate risk can be accepted. Using the example of FIG. 54, where the probability of GG was 72% and the probability of GC was 18%, the ratio between 72% and 18% is 4.0, therefore, whether M is set to 3, 3.5, or 4, the system would call the genotype as GG. Although the example uses a ratio between the two highest peak probabilities to determine if a genotype confidently can be called, it will be appreciated that other methods may be substituted. It will also be appreciated that the above techniques may be used for calculating probabilities and choosing genotypes (or more general DNA patterns) containing of combinations of more than two peaks.

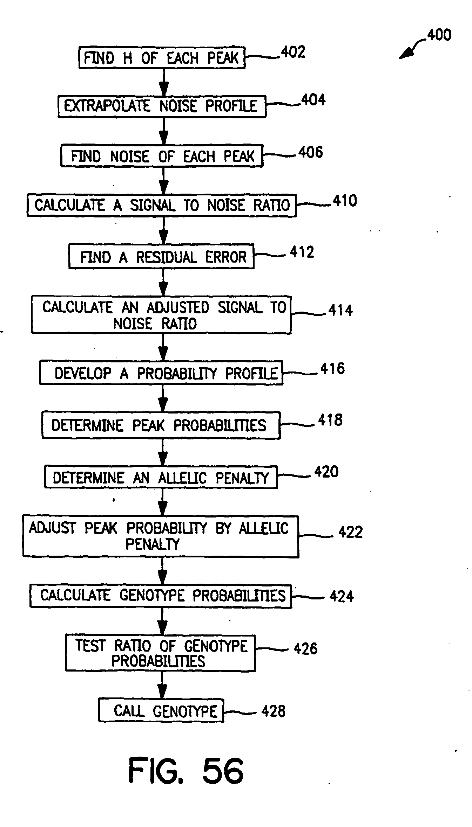

Referring now to FIG. 56, a flow chart is shown generally defining the process of statistically calling genotype described above. In FIG. 56 block 402 shows that the height of each peak is determined and that in block 404 a noise profile is extrapolated for each peak. The signal is determined from the height of each peak in block 406 and the noise for each peak is determined using the noise profile in block 408. In block 410, the signal-to-noise ratio is calculated for each peak. To account for a non-Gaussian peak shape, a residual error is determined in block 412 and an adjusted signal-to-noise ratio is calculated in block 414. Block 416 shows that a probability profile is developed, with the probability of each peak existing found in block 418. An allelic penalty may be applied in block 42D, with the allelic penalty applied to the adjusted peak probability in block 422. The probability of each combination of components is calculated in block 424 with the ratio between the two highest probabilities being determined in block 426. If the ratio of probabilities exceeds a threshold value then the genotype is called in block 428.

In another example, the computing device 20 (Fig. 24) supports "standardless" genotyping by identifying data peaks that contain putative SNPs. Standardless genotyping is used, for example, where insufficient information is known about the samples to determine a distribution of expected peak locations, against which an allelic penalty as described above can be reliably calculated. This permits the computing device to be used for identification of peaks that contain putative SNPs from data generated by any assay that fragments a targeted DNA molecule. For such standardless genotyping, peaks that are associated with an area under the data curve that deviates significantly from the typical area of other peaks in the data spectrum are identified and their corresponding mass (location along the x-axis) is determined.

More particularly, peaks that deviate significantly from the average area of other peaks in the data are identified, and the expected allelic ratio between data peaks is defined in terms of the ratio of the area under the data peaks. Theoretically, where each genetic loci has the same molar concentration of analyte, the area under each corresponding peak should be the same, thus producing a 1.0 ratio of the peak area between any two peaks. In accordance with the invention, peaks having a smaller ratio relative to the other peaks in the data will not be recognized as peaks. More particularly, peaks having an area ratio smaller than 30% relative to a nominal value for peak area will be assigned an allelic penalty. The mass of the remaining peaks (their location along the x-axis of the data) will be determined based on oligonucleotide standards.

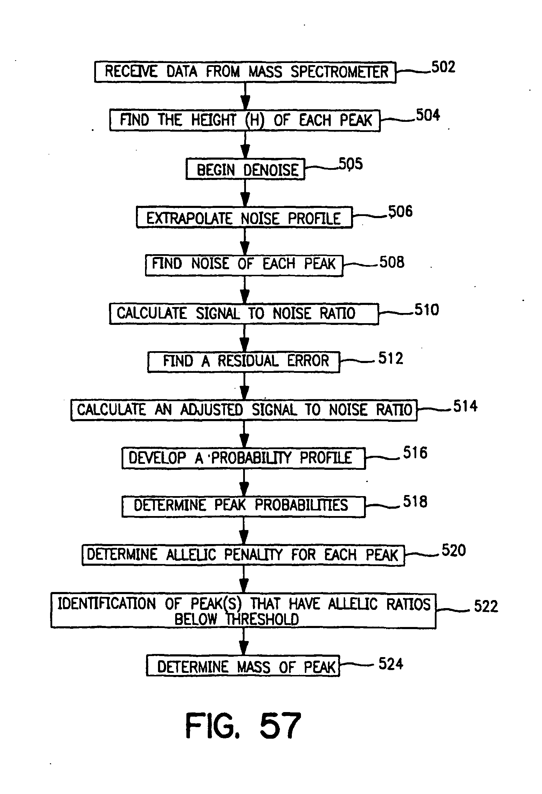

Fig. 57 shows a flow diagram representation of the processing by the computing device 20 (Fig. 24) when performing standardless genotyping. In the first operation, represented by the flow diagram box numbered 502, the computing device receives data from the mass spectrometer. Next, the height of each putative peak in the data sample is determined, as indicated by the block 504. After the height of each peak in the mass spectrometer data is determined, a de-noise process 505 is performed, beginning with an extrapolation of the noise profile (block 506), followed by finding the noise of each peak (block 508) and calculating the signal to noise ratio for each data sample (block 510). Each of these operations may be performed in accordance with the description above for denoise operations 45 of Fig. 25. Other suitable denoise operations will occur to those skilled in the art.

The next operation is to find the residual error associated with each data point. This is represented by the block 512 in Figure 57. The next step, block 514, involves calculating an adjusted signal to noise ratio for each identified peak. A probability profile is developed next (block 516), followed by a determination of the peak probabilities at block 518. In the preferred embodiment, the denoise operations of Fig. 57, comprising block 502 to block 518, comprises the corresponding operations described above in conjunction with Fig. 56 for block 402 through block 418, respectively.

The next action for the standardless genotype processing is to determine an allelic penalty for each peak, indicated by the block 524. As noted above, the standardless genotype processing of Fig. 57 determines an allelic penalty by comparing area under the peaks. Therefore, rather than compare signal strength ratios to determine an allelic penalty, such as described above for Fig. 53, the standardless processing determines the area under each of the identified peaks and compares the ratio of those areas. Determining the area under each peak may be computed using conventional numerical analysis techniques for calculating the area under a curve for experimental data.

Thus, the allelic penalty is assigned in accordance with Fig. 58, which shows that no penalty is assigned to peaks having a peak area relative to an expected average area value that is greater than 0.30 (30%). The allelic penalty is applied to the peak probability value, which may be determined according to the process such as described in Fig. 52. It should be apparent from Fig. 58 that the allelic penalty imposed for peaks below a ratio of 30% is that such peaks will be removed from further measurement and processing. Other penalty schemes, however, may be imposed in accordance with knowledge about the data being processed, as determined by those skilled in the art.

After the allelic penalty has been determined and applied, the standardless genotype processing compares the location of the remaining putative peaks to oligonucleotide standards to determine corresponding masses in the processing for block 524. For standardless genotype data, the processing of the block 524 is performed to determine mass and genotype, rather than performing the operations corresponding to block 424, 426, and 428 of Fig. 33. Techniques for performing such comparisons and determining mass will be known to those skilled in the art.

In another example the computing device 20 (Fig. 24) permits the detection and determination of the mass (location along the x-axis of the data) of the sense and antisense strand of fragments generated in the assay. If desired, the computing device may also detect and determine the quantity (area under each peak) of the respective sense and antisense strands, using a similar technique to that described above for standardless genotype processing. The data generated for each type of strand may then be combined to achieve a data redundancy and to thereby increase the confidence level of the determined genotype. This technique obviates primer peaks that are often observed in data from other diagnostic methods, thereby permitting a higher level of multiplexing. In addition, when quantitation is used in pooling experiments, the ratio of the measured peak areas is more reliably calculated than the peak identifying technique, due to data redundancy.

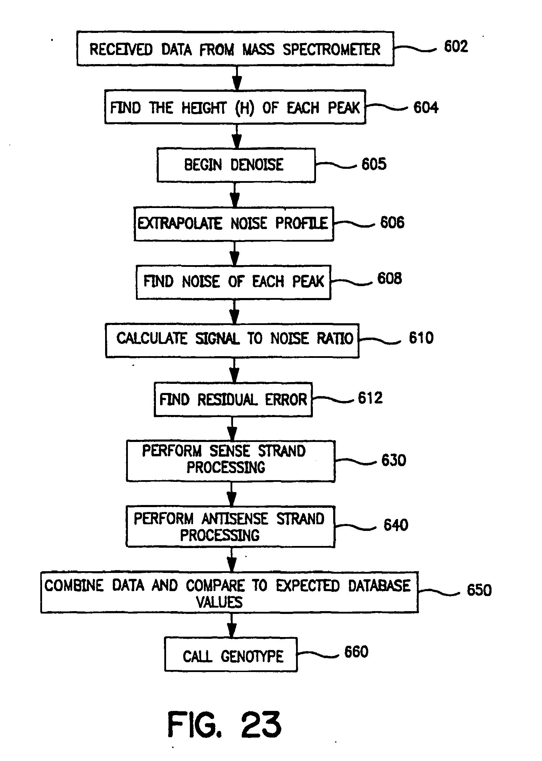

Fig. 23 is a flow diagram that illustrates the processing implemented by the computing device 20 to perform sense and antisense processing. In the first operation, represented by the flow diagram box numbered 602, the computing device receives data from the mass spectrometer. This data will include data for the sense strand and antisense strand of assay fragments. Next, the height of each putative peak in the data sample is determined, as indicated by the block 604. After the height of each peak in the mass spectrometer data is determined, a de-noise process 605 is performed, beginning with an operation that extrapolates the noise profile (block 606), followed by finding the noise of each peak (block 608) and calculating the signal to noise ratio for each data sample (block 610). Each of these operations may be performed in accordance with the description above for the denoise operations 45 of Fig. 25. Other suitable denoise operations will occur to those skilled in the art. The next operation is to find the residual error associated with each data point. This is represented by the block 612 in Figure 36.

After the residual error for the data of the sense strand and antisense strand has been performed, processing to identify the genotypes will be performed for the sense strand and also for the antisense strand. Therefore, Fig. 23 shows that processing includes sense strand processing (block 630) and antisense strand processing (block 640). Each block 630, 640 includes processing that corresponds to adjusting the signal to noise ratio, developing a probability profile, determining an allelic penalty, adjusting the peak probability by the allelic penalty, calculating genotype probabilities, and testing genotype probability ratios, such as described above in conjunction with blocks 414 through 426 of Fig. 56. The processing of each block 630, 640 may, if desired, include standardless processing operations such as described above in conjunction with Fig. 57. The standardless processing may be included in place of or in addition to the processing operations of Fig. 56.

After the genotype probability processing is completed, the data from the sense strand and antisense strand processing is combined and compared to expected database values to obtain the benefits of data redundancy as between the sense strand and antisense strand. Those skilled in the art will understand techniques to take advantage of known data redundancies between a sense strand and antisense strand of assay fragments. This processing is represented by the block 650. After the data from the two strands is combined for processing, the genotype processing is performed (block 660) and the genotype is identified.

Since modifications will be apparent to those of skill in this art, it is intended that this invention be limited only by the scope of the appended claims.

## Claims

1. A method of using a database for haplotyping polymorphisms in a population, comprising:
sorting a database, according to a selected parameter to identify members that match the selected parameter, wherein the database comprises datapoints comprising identifying information for members of a target population of organisms not manifesting any disease state, historical information for said members, genotypic information for said member, and data related to said members and their immediate family, and an indexer that associates each member and the datapoints, data, and genotypic information;
a) obtaining samples previously isolated from the identified members;
b) isolating a nucleic acid from each sample;
c) pooling each isolated nucleic acid;
d) optionally amplifying the amount of nucleic acid;
e) contacting the nucleic acid with at least one enzyme to produce fragments thereof;
f) forming single-stranded nucleic acid and spitting each single strand into a separate reaction vessel;
g) contacting each single-stranded nucleic acid with an adaptor nucleic acid to form an adaptor complex;
h) contacting the adaptor complex with a nuclease and a ligase to produce a ligated adaptor;
i) amplifying the ligated adaptor to produce an extended product;
j) obtaining a mass spectrum of each nucleic acid resulting from step i) and detecting a polymorphism by identifying a signal corresponding to the extended product;
k) repeating steps g) through j) utilizing an adaptor nucleic acid able to hybridize with the product of step i) and which hybridizes to a different sequence on the same strand; whereby
the polymorphisms are haplotyped by detecting more than one extended product.

2. The method of claim. 1 , wherein the nuclease is Fen-1.

3. The method of claims 1, wherein the enzyme is a nickase.

4. The method of claim 3, wherein the nickase is selected from the group consisting of NY2A and NYS1.

5. The method of any of claims 1 to 4, wherein the samples are selected from the group consisting of blood, blood fractions, cells, subcellular organelles, tissue, body fluid, seed, microbe, pathogen, and reproductive tissue samples.

6. The method of any one of claims 1 to 5, wherein the datapoints are answers to questions regarding one or more
parameters selected from the group consisting of ethnicity, age, gender, height, weight, alcohol intake, number of pregnancies, number of live births, vegetarianism, type of physical activity, state of residence and/or length of residence in a particular state, educational level, age of parent at death, cause of parent death, former or current smoker, length of time as a smoker, frequency of smoking, occurrence of a disease in immediate family (parent, siblings, children), use of prescription drugs and/or reason therefore, length and/or number of hospital stays, and exposure to environmental factors.

7. The method of any one of claims 1 to 6, wherein the organisms are selected from among animals, bacteria, fungi, protozoans, and parasites and each datapoint is associated with parameters representative of the organism type and identifying information.

8. The method of claim 7, wherein the animals are mammals.

9. The method of claim 8, wherein the animals are humans.

10. The method of any one of claims 1 to 9, wherein said database further comprises phenotypic data.

11. The method of any one of claims 5to 10, wherein the data includes one of physical characteristics, background data, medical data, and historical data.

12. The method of any one of claims 5to 11 wherein the genotypic data is from nucleic acid obtained from an organism.

13. The method of any one of claims 5to 12 (wherein the genotypic data includes genetic markers, non-coding regions, microsatellites, RFLPs and VNTRs.

14. The method of any one of claims 5 to 13 wherein the database is a relational database.

15. The method of claim 14, wherein the data are related to an indexer datapoint representative of each organism from whom data is obtained.

16. The method of any one of claims 5 to 15, wherein the database is stored in a memory of a computer storage medium.

17. The method of any one of claims 5 to 16, wherein the mass spectrometric format is selected from among Matrix-Assisted Laser Desorption/Ionization, Time-of-Flight (MALDITOF), Electrospray (ES), IR-MALDI, Ion Cyclotron Resonance (ICR), Fourier Transform, and combinations thereof.

18. The method of claim 1, further comprising sorting the database to identify members that match a second selected parameter;
obtaining samples previously isolated from the identified members;
isolating a nucleic acid from each sample;
pooling each isolated nucleic acid;
optionally amplifying the amount of nucleic acid;
contacting the nucleic acid with at least one enzyme to produce fragments thereof;
obtaining a mass spectrum of the resulting fragments; whereby the polymorphisms are detected by detecting signals corresponding to the polymorphisms;
haplotyping the polymorphisms by determining from the mass spectrum that the polymorphisms are located on the same strand of the nucleic acid; and
comparing the haplotype of the members identified using the first selected parameter of claim 1 to the haplotype of the members identified using the second selected parameter.

19. The method of claim 18, wherein the first selected parameter includes a different age of the individual than the second selected parameter.

## Patentansprüche

1. Verfahren zur Verwendung einer Datenbank zur Haplotypisierung von Polymorphismen in einer Population, welches umfasst:
Sortieren einer Datenbank nach einem ausgewählten Parameter, um Mitglieder zu identifizieren, die mit dem ausgewählten Parameter übereinstimmen, wobei die Datenbank Datenpunkte umfasst, die die Identifizierungs-Informationen für Mitglieder einer Zielpopulation von Organismen, die keine Krankheiten zeigen, historische Informationen für die Mitglieder, Genotyp-Informationen für das Mitglied und Daten, die die Mitglieder und ihre engsten Angehörigen betreffen und einen Indexer, der jedes Mitglied und die Datenpunkte, Daten und Genotyp-Informationen verbindet, umfassen;
a) Erhalten von Proben, die vorher aus den identifizierten Mitgliedern isoliert wurden;
b) Isolieren einer Nukleinsäure aus jeder Probe;
c) Vereinigen jeder isolierten Nukleinsäure;
d) gegebenenfalls Amplifizieren der Menge an Nukleinsäure;
e) in Kontakt bringen der Nukleinsäure mit mindestens einem Enzym, um Fragmente davon herzustellen;
f) Bilden von Einzelstrang-Nukleinsäure und Aufteilung jedes einzelnen Stranges in ein separates Reaktionsgefäß;
g) in Kontakt bringen jeder Einzelstrang-Nukleinsäure mit einer Adapter-Nukleinsäure, um einen Adapter-Komplex zu bilden;
h) in Kontakt bringen des Adapter-Komplexes mit einer Nuklease und einer Ligase, um einen ligierten Adapter herzustellen;
i) Amplifizieren des ligierten Adapters, um ein verlängertes Produkt herzustellen;
j) Erhalten eines Massenspektrums jeder Nukleinsäure, die aus Schritt i) resultiert und Detektieren eines Polymorphismus, indem ein Signal identifiziert wird, das dem verlängerten Produkt entspricht;
k) Wiederholen der Schritte g) bis j), wobei eine Adapter-Nukleinsäure verwendet wird, die mit dem Produkt aus Schritt i) hybridisieren kann und die an andere
Sequenzen auf demselben Strang hybridisiert; wobei
die Polymorphismen durch die Detektion von mehr als einem verlängerten Produkt haplotypisiert werden.

2. Verfahren nach Anspruch 1, wobei die Nuklease Fen-1 ist.

3. Verfahren nach Anspruch 1, wobei das Enzym eine Nickase ist.

4. Verfahren nach Anspruch 3, wobei die Nickase ausgewählt ist aus der Gruppe bestehend aus NY2A und NYS1.

5. Verfahren nach einem der Ansprüche 1 bis 4, wobei die Proben ausgewählt sind aus der Gruppe bestehend aus Blut, Blufraktionen, Zellen, subzellulären Organellen, Gewebe, Köperflüssigkeiten, Samen, Mikroben, Pathogenen und Reproduktionsgewebe-Proben.

6. Verfahren nach einem der Ansprüche 1 bis 5, wobei die Datenpunkte Antworten auf Fragen sind, bezüglich eines oder mehrerer Parameter(s) ausgewählt aus der Gruppe bestehend aus Ethnizität, Alter, Geschlecht, Größe, Gewicht, Alkoholkonsum, Anzahl an Schwangerschaften, Anzahl von Lebendgeburten, Vegetarismus, Art von körperlicher Aktivität, Staat des Wohnsitzes und/oder Dauer des Wohnsitzes in einem bestimmten Staat, Bildungsgrad, Alter der Eltern zum Todeszeitpunkt, Ursache des Tods der Eltern, ehemaliger oder aktueller Raucher, Dauer als Raucher, Häufigkeit des Rauchens, Auftreten einer Krankheit bei engsten Angehörigen (Eltern, Geschwister, Kinder), Einnahme von verschreibungspflichtigen Medikamenten und/oder ein Grund dafür, Dauer und/oder Anzahl an Krankenhausaufenthalten und Aussetzen gegenüber Umweltfaktoren.

7. Verfahren nach einem der Ansprüche 1 bis 6, wobei die Organismen ausgewählt sind aus Tieren, Bakterien, Pilzen, Protozoen und Parasiten und jeder Datenpunkt mit Parametern, die repräsentativ für die Art des Organismus sind, und Identffizierungs-Informationen verbunden ist.

8. Verfahren nach Anspruch 7, wobei die Tiere Säugetiere sind.

9. Verfahren nach Anspruch 8, wobei die Tiere Menschen sind.

10. Verfahren nach einem der Ansprüche 1 bis 9, wobei die Datenbank weiter Phänotyp-Daten umfasst.

11. Verfahren nach einem der Ansprüche 5 bis 10, wobei die Daten eines von körperlichen Eigenschaften, Hintergrunddaten, medizinischen Daten und historischen Daten einschließen.

12. Verfahren nach einem der Ansprüche 5 bis 11, wobei die Genotyp-Daten aus einer Nukleinsäure stammen, die aus einem Organismus erhalten wurde.

13. Verfahren nach einem der Ansprüche 5 bis 12, wobei die Genotyp-Daten genetische Marker, nicht-kodierende Regionen, Mikrosatelliten, RFLPs und VNTRs einschließen.

14. Verfahren nach einem der Ansprüche 5 bis 13, wobei die Datenbank eine relationale Datenbank ist.

15. Verfahren nach Anspruch 14, wobei sich die Daten auf einen Indexer-Datenpunkt beziehen, der repräsentativ für jeden Organismus ist, aus dem Daten erhalten werden.

16. Verfahren nach einem der Ansprüche 5 bis 15, wobei die Datenbank in einem Speicher auf einem Computerspeichermedium gespeichert wird.

17. Verfahren nach einem der Ansprüche 1 bis 16, wobei das Massenspektrometer-Format ausgewählt ist aus Matrixunterstützter Laserdesorption/Ionisation Flugzeit ("Matrix-assisted Laser Desorption/Ionisation Time of Flight", MALDI-TOF), Elektrospray (ES), IR-MALDI, lonencyclotronresonanz (ICR), Fouriertransformation und Kombinationen davon.

18. Verfahren nach Anspruch 1, weiter umfassend Sortieren der Datenbank, um Mitglieder zu identifizieren, die mit einem zweiten ausgewählten Parameter übereinstimmen;
Erhalten von Proben, die vorher aus den identifizierten Mitgliedern isoliert wurden;
Isolieren einer Nukleinsäure aus jeder Probe;
Vereinigen jeder isolierten Nukleinsäure;
gegebenenfalls Amplifizieren der Menge an Nukleinsäure;
in Kontakt bringen der Nukleinsäure mit mindestens einem Enzym, um Fragmente davon herzustellen;
Erhalten eines Massenspektrums der resultierenden Fragmente;
wobei die Polymorphismen detektiert werden, indem Signale detektiert werden, die den Polymorphismen entsprechen;
Haplotypisieren der Polymorphismen, indem mit dem Massenspektrum bestimmt wird, dass die Polymorphismen auf demselben Strang der Nukleinsäure lokalisiert sind; und
Vergleichen des Haplotyps der Mitglieder, die unter Verwendung des ersten ausgewählten Parameters aus Anspruch 1 identifiziert wurden, mit dem Haplotyp der Mitglieder, die unter Verwendung des zweiten ausgewählten Parameters identifiziert wurden.

19. Verfahren nach Anspruch 18, wobei der erste ausgewählte Parameter ein anderes Alter des Individuums als der zweite ausgewählte Parameter einschließt.

## Revendications

1. Procédé d'utilisation d'une base de données pour déterminer les haplotypes de polymorphismes dans une population, consistant à :
trier une base de données, en fonction d'un paramètre sélectionné, pour identifier des membres qui correspondent au paramètre sélectionné, dans lequel la base de données comprend des points de données comprenant des informations d'identification de membres d'une population cible d'organismes ne manifestant pas d'état pathologique, des informations historiques concernant lesdits membres, des informations génotypiques concernant lesdits membres, et des données concernant lesdits membres et leurs familles immédiates, et un dispositif d'indexation qui associe chaque membre aux points de données, aux données, et aux informations génotypiques ;
a) obtenir des échantillons précédemment isolés à partir des éléments identifiés ;
b) isoler un acide nucléique à partir de chaque échantillon ;
c) mettre en commun chaque acide nucléique isolé ;
d) amplifier facultativement la quantité d'acide nucléique ;
e) mettre l'acide nucléique en contact avec au moins un enzyme pour produire des fragments de celui-ci ;
f) former un acide nucléique à un seul filament et décomposer chaque filament unique dans un récipient réactionnel séparé ;
g) mettre chaque acide nucléique à un seul filament en contact avec un acide nucléique adaptateur pour former un complexe adaptateur ;
h) mettre le complexe adaptateur en contact avec une nucléase et une ligase afin de produire un adaptateur ligaturé ;
i) amplifier l'adaptateur ligaturé pour produire un produit étendu ;
j) obtenir un spectre de masse de chaque acide nucléique résultant de l'étape i) et détecter un polymorphisme en identifiant un signal correspondant au produit étendu ;
k) répéter les étapes g) à j) en utilisant un acide nucléique adaptateur capable de s'hybrider avec le produit de l'étape i) et qui s'hybride avec une séquence différente sur le même filament ; d'où il résulte que
les polymorphismes sont soumis à une détermination des haplotypes en détectant plus d'un produit étendu.

2. Procédé selon la revendication 1, dans lequel la nucléase est le Fen-1.

3. Procédé selon la revendication 1, dans lequel l'enzyme est une nickase.

4. Procédé selon la revendication 3, dans lequel la nickase est sélectionnée dans le groupe constitué de NY2A et de NYS1.

5. Procédé selon l'une quelconque des revendications 1 à 4, dans lequel les échantillons sont sélectionnés dans le groupe constitué de sang, de fractions sanguines, de cellules, d'organelles subcellulaires, de tissus, de fluide corporel, de semence, de microbes, d'agents pathogènes ou d'échantillons de tissus reproducteurs.

6. Procédé selon l'une quelconque des revendications 1 à 5, dans lequel les points de données sont des réponses à des questions concernant un ou plusieurs de paramètres sélectionnés dans le groupe constitué de l'origine ethnique, de l'âge, du sexe, de la taille, du poids, de l'absorption d'alcool, du nombre de grossesses, du nombre de naissances vivantes, d'un régime végétarien, du type d'activité physique, de l'état de résidence et/ou de la longueur du séjour dans un état particulier, du niveau d'éducation, de l'âge d'un parent lors de sa mort, de la cause de la mort du parent, de l'antécédent tabagique ou du tabagisme actuel, de la durée du tabagisme, de la fréquence d'usage du tabac, de la survenue d'une maladie dans la famille proche (parent, fratrie, enfants), de l'utilisation de médicaments prescrits et/ou de la raison de cette utilisation, de la longueur et/ou du nombre de séjours en hôpital, et de l'exposition à des facteurs environnementaux.

7. Procédé selon l'une quelconque des revendications 1 à 6, dans lequel les organismes sont sélectionnés parmi des animaux, des bactéries, des champignons, des protozoaires et des parasites, et chaque point de données est associé à des paramètres représentatifs du type d'organisme et à des informations d'identification.

8. Procédé selon la revendication 7, dans lequel les animaux sont des mammifères.

9. Procédé selon la revendication 8, dans lequel les animaux sont des êtres humains.

10. Procédé selon l'une quelconque des revendications 1 à 9, dans lequel ladite base de données comprend en outre des données phénotypiques.

11. Procédé selon l'une quelconque des revendications 5 à 10, dans lequel les données comprennent l'une de caractéristiques physiques, de données d'antécédents, de données médicales et de données historiques.

12. Procédé selon l'une quelconque des revendications 5 à 11, dans lequel les données génotypiques proviennent de l'acide nucléique obtenu à partir d'un organisme.

13. Procédé selon l'une quelconque des revendications 5 à 12, dans lequel les données génotypiques comprennent des marqueurs génétiques, des régions non codantes, des microsatellites, des RFLP (polymorphismes de restriction) et des VNTR (répétitions de tandems en nombre variable).

14. Procédé selon l'une quelconque des revendications 5 à 13, dans lequel la base de données est une base de données relationnelle.

15. Procédé selon la revendication 14, dans lequel les données sont liées à un point de données d'indexation représentatif de chaque organisme sur lequel des données sont obtenues.

16. Procédé selon l'une quelconque des revendications 5 à 15, dans lequel la base de données est stockée dans une mémoire d'un support de stockage d'ordinateur.

17. Procédé selon l'une quelconque des revendications 5 à 16, dans lequel le format spectrométrique de masse est sélectionné parmi le format de Désorption/Ionisation Laser Assisté par Matrice, le format de temps de vol (MALDITOF), l'Electropulvérisation (ES), le format IR-MALDI, le format de résonance cyclotronique d'ions (ICA), le format de Transformée de Fourier, et leurs combinaisons.

18. Procédé selon la revendication 1, consistant en outre à trier la base de données pour identifier des éléments qui concordent avec un deuxième paramètre sélectionné ;
obtenir des échantillons précédemment isolés à partir des éléments identifiés ;
isoler un acide nucléique à partir de chaque échantillon ;
mettre en commun chaque acide nucléique isolé ;
amplifier facultativement la quantité d'acide nucléique ;
mettre l'acide nucléique en contact avec au moins un enzyme afin de produire des fragments de celui-ci ;
obtenir un spectre de masse des fragments qui en résultent ; de sorte que les polymorphismes soient détectés en détectant des signaux correspondant aux polymorphismes ;
déterminer l'haplotype des polymorphismes en déterminant à partir du spectre de masse le fait que les polymorphismes sont situés sur le même filament de l'acide nucléique ; et
comparer l'haplotype des éléments identifiés en utilisant le premier paramètre sélectionné selon la revendication 1, à l'haplotype des éléments identifiés en utilisant le deuxième paramètre sélectionné.

19. Procédé selon la revendication 18, dans lequel le premier paramètre sélectionné comprend un âge de l'individu qui est différent du deuxième paramètre sélectionné.
